(19) Europäisches Patentamt
European Patent Office
Office européen des brevets

(11) EP 3 759 108 B1

(12) **EUROPÄISCHE PATENTSCHRIFT**

(45) Veröffentlichungstag und Bekanntmachung des Hinweises auf die Patenterteilung:
**30.08.2023 Patentblatt 2023/35**

(21) Anmeldenummer: **19706670.7**

(22) Anmeldetag: **26.02.2019**

(51) Internationale Patentklassifikation (IPC):
***C07D 487/04*** *(2006.01)* ***C07D 519/00*** *(2006.01)*
***A61P 35/00*** *(2006.01)* ***A61K 38/00*** *(2006.01)*

(52) Gemeinsame Patentklassifikation (CPC):
**C07D 487/04; A61K 31/55; A61P 35/00; C07D 519/00**

(86) Internationale Anmeldenummer:
**PCT/EP2019/054715**

(87) Internationale Veröffentlichungsnummer:
**WO 2019/162524 (29.08.2019 Gazette 2019/35)**

(54) **POLYPROLIN-MIMETIKA DES PROLIN-ABGELEITETEN MODULS-15**

POLYPROLIN MIMETICS OF PROLINE DERIVED MODULE -15

MIMÉTIQUE DE POLYPROLINE DU MODULE 15 DÉRIVÉE DE LA PROLINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.02.2018 EP 18158650**
**04.06.2018 EP 18175731**

(43) Veröffentlichungstag der Anmeldung:
**06.01.2021 Patentblatt 2021/01**

(73) Patentinhaber:
- **Forschungsverbund Berlin E.V.**
**12489 Berlin (DE)**
- **Universität zu Köln**
**50923 Köln (DE)**

(72) Erfinder:
- **KÜHNE, Ronald**
**12683 Berlin (DE)**
- **SCHMALZ, Hans-Günther**
**50321 Brühl (DE)**
- **DOHMEN, Stephan**
**50939 Köln (DE)**
- **BARONE, Matthias**
**10439 Berlin (DE)**
- **MÜLLER, Matthias**
**10559 Berlin (DE)**
- **CHIHA, Slim**
**50674 Köln (DE)**
- **ALBAT, Dominik**
**50676 Köln (DE)**

(74) Vertreter: **Hertin und Partner**
**Rechts- und Patentanwälte PartG mbB**
**Kurfürstendamm 54/55**
**10707 Berlin (DE)**

(56) Entgegenhaltungen:
**WO-A1-2013/030111 WO-A1-2016/092069**

- **CÉDRIC REUTER ET AL: "Supporting Information: Stereoselective synthesis of proline-derived dipeptide scaffolds (ProM-3 and ProM-7) rigidified in a PPII helix conformation", EUROPEAN JOURNAL OF ORGANIC CHEMISTRY, Bd. 2014, Nr. 13, 25. März 2014 (2014-03-25), Seiten 2664-2667, XP055506287, DE ISSN: 1434-193X, DOI: 10.1002/ejoc.201301875**

Bemerkungen:
Das gesamte Dokument mit Referenztabelle(n) und Sequenzliste(n) kann von der Webseite des EPA heruntergeladen werden

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 759 108 B1

## Beschreibung

Die vorliegende Erfindung betrifft chemische Verbindungen, die insbesondere als Struktur-Mimetika Prolinreicher Peptide eingesetzt werden können. Die Verbindungen der vorliegenden Erfindung sind in der Lage ena/VASP-EVH1-vermittelte Protein-Protein-Wechselwirkungen selektiv zu hemmen. Die Erfindung betrifft weiterhin die Verwendung dieser Verbindungen als pharmazeutische Wirkstoffe sowie die Verwendung der pharmazeutischen Wirkstoffe zur Behandlung von Tumorerkrankungen.

## HINTERGRUND ZUR ERFINDUNG

Bei fast sämtlichen physiologischen und biochemischen Prozessen in lebenden Organismen spielen Wechselwirkungen zwischen Proteinen eine zentrale Rolle. Proteine erfüllen nicht nur biokatalytische Aufgaben (zum Beispiel als Enzyme) sondern sind an verschiedenen biologischen Prozessen beteiligt. Eine selektive Hemmung der körperlichen Interaktion zwischen Peptidliganden und den entsprechenden Proteinrezeptoren kann einen wesentlichen Einfluss auf verschiedene biologische Mechanismen in der Zelle haben.

Es besteht daher im Stand der Technik das Bedürfnis, insbesondere bei der Behandlung von Krankheiten, Moleküle zu entwickeln, die spezifische Protein-Protein-Wechselwirkungen hemmen, um biologisch bzw. medizinisch relevante technische Effekte zu erzielen.

Ein Beispiel für solche Inhibitoren sind Struktur-Mimetika von Diprolin-Einheiten. Im Vergleich zu anderen Aminosäuren nimmt Prolin auf Basis seiner chemischen Struktur eine Sonderrolle ein. Prolin ist die einzige sekundäre, proteinogene Aminosäure, die nach Knüpfung einer Peptidbindung über kein freies NH-Proton zur Ausbildung von Wasserstoffbrückenbindungen verfügt. Aus diesem Grund sind Polyprolin-Sequenzen nicht in der Lage klassische alpha-Helix oder beta-Faltblatt Sekundärstrukturen einzunehmen. Aufgrund dieser besonderen physikalischen Eigenschaften sind Polyprolin-Aminosäuresequenzen (z. B. Prolin-reiche Motive (PRM)) sehr gut geeignet, spezifische Protein-Protein-Wechselwirkungen mit Rezeptoren (z. B. Prolin-reiche Motive-bindende Domänen (PBD)) zu formieren. Durch die Bereitstellung von Polyprolin-Mimetika können einige dieser Wechselwirkungen selektiv gehemmt werden.

In der WO 2008/040332, WO 2013/030111 und WO 2016/092069 sind weitere chemische Verbindungen offenbart, die als Diprolin-Mimetika funktionieren. Beispielsweise werden in WO 2013/030111 Peptidverbindungen offenbart, die zwei aneinandergrenzende Diprolin-Mimetika anstelle von vier Prolin-Aminosäuren aufweisen, wobei die Diprolin-Mimetika von Peptidsequenzen flankiert sind. Die darin beschriebenen Peptide zeigen eine gute Affinität zu einer VASP-EVH1-Domäne, sind jedoch auf Basis ihrer besonderen Peptid-Struktur aufwändig herzustellen. Die Peptidstrukturen werden weiterhin aufgrund der Peptidstruktur und der Anwesenheit von endogenen Proteasen ggf. *in vivo* abgebaut. Einige dieser Verbindungen zeigen ebenfalls eine suboptimale Zellpermeabilität, die für die Anwendung als Medikament durchaus nachteilhaft ist. In Reuter et al (Eur. J. Org. Chem., 2014, 13, 2664-2667) wird eine ähnliche Verbindung wie in der WO 2008/040332 offenbart, die eine bizyklische Ringstruktur aufweist, nämlich ein "Westring-geöffnetes" ProM-1-Derivat.

Obwohl die darin offenbarten Verbindungen Protein-Protein-Wechselwirkungen durch selektive Bindung an einen Rezeptor inhibieren können, besteht weiterhin das Bedürfnis, weitere Polyprolin-Mimetika bereitzustellen, die verbesserte Bindungseigenschaften und entsprechende biologische Wirkungen aufweisen.

## DETAILLIERTE DARSTELLUNG DER ERFINDUNG

Im Lichte des Standes der Technik war es daher Aufgabe der Erfindung, Verbindungen bereitzustellen, die als Mimetika für polyprolinreiche Peptide verwendet werden können, insbesondere als pharmazeutische Wirkstoffe für die Behandlung von verschiedenen Krankheiten. Eine weitere Aufgabe der Erfindung war die Bereitstellung von neuartigen Krebsmedikamenten, die effektiv gegen die Invasion und Motilität von Krebszellen wirken.

Das erfindungsgemäße Problem wird gelöst durch die Bereitstellung einer Verbindung gemäß Formel I:

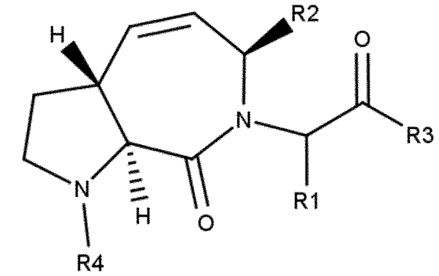

wobei

R1: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise einen R-Rest einer Aminosäure, wobei die genannten Reste optional substituiert sind,

R2: Alkyl, Aryl, Alkoxyalkyl, Cycloalkyl, Mercaptoalkyl, Sulfidoalkyl, Arylalkyl, Fluoralkyl, wobei die genannten Reste optional substituiert sind,

R3: OH, Alkyl, O-Alkyl, O-Aryl, NRR' (R, R': H oder Alkyl), NROR' (R, R': H, Alkyl), Heteroaryl,

R4: H, Alkyl, Acyl, Aryl, Heteroaryl, Cycloalkyl, Alkylheteroaryl, Alkylcarbonyl, Arylalkyl, Arylcarbonyl, Alkoxycarbonyl, Aminocarbonyl, Aryloxycarbonyl, Alkylsulfonyl, Arylsulfonyl, Aminoacyl und/oder Peptidyl, oder R5, wobei die genannten Reste optional substituiert sind,

R5:

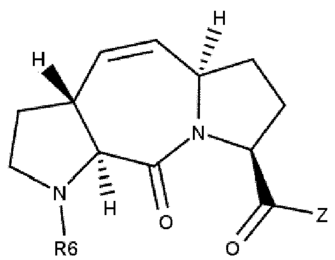

or

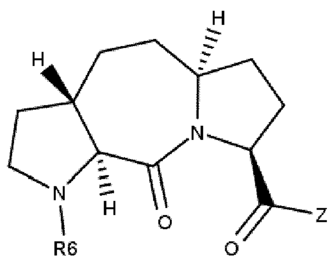

or

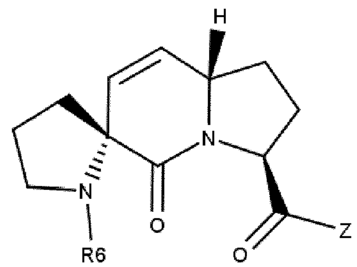

Z: Bindung zur Struktur gemäß Formel I,

R6: H, Alkyl, Aryl, Cycloalkyl, Alkylcarbonyl, Arylcarbonyl, Alkoxycarbonyl, Arylalkyl, Aminocarbonyl, Aryloxycarbonyl, Alkylsulfonyl, Arylsulfonyl, Aminoacyl und/oder Peptidyl, wobei die genannten Reste optional substituiert sind, oder

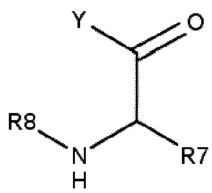

Y: Bindung zur Struktur gemäß R5,

R7: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise ein R-Rest einer Aminosäure, wobei die genannten Reste optional substituiert sind,

R8: H, Alkyl, Aryl, Acyl, Heteroaryl, Cycloalkyl, Alkyl-Heteroaryl, Alkylcarbonyl, Arylalkyl, Arylcarbonyl, Alkoxycarbonyl, Aminocarbonyl, Aryloxycarbonyl, Alkylsulfonyl, Arylsulfonyl, Aminoacyl und/oder Peptidyl, wobei die genannten Reste optional substituiert sind.

Die erfindungsgemäßen Verbindungen weisen überraschend gute Bindungseigenschaften auf.

Wie unten ausführlich dargestellt wird, betrifft die Erfindung Polyprolin-Mimetika ähnlich zu WO 2008/040332, WO 2013/030111 und WO 2016/092069, wobei jedoch eine Ring-Substruktur "geöffnet wurde". Die Scaffolds der vorliegenden Erfindung werden in einige Ausführungsformen als ProM-15 benannt, wobei die früheren Generationen unterschiedliche ProM-Zahlen (z. B. ProM-1, -2 oder-9) tragen, z. B.:

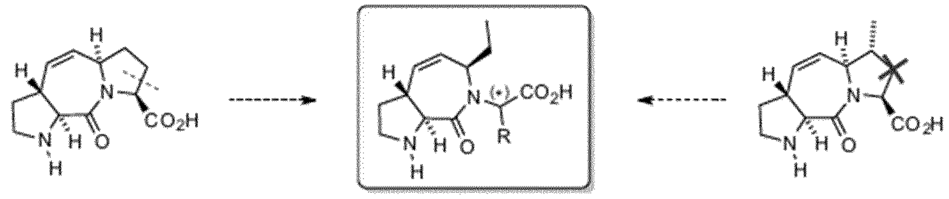

ProM-1 ProM-15 ProM-9

ProM-15 kann daher als ein "Ostring-geöffnetes" ProM-1-Derivat oder als ein, um ein C-Atom reduziertes, ProM-9-Derivat verstanden werden. Die Strukturen der Formel I können als "ProM-15" Strukturen oder Derivate benannt werden.

Einerseits zeigen Computer-Modeling-Studien, dass eine gesteigerte Flexibilität des C-Terminus von ProM-1 eine vielversprechende Option zur Erhöhung der Bindungsaffinität an die Ena/VASP-EVH1-Domäne durch eine mögliche Ausbildung von Wasserstoffbrücken darstellen würde. Die Strukturen der vorliegenden Erfindung weisen in der Tat eine erhöhte Affinität zum die Ena/VASP-EVH1 -Target auf.

Andererseits bietet ProM-15 die Möglichkeit, unter Reduktion des Molekulargewichtes, den Methylsubstituenten von ProM-9 zu "imitieren" und somit die zuvor erfolgreich adressierte hydrophobe Bindungstasche ebenfalls zu nutzen. ProM-15 wird dementsprechend dadurch gekennzeichnet, dass die positiven Bindungseigenschaften von ProM-9 mit einer Verringerung des Molekulargewichtes und einer Erhöhung der C-terminalen Flexibilität vereint werden.

In einer weiteren Ausführungsform betrifft die Erfindung eine Verbindung gemäß Formel II-a oder Formel II-b:

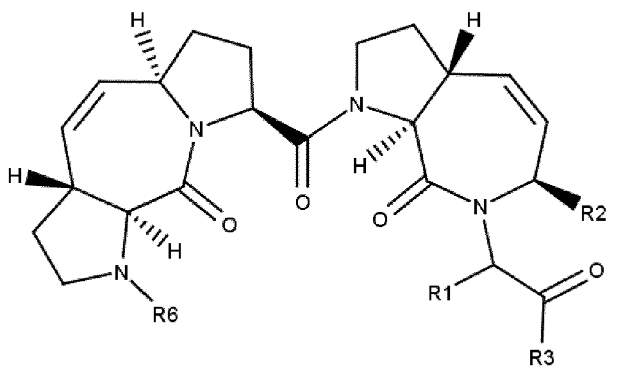

Formel II-a

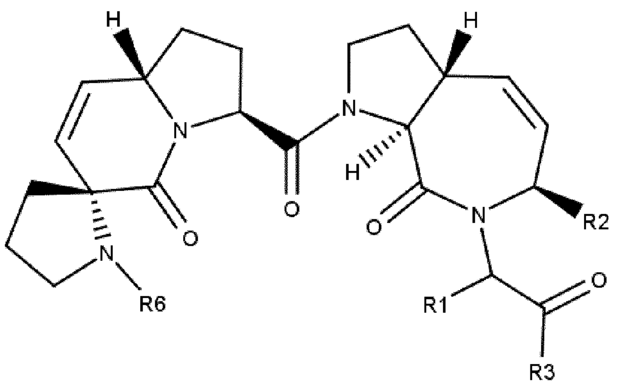

Formel II-b

wobei

R1: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise einen R-Rest einer Aminosäure, wobei die genannten Reste optional substituiert sind,

R2: Alkyl, Aryl, Alkoxyalkyl, Cycloalkyl, Mercaptoalkyl, Sulfidoalkyl, Arylalkyl, Fluoralkyl

R3: OH, Alkyl, O-Alkyl, O-Aryl, NRR' (R, R': H oder Alkyl), NROR' (R, R': H, Alkyl), Heteroaryl,

R6: H, Alkyl, Aryl, Cycloalkyl, Alkylcarbonyl, Arylcarbonyl, Alkoxycarbonyl, Arylalkyl, Aminocarbonyl, Aryloxycarbonyl, Alkylsulfonyl, Arylsulfonyl, Aminoacyl und/oder Peptidyl, wobei die genannten Reste optional substituiert sind, oder

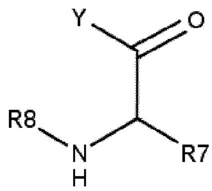

Y: Bindung zur Struktur gemäß Formel II-a oder II-b,

R7: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise ein R-Rest einer Aminosäure, wobei die genannten Reste optional substituiert sind,

R8: H, Alkyl, Aryl, Acyl, Heteroaryl, Cycloalkyl, Alkyl-Heteroaryl, Alkylcarbonyl, Arylalkyl, Arylcarbonyl, Alkoxycarbonyl, Aminocarbonyl, Aryloxycarbonyl, Alkylsulfonyl, Arylsulfonyl, Aminoacyl und/oder Peptidyl, wobei die genannten Reste optional substituiert sind.

In einer weiteren Ausführungsform betrifft die Erfindung eine Verbindung gemäß Formel II-c,

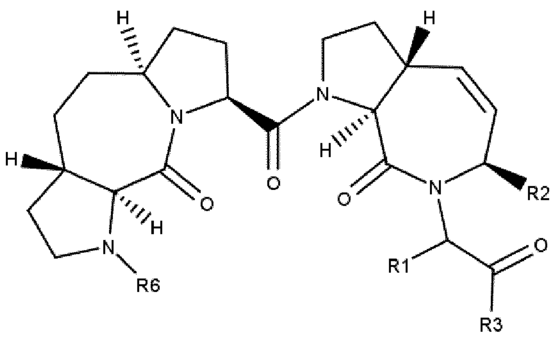

Formel II-c

wobei die Substituenten R1-R8 die gleichen sind wie oben für Formel II-a oder II-b.

In einer Ausführungsform betrifft die Erfindung eine Verbindung wie hierin beschrieben, dadurch gekennzeichnet, dass R7: CH2-aryl ist, insbesondere substituiertes -CH2-Phenyl, wobei der Substituent an der ortho-Position der Phenylgruppe positioniert ist, wobei der Substituent bevorzugt Halogen ist, insbesondere Cl.

In einer Ausführungsform betrifft die Erfindung eine Verbindung wie hierin beschrieben, dadurch gekennzeichnet, dass R6: N-Acyl-(2-chlorphenyl)alaninoyl.

In einigen Ausführungsformen der Erfindung bedeutet "optional substituiert" ein oder mehrere Wasserstoffatome der Kohlenwasserstoffgruppe durch einen oder mehrere Substituenten ersetzt sind, ausgewählt aus der Gruppe bestehend aus Halogen (vorzugsweise Fluor, Chlor, Brom und Iod), -OH, -CN, -SH, Amin (vorzugsweise -NH2, -NHCH3), -NO2, und -Alkyl, wobei Alkyl gegebenenfalls mit einem oder mehreren Substituenten optional substituiert ist; wobei vorzugsweise 1, 2, 3 oder 4 optionale Substituenten vorhanden sind. Diese Ausführungsformen der "optionalen Substitution" gelten für alle Ausführungsformen, die den Begriff "optional substituiert" benennen und/oder umfassen.

In einer Ausführungsform betrifft die Erfindung eine Verbindung wie hierin beschrieben, dadurch gekennzeichnet, dass R1: ein R-Rest einer Aminosäure, der sich von einer natürlichen (kanonischen) oder unnatürlichen D- oder L-konfigurierten Aminosäure ableitet.

In einer Ausführungsform betrifft die Erfindung eine Verbindung wie hierin beschrieben, dadurch gekennzeichnet, dass R2: $CH_2OMe$, $CH_2SMe$, $CH_2CH_2OR$, $CH_2CH_2SR$ (R: C1-C4 Alkyl oder H), benzyl, $CH_2$-benzyl, oder $CH_2CH_2$-benzyl.

In einer Ausführungsform betrifft die Erfindung eine Verbindung wie hierin beschrieben, dadurch gekennzeichnet, dass R2: C1-C4 Alkyl, vorzugsweise Methyl, Ethyl, Propyl, Iso-Propyl, Butyl, Iso-Butyl, Sec-Butyl. In einer Ausführungsform ist Ethyl als R2 vom Schutzbereich ausgeschlossen.

In einer Ausführungsform betrifft die Erfindung eine Verbindung wie hierin beschrieben, dadurch gekennzeichnet, dass R2: Methyl, Propyl, Iso-Propyl, Butyl, Iso-Butyl, Sec-Butyl.

In bevorzugten Ausführungsformen der Erfindung ist R2 Iso-Butyl oder Propyl, z.B. in den hier beschriebenen Derivaten ProM-17 (R2: Iso-Butyl) oder ProM-21 (R2: Propyl). Entsprechende Substanzen und Beispiele sind unten aufgeführt. Solche Verbindungen unterscheiden sich von ProM-15 an der R2-Position (in ProM-15 R2: Ethyl). In weiteren bevorzugten Ausführungsformen sind in den ProM-15, ProM-17 und/oder ProM-21-Scaffolds R1: H, R3: OMe und R4: H bzw. Boc (tert-Butyloxycarbonyl; Boc-Schutzgruppe).

In einer Ausführungsform betrifft die Erfindung eine Verbindung wie hierin beschrieben, dadurch gekennzeichnet, dass wenn R3: OH, die Verbindung als Salz vorliegt.

In einer Ausführungsform betrifft die Erfindung eine Verbindung wie hierin beschrieben, dadurch gekennzeichnet, dass R4, R6: können gleich oder unterschiedlich sein, H, Alkyl, Aryl, Acyl, Peptidyl, Alkylsulfonyl, Arylsulfonyl, Heteroaryl, wobei die genannten Reste optional substituiert sind.

In einer weiteren Ausführungsform betrifft die Erfindung eine Verbindung gemäß Formel II-a, II-b, oder II-c, dadurch gekennzeichnet, dass R6:

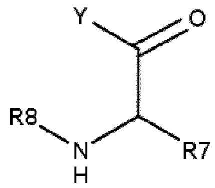

Y: Bindung zur Struktur gemäß Formel II-a oder II-b,

R7: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise ein R-Rest einer Aminosäure, wobei die genannten Reste optional substituiert sind,

R8: H, Alkyl, Aryl, Acyl, Peptidyl, Alkylsulfonyl, Arylsulfonyl, Heteroaryl, wobei die genannten Reste optional substituiert sind.

In einer weiteren Ausführungsform betrifft die Erfindung eine Verbindung gemäß Formel II-a, II-b, oder II-c, dadurch gekennzeichnet, dass R6:

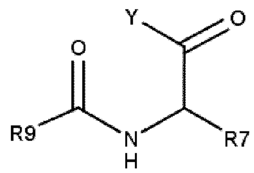

wobei

Y: Bindung zur Struktur gemäß Formel II-a oder II-b,

R7: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise ein R-Rest einer Aminosäure, wobei die genannten Reste optional substituiert sind,

R9: H, Alkyl oder ein optional substituierter Alkylrest.

In einer Ausführungsform der Erfindung, weist die erfindungsgemäße Verbindung, die ProM-15 enthält, eine Struktur gemäß Formel I-a auf, nämlich:

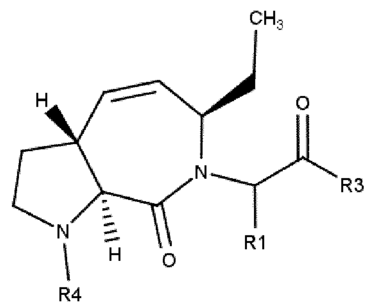

In einer Ausführungsform der Erfindung, weist die erfindungsgemäße Verbindung, die ProM-17 enthält, eine Struktur gemäß Formel I-b auf, nämlich:

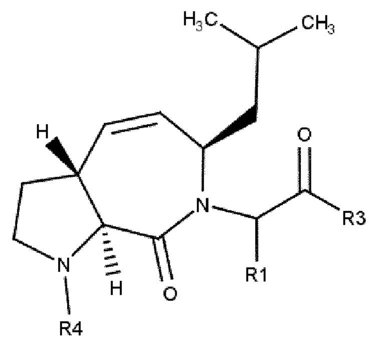

In einer Ausführungsform der Erfindung, weist die erfindungsgemäße Verbindung, die ProM-21 enthält, eine Struktur gemäß Formel I-c auf, nämlich:

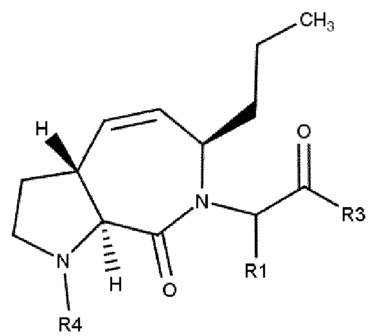

In weiteren Ausführungsformen der Erfindung mit Strukturen gemäß den Formeln I-a, I-b und I-c gelten die Definitionen der Gruppen R4, R1 und R3 wie für Ausführungsformen mit Strukturen gemäß Formel I, wie oben beschrieben.

In weiteren Ausführungsformen der Erfindung weisen die erfindungsgemäßen Verbindungen Strukturen gemäß der Formeln II-a oder II-b auf, wobei R2 Ethyl, Iso-Butyl oder Propyl ist, z.B. wie in den hier beschriebenen Derivaten ProM-15 (R2: Ethyl), ProM-17 (R2: Iso-Butyl) oder ProM-21 (R2: Propyl). Diese Ausführungsformen entsprechen den Formeln I-a, I-b und I-c.

In einer Ausführungsform betrifft die Erfindung eine Verbindung gemäß Formel III:

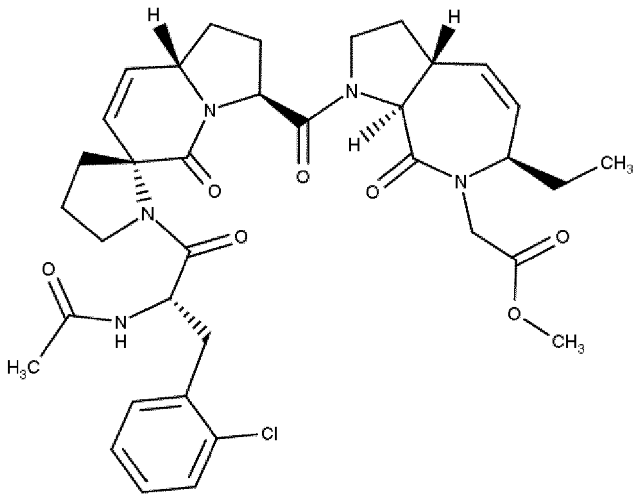

Die Verbindung gemäß Formel III wird mit der hierin verwendeten systematischen Nomenklatur als "Ac-[2Cl-Phe]-[ProM-2]-[ProM-15]-OMe" bezeichnet, wobei gemäß Formel II-a oder II-b R6: Ac-[2Cl-Phe], oder Ac-[L-2Cl-F], R1: H, R2: Ethyl und R3: OMe ist. In den Beispielen wird diese Verbindung als 247 benannt.

Die Erfindung betrifft ebenfalls weitere Verbindungen, welche die Struktur der ProM-15-Grundstruktur (z. B. Formel I) aufweisen, und an andere ProM-Scaffolds (ProM-1 to 10, vorzugsweise ProM-1, -2, -4, -6 und -9, oder Derivate davon) gekoppelt sind. Beispiele für weitere ProM-Scaffolds sind unten erwähnt. Eine solche Kopplung erfolgt vorzugsweise durch die gleiche Bindung wie in den Formlen II-a, -b, c- und III dargestellt wird. Weitere erfindungsgemäßen Strukturen sind durch die Formeln IV bis VIII dargestellt.

In einer Ausführungsform betrifft die Erfindung eine Verbindung gemäß Formel IV:

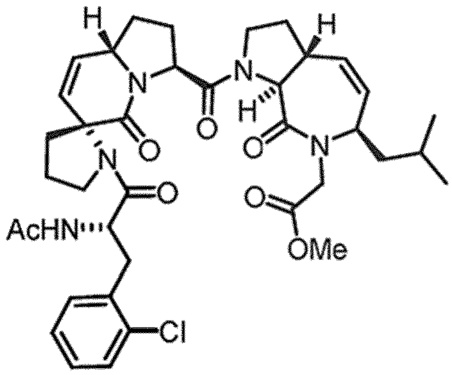

Die Verbindung gemäß Formel IV wird mit der hierin verwendeten systematischen Nomenklatur als "Ac-[L-2Cl-F]-[ProM-2]-[ProM-17]-OMe" bezeichnet.

In einer Ausführungsform betrifft die Erfindung eine Verbindung gemäß Formel V:

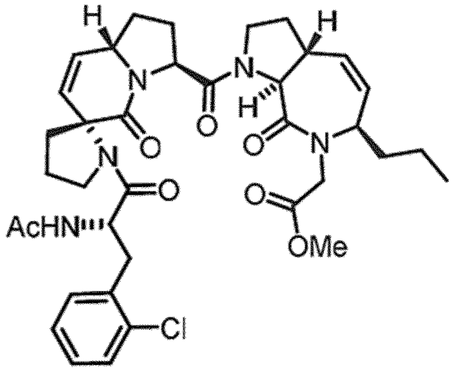

Die Verbindung gemäß Formel V wird mit der hierin verwendeten systematischen Nomenklatur als "Ac-[L-2Cl-F]-[ProM-2]-[ProM-21]-OMe" bezeichnet.

In einer Ausführungsform betrifft die Erfindung eine Verbindung gemäß Formel VI:

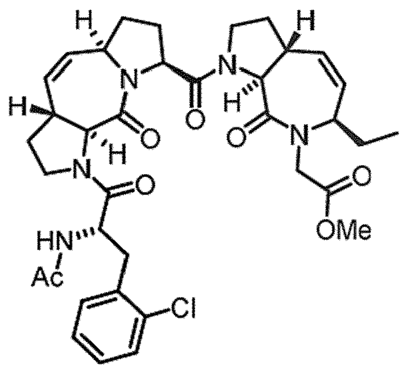

Die Verbindung gemäß Formel VI wird mit der hierin verwendeten systematischen Nomenklatur als "Ac-[L-2Cl-F]-[ProM-1]-[ProM-15]-OMe" bezeichnet.

In einer Ausführungsform betrifft die Erfindung eine Verbindung gemäß Formel VII:

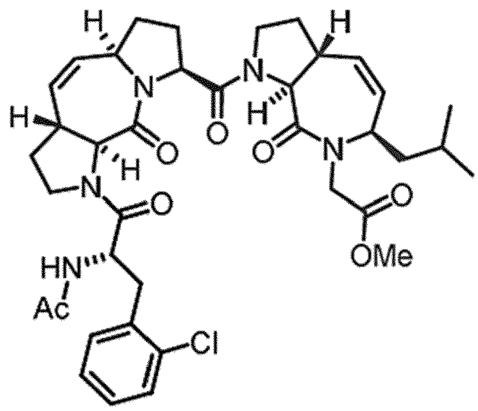

Die Verbindung gemäß Formel VII wird mit der hierin verwendeten systematischen Nomenklatur als "Ac-[L-2Cl-F]-[ProM-1]-[ProM-17]-OMe" bezeichnet.

In einer Ausführungsform betrifft die Erfindung eine Verbindung gemäß Formel VIII:

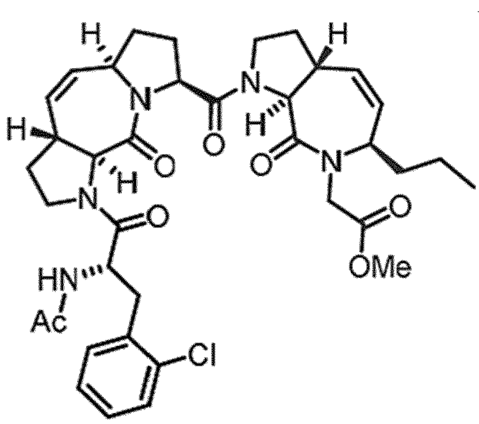

Die Verbindung gemäß Formel VIII wird mit der hierin verwendeten systematischen Nomenklatur als "Ac-[L-2Cl-F]-[ProM-1]-[ProM-21]-OMe" bezeichnet.

Die unten aufgeführten Beispiele der Anmeldung zeigen, dass die erfindungsgemäßen Verbindungen eine erhöhte Bindungsaffinität an die Ena/VASP-EVH1-Domäne aufweisen. Dies könnte auf einer gesteigerten Flexibilität des C-Terminus von ProM-1 sowie einer möglichen Ausbildung von Wasserstoffbrücken beruhen. Die Strukturen der

vorliegenden Erfindung (zumindest Verbindungen gemäß Formel III, IV, V, VI, VII, und VIII; Tabellen 2 und 3) haben im Vergleich zum Stand der Technik (z.B. [ProM-2]-[ProM-1] wie in der WO 2016/092069) eine erhöhte Affinität zum Ena/VASP-EVH1-Target.

Die Strukturen gemäß ProM-15, -17, -21 und ggf. weitere ähnliche Konfigurationen bieten die Möglichkeit, unter Reduktion des Molekulargewichtes, den Methylsubstituenten von ProM-9 zu "imitieren" und somit die zuvor erfolgreich adressierte hydrophobe Bindungstasche zu nutzen. Die erfindungsgemäßen Strukturen werden dementsprechend dadurch gekennzeichnet, dass die positiven Bindungseigenschaften von ProM-9 mit einer Verringerung des Molekulargewichtes und einer Erhöhung der C-terminalen Flexibilität vereint werden. Es wäre für einen durchschnittlichen Fachmann im Lichte des Standes der Technik nicht möglich gewesen, diese funktionellen Verbesserungen auf Basis der erfindungsgemäßen Strukturen vorherzusagen.

In einer Ausführungsform betrifft die Erfindung eine Verbindung wie hierin beschrieben, dadurch gekennzeichnet, dass sie als Ligand für eine Ena/VASP-EVH1-Domäne funktioniert oder verwendet wird.

In einer Ausführungsform betrifft die Erfindung eine Verbindung wie hierin beschrieben, die eine Ena/VASP-EVH1-vermittelte Protein-Protein-Wechselwirkung inhibiert.

In einer Ausführungsform wird die erfindungsgemäße Verbindung dadurch gekennzeichnet, dass die Verbindung eine Dissoziationskonstante (Kd) im Komplex mit einer Ena/VASP-EVH1-Domäne von $\leq 5\ \mu M$, bevorzugt $\leq 2\ \mu M$, weiterhin bevorzugt $\leq 500$ nM aufweist.

Die Erfindung betrifft weiterhin eine pharmazeutische Zusammensetzung umfassend eine Verbindung wie hierin beschrieben, bevorzugt mit einem pharmazeutisch annehmbaren Träger.

Die Erfindung betrifft weiterhin eine Verbindung oder Zusammensetzung wie hierin beschrieben zur Verwendung als Medikament zur Behandlung von Tumorerkrankungen. In einer Ausführungsform ist die Tumorerkrankung Brustkrebs.

Die Erfindung betrifft weiterhin eine Verbindung oder Zusammensetzung wie hierin beschrieben zur Verwendung als Medikament zur Behandlung von Tumorerkrankungen, dadurch gekennzeichnet, dass die Verbindung die Metastasierung eines Tumors inhibiert.

Die Erfindung betrifft weiterhin eine Verbindung oder Zusammensetzung wie hierin beschrieben zur Verwendung als Medikament zur Behandlung von Tumorerkrankungen, dadurch gekennzeichnet, dass die Metastasierung eines Tumors durch Inhibition der Chemotaxis und/oder Motilität der Tumorzellen auf Basis einer Störung der Aktinfilamentsynthese inhibiert wird.

Die Erfindung betrifft Verbindungen als Ligand für eine Ena/VASP-EVH 1-Domäne. Die EVH1-Domäne besteht aus etwa 115 Aminosäuren und kommt in einer Vielzahl signalgebender Multidomänenproteine vor. Neben einigen weiteren gehört dazu auch die Familie der Ena/VASP-Proteine, die als molekulare Adaptoren wirken und die Actindynamik des Cytoskeletts modulieren. Man unterteilt die EVH1-Domänen nach ihrer Ligandenpräferenz in drei Klassen. Die erste Klasse erkennt insbesondere spezifisch ein [F/W/L]PPPP-Kernmotiv, das sich in fokalen Adhäsionsproteinen wie zum Beispiel Vinculin, Zyxin oder RIAM, in Lamellipodin, einem wichtigen Protein des Leitsaums, in einigen Proteinen des WAVE-Komplexes, wie auch im ActA-Protein des intrazellulären Bakteriums *Listeria monocytogenes* wiederfindet. Ena/VASP Proteine werden außerdem in Filopodien und Invadopodien nachgewiesen, wo sie mit ihrer EVH1-Domäne mit Forminen wechselwirken.

In einem weiteren Aspekt betrifft die Erfindung die Verwendung der erfindungsgemäßen Verbindungen als Ligand für eine Domäne ausgewählt aus der Gruppe umfassend Src-Homologie-3-Domänen, WW-Domänen, GYF-Domänen, UEV-Domänen und/oder Profilin.

Die Verbindung der vorliegenden Erfindung ist in einer Ausführungsform dadurch gekennzeichnet, dass die Verbindung eine Ena/VASP-EVH1-vermittelte Protein-Protein-Wechselwirkung inhibiert. Demgemäß betrifft die Erfindung auch die Verwendung der neuen Strukturmimetika der PolyprolinII-Helix als überraschend gute Inhibitoren von Protein-Protein-Wechselwirkungen, bei denen insbesondere EVH1-Domänen, SH3-Domänen, WW-Domänen, GYF-Domänen, UEV-Domänen und Profilin beteiligt sind. Proteine, die diese Domänen enthalten, wie z. B. VASP (EVH1-Domäne) oder YAP (WW) spielen u. a. bei der Regulation der Zellmotilität (insbesondere VASP) und der Zellproliferation (insbesondere YAP) eine bedeutsame Rolle. So ist beispielsweise VASP in hochinvasiven Brustkrebszellen stark überexprimiert.

Die Verbindung der vorliegenden Erfindung ist in einer Ausführungsform dadurch gekennzeichnet, dass die Verbindung eine Dissoziationskonstante (Kd) im Komplex mit einer Ena/VASP-EVH1-Domäne von $\leq 20\ \mu M$, bevorzugt $\leq 5$ oder $2\ \mu M$, weiterhin bevorzugt $\leq 500$ nM aufweist. Die besonders niedrigen Dissoziationskonstanten stellen ein überraschendes Ergebnis dar. Es war vorher weder bekannt noch im Stand der Technik erwähnt, dass Polyprolin-Mimetika solche Bindungseigenschaften an EVH1-Domänen aufweisen können.

In einem weiteren Aspekt betrifft die Erfindung eine pharmazeutische Zusammensetzung umfassend eine oder mehrere erfindungsgemäße Verbindungen, bevorzugt mit einem pharmazeutisch annehmbaren Träger. Bevorzugte pharmazeutische Träger sind beispielsweise Füllmittel, Streckmittel, Bindemittel, Feuchthaltemittel, Lösungsverzögerer, Sprengmittel, Resorptionsbeschleuniger, Netzmittel, Absorptionsmittel und/oder Gleitmittel.

Die Erfindung betrifft die Verwendung der genannten Verbindungen als pharmazeutische Wirkstoffe. Die Verwendung als pharmazeutischer Wirkstoff betrifft die erfindungsgemäßen Verbindungen zur Verwendung für chirurgische, therapeutische oder diagnostische Verfahren. Die Erfindung betrifft in einem weiteren Aspekt ein pharmazeutisches Mittel, das die erfindungsgemäßen Verbindungen gegebenenfalls zusammen mit einem pharmazeutisch verträglichen Träger umfasst.

In einer bevorzugten Ausführungsform der Erfindung werden die Verbindungen als Poly-Prolin-Mimetika eingesetzt. Vorteilhafterweise findet man prolinreiche Aminosäurensequenzen insbesondere in Peptiden, die an Signaltransduktionsvorgängen, insbesondere intrazellulären Signaltransduktionsvorgängen beteiligt sind. Im Sinne der Erfindung kann der Begriff der Mimetika auch als Analoga verstanden werden. Bevorzugt ist die Verwendung der erfindungsgemäßen Verbindungen zur Behandlung von Krankheiten, die mit einer Modifikation von intrazellulären Signaltransduktionsvorgängen assoziiert sind, die durch Poly-Prolin-Helix-Strukturen vermittelt werden, ausgewählt aus der Gruppe umfassend Tumorerkrankungen, bakterielle Infektionskrankheiten und/oder neurodegenerative Erkrankungen.

In einem weiteren Aspekt betrifft die Erfindung die Verwendung der erfindungsgemäßen Verbindungen als Medikament zur Behandlung von Tumorerkrankungen. In einer bevorzugten Ausführungsform der Erfindung ist die Tumorerkrankung Brustkrebs.

Die Verwendung einer erfindungsgemäßen Verbindung als Medikament zur Behandlung einer Tumorerkrankung wird vorzugsweise dadurch gekennzeichnet, dass die Verbindung die Metastasierung eines Tumors, bevorzugt durch Inhibition der Chemotaxis und/oder Motilität der Tumorzellen auf Basis einer Störung der Aktinfilamentsynthese, inhibiert.

Es war völlig überraschend, dass die erfindungsgemäßen Verbindungen eine besonders gute Wirksamkeit gegen die Chemotaxis und/oder Motilität von Tumorzellen zeigen würden. Die Hemmung der Chemotaxis und/oder Motilität von Tumorzellen spielt daher insbesondere eine Rolle bei der Behandlung und/oder Prävention von Tumormetastasen. Dies stellt einen unterschiedlichen technischen Effekt im Vergleich zur Hemmung der Zellproliferation dar.

Aktin ist ein Strukturprotein, das in allen eukaryotischen Zellen vorkommt. Es ist Bestandteil des Zytoskeletts und eines der fünf häufigsten Proteine in Eukaryoten. Aktin bildet aneinandergereiht als F-Aktin dynamische Aktinfilamente. Diese Mikrofilamente dienen der Stabilisierung der äußeren Zellform, der Ausbildung von Zellfortsätzen, intrazellulären Verlagerungen und gerichteten zellulären Bewegungen. Viele eukaryotische Zellen besitzen ein hohes Maß an Bewegungsfähigkeit, Zellmotilität oder auch Zellmigration genannt, um beispielsweise Eindringlinge im Körper unschädlich machen zu können (Zellen des Immunsystems), Wunden heilen zu können (z. B. Hautzellen) oder um allgemein Zellen zu bewegen (in der Entwicklung oder bei einzelligen Organismen wie z. B. Amöben). Die Zellmigration spielt ebenfalls eine wesentliche Rolle bei Krebsmetastasen. Diese Beweglichkeit beruht hauptsächlich auf zwei Prozessen: Der gerichteten Aktinpolymerisierung in die Bewegungsrichtung (geregelt durch eine Anzahl von Regulatoren die auf Signale von der Zellperipherie reagieren), und der Aktin-Myosin-Interaktion in Fibrillenbündeln (stress fibers), kontraktile Zugseile, die durch die Zelle verlaufen und formgebende Elemente mit der Unterlage verspannen.

Die von den erfindungsgemäßen Verbindungen hervorgerufene Störung der Aktinfilamentsynthese stellt ebenfalls einen neuen und vorteilhaften technischen Effekt dar. Durch eine Störung der Aktinfilamentsynthese können die erfindungsgemäßen Verbindungen die Motilität der Krebszellen inhibieren und somit die Krebsmetastasierung stark hemmen bzw. dieser vorbeugen.

Die bevorzugten Tumorerkrankungen sind ausgewählt aus der Gruppe umfassend Tumorerkrankungen des Hals-Nasen-Ohren-Bereichs, der Lunge, des Mediastinums, des Gastrointestinaltraktes, des Urogenital-Systems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppressionbezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs; das Melanom; das Hepatom; das Neuroblastom; das Papillom; das Apudo;, das Choristom; das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, Brown-Pearce-Karzinom, duktales Karzinom, Ehrlich-Tumor, in-situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchioloalveoläres Karzinom, Bronchial-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronischlymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom; Chordom; Craniopharyngiom; Dysgerminom;

Hamartom; Mesenchymom; Mesonephrom; Myosarkom; Ameloblastom; Cementom; Odontom; Teratom; Thymom; Chorio-blastom; Adenokarzinom; Adenom; Cholangiom; Cholesteatom; Cylindrom; Cystadeno-carcinom; Cystadenom; Granulosa-zelltumor; Gynadroblastom; Hidradenom; Inselzelltumor; Leydig-Zelltumor; Papillom; Sertoli-Zell-Tumor; Thekazell-tumor; Leiomyom; Leiomyosarkom; Myoblastom; Myom; Myosarkom; Rhabdomyom; Rhabdomyosarkom; Ependynom; Ganglioneurom; Gliom; Medulloblastom; Meningiom; Neurilemmom; Neuroblastom; Neuroepitheliom; Neurofibrom; Neurom; Paragangliom; nicht-chromaffines Paragangliom; Angiokeratom; angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangio-myom, Lymphangiosarkom; Pinealom; Cystosarkom phyllodes; Hemangiosarkom; Lymphangiosarkom; Myxosarkom; Ovarialkarzinom; Sarkom (zum Beispiel Ewing-Sarkom, experi-mentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des ürogenitaltrakts); Neuro-fibromatose und zervikale Plattenepitheldysplasie.

In einer weiter bevorzugten Ausführungsform ist die Krebserkrankung oder der Tumor ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungen-metastasen, Lebermetastasen, Knochenmetastasen, pleurale und perikardiale Metastasen und maligne Aszites.

In einer weiter bevorzugten Ausführungsform ist die Krebserkrankung oder der Tumor ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarm-krebs, der Ovarialkarzinome, der Zervikalkarzinome, Lungenkrebs, Prostatakrebs, Nierenzellkarzinome und/oder Lebermetastasen.

Bei der Behandlung der genannten Krankheiten ist es besonders bevorzugt, das pharmazeutische Mittel, welches die erfindungsgemäßen Verbindungen umfasst, als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zuzubereiten und/oder anzuwenden.

Bevorzugt liegt das pharmazeutische Mittel, welches die erfindungsgemäßen Verbindungen umfasst, in einer Konzentration von 0,1 bis 99,5, bevorzugt von 0,5 bis 95,0, besonders bevorzugt von 20,0 bis 80,0 Gew.-% in einer Zubereitung vor.

Bevorzugt wird diese Zubereitung oral, subkutan, intravenös, intramuskulär, intraperitoneal und/oder topisch gesetzt.

Bevorzugt wird das pharmazeutische Mittel, das die erfindungsgemäßen Verbindungen enthält, in Gesamtmengen von 0,05 bis 500 mg pro kg, bevorzugt von 5 bis 100 mg pro kg Körpergewicht, je 24 Stunden eingesetzt.

Bevorzugt erfolgt die Verabreichung der erfindungsgemäßen Verbindung oder pharmazeutischen Zusammensetzung in den Patienten oral, über Injektion, topisch, vaginal, rektal und/oder nasal.

Die Erfindung betrifft auch einen Kit, der mindestens eine der erfindungsgemäßen Verbindungen und/oder eines der erfindungsgemäßen pharmazeutischen Mittel, gegebenenfalls mit einer Information zum Kombinieren der Inhalte des Kits, umfasst - z. B. einen Beipackzettel oder eine Internet-Adresse, die auf Homepages mit weiteren Informationen verweist etc. Die Information zur Handhabung des Kits kann beispielsweise ein Therapie-Schema für die o. g. Krankheiten, insbesondere für die bevorzugten Krankheiten umfassen. Die Information kann jedoch auch Angaben

darüber umfassen, wie die erfindungsgemäßen Erzeugnisse innerhalb einer Diagnose der genannten Krankheiten einzusetzen sind. Der erfindungsgemäße Kit kann auch in der Grundlagenforschung verwendet werden.

Die Erfindung betrifft auch die Verwendung des Kits zur Prophylaxe und/oder Therapie von neurodegenerativen Erkrankungen, bakteriellen Infektionserkrankungen oder Tumorerkrankungen.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. "Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl. Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Beispiele für Heteroaryl sind auch 5- oder 6-gliedrige benzokondensierte Ringe.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst.

Der Begriff "Alkoxy" bedeutet ein über ein Sauerstoffatom gebundenen Alkylrest, Alkenyloxy bedeutet ein über ein Sauerstoffatom gebundenen Alkenylrest, Alkinyloxy bedeutet ein über ein Sauerstoffatom gebundenen Alkinylrest, Cycloalkyloxy bedeutet ein über ein Sauerstoffatom gebundenen Cycloalkylrest und Cycloalkenyloxy bedeutet ein über ein Sauerstoffatom gebundenen Cycloalkenylrest.

Erfindungsgemäß bedeutet "Hydroxyalkyl" eine Alkylgruppe wie oben definiert, die mit einer Hydroxygruppe substituiert ist.

Erfindungsgemäß bedeutet "Arylalkyl" eine Arylgruppe wie oben definiert, die mit einer Alkylgruppe substituiert ist.

Erfindungsgemäß bedeutet "Alkylheteroaryl" eine Alkylgruppe wie obebn definiert, die mit einer Heteroarylgruppe substituiert ist.

Der Begriff "Aminoalkyl" bezeichnet eine Aminogruppe, die über eine Alkylgruppe verknüpft ist. Repräsentative, aber nicht limitierende Beispiele für Aminoalkyl sind Aminomethyl, 2-(Amino)ethyl, Benzyl-(methyl)aminomethyl, Dimethylaminomethyl.

Der Begriff "Aminocarbonyl" bezeichnet eine Aminogruppe, die über eine Carbonylgruppe verknüpft ist. Repräsentative, aber nicht limitierende Beispiele für Aminocarbonyl sind Diethylaminocarbonyl, Benzylaminocarbonyl, Ethylaminocarbonyl.

Der Begriff "Alkylaminocarbonyl" bezeichnet eine Aminocarbonylgruppe, die über eine Alkylgruppe verknüpft ist. Repräsentative, aber nicht limitierende Beispiele für Aminocarbonylalkyl sind 2-Amino-2-oxoethyl, 2-(Benzylamino) -2-oxoethyl, 2-(Methylamine)-2-oxoethyl, 4-Amino-4-oxobutyl, 4-(Dimethylamino)-4-oxobutyl.

Der Begriff "Alkylcarbonyl" bezeichnet eine Alkylgruppe, die über eine Carbonylgruppe verknüpft ist. Repräsentative, aber nicht limitierende Beispiele für Alkylcarbonyl sind Acetyl, 1-Oxopropyl, 2,2-Dimethyl-1-oxopropyl, 1-Oxobutyl, 1-Oxopentyl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Mercaptoalkyl" eine Alkylgruppe mit einem Mercaptan (Thiol)-Substituenten.

Erfindungsgemäß bedeutet "Sulfidoalkyl" ein Alkylderivat des Schwefelwasserstoffs (H2S). Solche Alkylsulfide weisen die Struktur R-S-R' auf, wobei mindestens R oder R' alkyl ist. Sie werden auch als Thioether bezeichnet.

Erfindungsgemäß bedeutet "Alkylsulfonyl" oder "Arylsulfonyl" eine Alkylgruppe bzw. Arylgruppe enthaltend einer Sulfonylgruppe (-SO2-). Diese wird auch als Sulfongruppe bezeichnet. Sulfonylverbindungen gehören zu den organischen Derivaten von Sauerstoffsäuren des Schwefels, bei denen die beiden organischen Reste direkt an das Schwefelatom gebunden sind.

Die Bezeichnung "Peptidyl" bedeutet beispielsweise ein Radikal, gebildet aus einem Peptid, z. B. durch das Entfernen einer $NH_2$-Gruppe von einer Amidgruppe, oder aus einem Teil eines Peptids oder durch mehrere gekoppelte

Aminosäuren, z. B. ein Polypeptid. Die Peptidylgruppe kann daher COCHRxNHRy umfassen, wobei Rx vorzugsweise ein R-Rest einer Aminosäure ist (optional substituiert) und Ry vorzugsweise H, oder eine weitere Peptidylgruppe oder Teil davon (optional substituiert) sein kann. Die Peptidylgruppe ist vorzugsweise eine Struktur, die von der folgenden Formel umfasst ist:

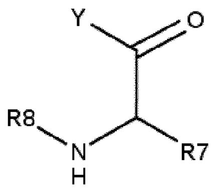

Y: Bindung zur nebenliegenden Struktur (z. B. gemäß R5 der Formel I),

R7: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise ein R-Rest einer Aminosäure, wobei die genannten Reste optional substituiert sind,

R8: H, Alkyl, Aryl, Acyl, Heteroaryl, Cycloalkyl, Alkyl-Heteroaryl, Alkylcarbonyl, Arylalkyl, Arylcarbonyl, Alkoxycarbonyl, Aminocarbonyl, Aryloxycarbonyl, Alkylsulfonyl, Arylsulfonyl, Aminoacyl und/oder Peptidyl, wobei die genannten Reste optional substituiert sind.

In verschiedene Ausführungsformen der Erfindung sind R-Reste einer Aminosäure als mögliche Substituenten vorgesehen, wobei die genannten Reste optional substituiert sind. Die R-Reste einer Aminosäure sind vorzugsweise aus der untengenannten Liste ausgewählt. Eine optionale Substitution der R-Reste ist ebenfalls möglich, z. B. durch Halogen (vorzugsweise Fluor, Chlor, Brom und Iod), -OH, -CN, -SH, Amin (vorzugsweise -NH2, -NHCH3), -NO2, oder-Alkyl, wobei Alkyl gegebenenfalls mit einem oder mehreren Substituenten optional substituiert ist; wobei vorzugsweise 1, 2, 3 oder 4 optionale Substituenten vorhanden sind, vorzugsweise aus der o. g. Liste. Diese Ausführungsformen der "optionalen Substitution" gelten für alle Ausführungsformen, die den Begriff "optional substituiert" benennen und/oder umfassen.

| Aminosäurerest | Abk. | Code | Seitenkette (R-Rest der Aminosäure) |
|---|---|---|---|
| Alanin | Ala | A | - |
| Arginin | Arg | R | -CH2CH2CH2NH-C(NH)NH2 |
| Asparagin | Asn | N | -CH2CONH2 |
| Asparaginsäure | Asp | D | -CH2COOH |
| Cystein | Cys | C | -CH2SH |
| Glutaminsäure | Glu | E | -CH2CH2COOH |
| Glutamin | Gln | Q | -CH2CH2CONH2 |
| Glycin | Gly | G | -H |
| Histidin | His | H | -CH2(C3H3N2) |
| Isoleucin | Ile | I | -CH(CH3)CH2CH3 |
| Leucin | Leu | L | -CH2CH(CH3)2 |
| Lysin | Lys | K | -CH2CH2CH2CH2NH2 |
| Methionin | Met | M | -CH2CH2SCH3 |
| Phenylalanin | Phe | F | -CH2(C6H5) |
| Prolin | Pro | P | -CH2CH2CH2- |
| Serin | Ser | S | -CH2OH |
| Threonin | Thr | T | -CH(OH)CH3 |

(fortgesetzt)

| Aminosäurerest | Abk. | Code | Seitenkette (R-Rest der Aminosäure) |
|---|---|---|---|
| Tryptophan | Trp | W | -CH2(C8H6N) |
| Tyrosin | Tyr | Y | -CH2(C6H4)OH |
| Valin | Val | V | -CH(CH3)2 |

## FIGUREN

**Fig. 1:** Schematischer Aufbau der Ena/VASP-Proteine.

**Fig. 2:** EVH1-Domäne. Die EVH1-Domäne besteht aus einer gedrehten β-Sandwich-Struktur, die zu einer Seite durch eine α-Helix geschlossen wird.

**Fig. 3:** Konzept zur Herstellung konformativ fixierter Diprolinanaloga in idealer PPII-Konformation.

**Fig. 4:** Synthetisierte PPII-Sekundärstrukturmimetika (ProMs). Zu allen abgebildeten ProM-Bausteinen sind die bis heute erschienenen Publikationen angegeben. **58**: J. Zaminer, C. Brockmann, P. Huy, R. Opitz, C. Reuter, M. Beyermann, C. Freund, M. Müller, H. Oschkinat, R. Kühne, H.-G. Schmalz, Angew. Chem. 2010, 122, 7265-7269., **80**: C. Reuter, P. Huy, J.-M. Neudörfl, R. Kühne, H.-G. Schmalz, Chem. Eur. J. 2011, 17, 12037-12044, **77**: C. Reuter, R. Opitz, A. Soicke, S. Dohmen, M. Barone, S. Chiha, M. T. Klein, J.-M. Neudörfl, R. Kühne, H.-G. Schmalz, Chem. Eur. J. 2015, 21, 8464-8470. **90**: C. Reuter, M. Kleczka, S. d. Mazancourt, J.-M. Neudörfl, R. Kühne, H.-G. Schmalz, Eur. J. Org. Chem. 2014, 2664-2667, **91**: A. Soicke, C. Reuter, M. Winter, J.-M. Neudörfl, N. Schlörer, R. Kühne, H.-G. Schmalz, Eur. J. Org. Chem. 2014, 6467-6480, **89**: V. Hack, C. Reuter, R. Opitz, P. Schmieder, M. Beyermann, J.-M. Neudörfl, R. Kühne, H.-G. Schmalz, Angew. Chem. Int. Ed. 2013, 52, 9539-9543, **73**: P. Huy, Dissertation, Universität zu Köln, 2011, **62**: A. Soicke, Dissertation, Universität zu Köln, 2014.

**Fig. 5:** Entwicklung eines Ena/VASP-EVH1 Inhibitors. (Kd = Bindungskonstante, LE = "Ligand efficiency", Da = Dalton). Oben rechts: FPPPP (grün) von 1evh überlagert mit 43 (orange) aus X-ray von EnaH-EVH1 im Komplex mit 43.

**Fig. 6:** Ena/VASP-EVH1 Inhibitor 43 (R. Opitz, M. Müller, C. Reuter, M. Barone, A. Soicke, Y. Roske, K. Piotukh, P. Huy, M. Beerbaum, B. Wiesner, M. Beyermann, P. Schmieder, C. Freund, R. Volkmer, H. Oschkinat, H.-G. Schmalz, R. Kühne, PNAS 2015, 112, 5011-5016).

**Fig. 7:** Modelle der Liganden. Modelle der Liganden Ac-[L-2Cl-F]-[ProM-2]-[ProM-15 (R = Bn)]-OH (links; der rote Pfeil weist auf die ebenfalls durch ProM-9 adressierte hydrophobe Tasche) und Ac-[L-2Cl-F]-[ProM-2]-[ProM-15 (R = (CH2)2CO2H)]-OH (rechts) an der EnaH-EVH1 Domäne (basierend auf der Kristallsturktur von Ac-[L-2Cl-F]-[ProM-2]-[ProM-1]-OH (43) mit EnaH-EVH1.

**Fig. 8:** Die auf Glycin und L-Phenylalanin basierenden PPII-Sekundärstrukturmimetika Boc-R,S,R-Gly-[ProM-15]-OH und Boc-R,S,R,S-Phe-[ProM-15]-OH. Die Buchstaben sind zur Verdeutlichung der Nomenklatur eingezeichnet.

**Fig. 9:** Neuartiger ProM-15-basierter Ena/VASP-EVH1 Inhibitor 115.

**Fig. 10:** Diverser α-Aminsosäure-ester synthetisierten Allylamine. Dargestellt sind die durch Pd-katalysierte N-Allylierung diverser α-Aminsosäure-ester synthetisierten Allylamine und die unter optimierten Bedingungen erreichten Diastereomerenüberschüsse bei Einsatz der chiralen Bisphosphin-Liganden 168 bzw. ent-168 nach Schema 4.15 (betrachtet wurden bei den angegebenen de's die beiden aus der Katalyse resultierenden Diastereomere, bei denen das Aminosäurestereozentrum durch Wahl des entsprechenden N-Nukleophils definiert war).

**Fig. 11:** Kristallstrukturen der Bicyclen Boc-R,S,R-Gly-[ProM-15]-OtBu und Boc-S,R,S-Gly-[ProM-15]-OtBu über den entsprechenden Formelbildern.

**Fig. 12:** Kristallstrukturen der diastereomeren Phe-[ProM-15]-Bicyclen über den entsprechenden Formelbildern.

**Fig. 13:** Kristallstruktur des Dipeptids dia-214-b in zwei Ansichten neben dem entsprechenden Formelbild.

**Fig. 14:** Kristallstruktur des Bicyclus Boc-R,S,R,S-Ala-[ProM-15]-OMe neben dem entsprechenden Formelbild.

**Fig. 15:** Überlagerung der Kristallstrukturen der Liganden 247 und 252 im Komplex mit der EnaH-EVH1-Domäne. Links: Vollständige Ansicht der Bindungsfurche. Rechts: Detaillierte Aufsicht auf ProM-15 und ProM-9.

**Fig. 16:** Kristallstruktur vom ProM-15-Liganden 247 im Komplex mit der EnaH-EVH1-Domäne. Die Elektronendichte der einzelnen Atome ist dargestellt. Die zentrale, stabilisierende Wasserstoffbrücke vom ProM-2-Scaffold zum Trp23 wurde in eingezeichnet, wobei die vermutete, neue Wasserstoffbrücke gestrichelt dargestellt wurde.

**Fig. 17:** Dargestellt sind alle, im Rahmen der Erfindung durch Pd-katalysierte N-Allylierung diverser $\alpha$-Aminsosäureester nach Schema 5.3 synthetisierten Allylamine und die unter optimierten Bedingungen erreichten Enantio- bzw. Diastereomerenüberschüsse und isolierten Ausbeuten.

**BEISPIELE**

**Ena/VASP-Homologie-1-Domänen (EVH1)**

Die aus etwa 115 Aminosäuren bestehenden EVH1-Domänen spielen bei zahlreichen biologischen Prozessen eine Rolle und werden in einer Vielzahl von signalgebenden Multidomänenproteinen gefunden. Bis heute sind drei Familien von EVH1-Domänen bekannt, wobei deren Klassifizierung anhand der Ligandenpräferenz erfolgte.

Die erste Klasse besteht aus den Ena/VASP-Proteinen, die maßgeblich am Auf- und Abbau des Aktin-Cytoskeletts beteiligt sind. Klasse-II-EVH1-Domänen (Homer/Vesl-Familie) werden in postsynaptischen Rezeptor-assoziierten Proteinen gefunden und die dritte Klasse der EVH1-Domänen bilden die an der Actin-aggregation regulierend beteiligten Wiscott-Aldrich-Syndrom-Proteine (WASP).

**Die Ena/VASP-EVH1-Domäne (Klasse-1-EVH1)**

Die Ena/VASP-Proteine (Ena, Mena, VASP, Evl) modulieren die Actindynamik im Cytoskelett und nehmen somit eine Schlüsselrolle im Bereich der Zellmotilität ein. Dadurch beeinflussen sie eine Reihe von wichtigen physiologischen Prozessen, wie z. B. die Entwicklung des Nervensystems oder die Funktion des Immunsystems. Resultierend spielen sie auch bei krankheitsbedingten Vorgängen wie der Tumor Metastasierung eine entscheidende Rolle und stellen somit ein hoch interessantes pharmakologisches "Target", z. B. zur Entwicklung eines Metastaseinhibitors, dar.

Alle Proteine der Ena/VASP-Familie weisen denselben Grundaufbau auf, der schematisch in Fig. 1 gezeigt ist.

Am N-Terminus eines Ena/VASP-Proteins befindet sich die EVH1-Domäne. Ihre Struktur wurde durch Röntgenkristallstrukturanalyse sowie NMR-Spektroskopie aufgeklärt und weist große Ähnlichkeit mit den Strukturen der Pleckstrin-Homologie-(PH)- und denPhosphotyrosinbindenden (PTB) Domänen auf, obwohl die jeweiligen Aminosäuresequenzen nur wenig übereinstimmen.

Die EVH1-Domäne besteht aus einer gedrehten $\beta$-Sandwich-Struktur, die zu einer Seite durch eine $\alpha$-Helix geschlossen wird (Fig. 2).

Auf die EVH1-Domäne folgend findet sich eine prolinreiche Region, welche zur Bindung an SH3- und WW-Domänen-enthaltende Proteine und an Profilin dient.

Am C-Terminus ist eine EVH2-Domäne lokalisiert, die eine G-Aktin- und eine F-Aktin-Bindungsstelle enthält. Zudem weist sie ein "Coiled Coil"-Motiv auf, das die Tetramerisierung des Systems vermittelt.

Es ist offensichtlich, dass eine Modulation der Funktion über alle Untereinheiten denkbar ist. Diese Studie hat sich dabei auf die Adressierung der PRM-EVH1-Interaktion konzentriert, die im Folgenden näher betrachtet wird.

Die EVH1-Domänen von Ena/VASP-Proteinen erkennen spezifisch die prolinreiche Sequenz "FPPPP", welche sich beispielsweise in allen fokalen Adhäsionsproteinen (z. B. Zyxin und Vinculin) oder auch in Lamellipodin findet. Diese Cytoskelett-assozierten Bindungspartner der EVH1-Domäne dienen zur Lokalisierung der Ena/VASP-Proteine an den benötigten Wirkungsorten (z. B. fokale Adhäsionen oder Lamellipodien).

Eine Delokalisierung der Ena/VASP-Proteine von diesen Stellen durch das PRM bzw. die PPII-Helix imitierende Peptidliganden stellt somit ein potentielles Modulierungskonzept dar.

Ein weiterer Bindungsparter der Ena/VASP-EVH1-Domäne ist das ActA-Oberflächenprotein des intrazellulären Pathogens Listeria monocytogenes, welches ebenso das "FPPPP"-Bindungsmotiv enthält. Das Bakterium ist hierdurch in der Lage Ena/VASP-Proteine auf seiner Oberfläche zu lokalisieren und somit ihre Funktion, die Bildung von Aktinfilamenten, zur Fortbewegung zu nutzen.

Jedoch konnte gezeigt werden, dass durch Inhibition dieser PRM-EVH1-Interaktion, z. B. durch die Entfernung des FPPPP-Motivs aus dem ActA-Protein oder die Injektion FPPPP-enthaltender Peptide in das Cytoplasma, die Motilität des Pathogens drastisch reduziert werden konnte.

**Design der ProM-Scaffolds**

Das Konzept hinter den von Schmalz und Kühne entwickelten Diprolinmimetika ist in Fig. 3 verdeutlicht.

Von Kühne und Brockmann durchgeführte, computergestützte molekulare Modellierungen zeigten, dass durch die Einführung einer "starren" C2-Vinylidenbrücke (in grün gezeigt) zwischen zwei benachbarten Prolinen (2) in stereodefinierter Form ein Tricyclus (ProM-1, "prolinreiches Modul") resultiert, der in nahezu perfekter PPII-Konformation fixiert ist. Im Zuge ihrer Untersuchungen modellierten sie eine zweite spirocyclische Struktur (ProM-2), die ebenso eine fast ideale PPII-Helix imitiert.

**Synthese und Verwendung von ProM-1**

Das erste synthetisierte Diprolinmimetikum wurde als N-terminal Fmoc-geschützte Variante (Fmoc-[ProM-1]-OH) hergestellt (Schema 1)

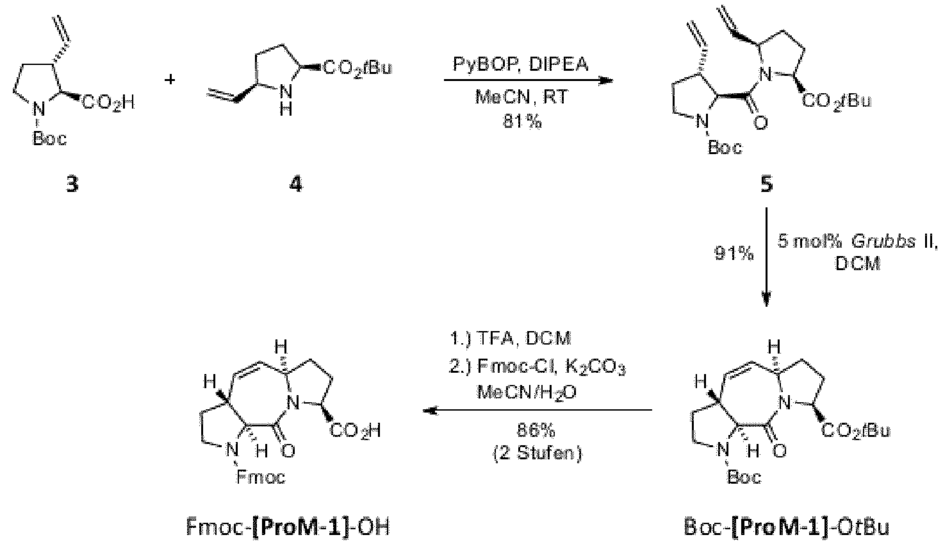


Schema 1: Synthese von Fmoc-[ProM-1]-OH.

Ausgehend von den beiden enantiomerenreinen Vinylprolinbausteinen 3 und 4 wurde nach Peptidkupplung und nachfolgender Ruthenium-katalysierter Ringschlussmetathese zuerst der Boc- und tert-Butyl-geschützte Tricyclus Boc-[ProM-1]-OtBu erhalten. Anschließende Schutzgruppenmanipulation lieferte das gewünschte und für spätere Festphasenpeptidsynthesen geeignete Fmoc-[ProM-1]-OH.

**Synthese von ProM-2**

Die Herstellung des spirocyclischen Diprolinanalogons Fmoc-[ProM-2]-OH ist möglich in Analogie zur Synthese von Fmoc-[ProM-1]-OH (Schema 2)

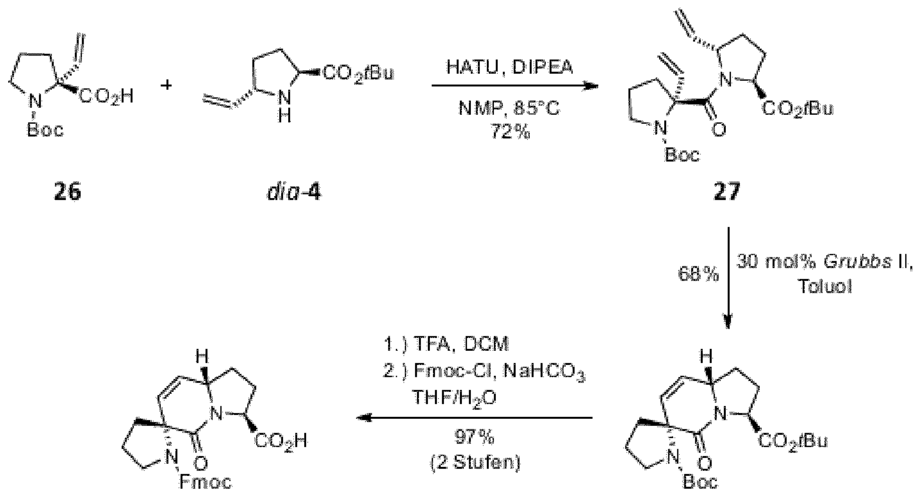


**26** *dia*-**4** **27**

Fmoc-**[ProM-2]**-OH Boc-**[ProM-2]**-O*t*Bu

Schema 2: Synthese von Fmoc-[ProM-2]-OH

So konnte ausgehend von den Vinylprolinen 26 und dia-4 nach Peptidkupplung zunächst das Dipeptid 27 und nach Ringschlussmetathese der Tricyclus Boc-[ProM-2]-OtBu erhalten werden. Die erhöhte sterische Hinderung des Bausteins 27 im Vergleich zu 5 bei der ProM-1 Synthese erforderte jedoch etwas harschere Kupplungsbedingungen und den Einsatz größerer Mengen Metathesekatalysator. Finale Schutzgruppenmanipulation lieferte auch hier das gewünschte und für die Festphasenpeptidsynthese geeignete Fmoc-[ProM-2]-OH.

**Modulare ProM-Bausteine** - **ProM-Bibliothek**

Anhand der Synthesen von ProM-1 und ProM-2 ist die generelle Synthesestrategie der ProM-Bausteine 33 erkennbar (Schema 3).

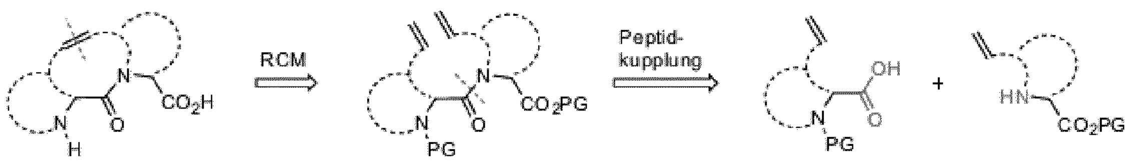


**33** **34** **35** **36**

Schema 3: Allgemeine retrosynthetische Zerlegung der ProM-Scaffolds.

Retrosynthetisch lassen sie sich über eine Ringschlussmetathese (RCM) auf ein Dipeptid 34 zurückführen und durch Peptidkupplung in einen enantiomerenreinen, vinylierten Säure-baustein 35 und einen enantiomerenreinen, vinylierten Aminbaustein 36 zerlegen. Durch strukturelle Variation der Bausteine 35 und 36 sollte es daher möglich sein auch die finalen ProM-Diprolinmimetika auf die Bedürfnisse der jeweiligen Proteinoberfläche anzupassen bzw. zu optimieren.

Zusätzlich zu den diskutierten Mimetika ProM-1 und ProM-2 wurden bereits neun weitere ProM-Scaffolds synthetisiert (Fig. 4). Zu allen abgebildeten ProM-Bausteinen sind die bis heute erschienenen Publikationen angegeben.

Konkrete Beispiele für die strukturelle Variabilität der ProMs stellen zum einen die Serie ProM-8, ProM-1, ProM-4, sowie zum anderen der Scaffold ProM-9 dar (Schema 4).

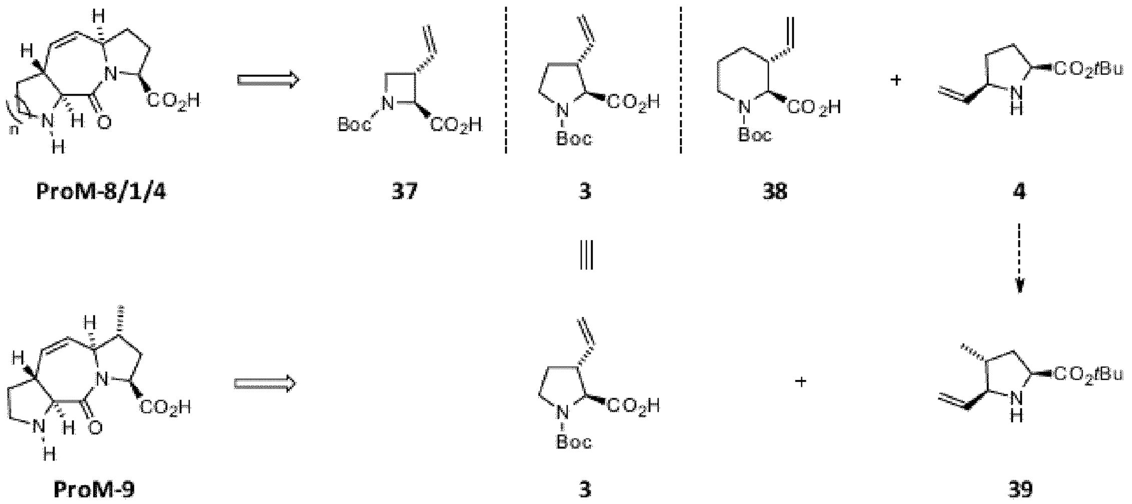

ProM-8/1/4 37 3 38 4

ProM-9 3 39

Schema 4: Retrosynthese der Bausteine ProM-8, ProM-1, ProM-4 und ProM-9.

Durch gezielte Synthese der homologen Reihe von Säurebausteinen 37, 3 und 38 konnte das Konzept der Ringverkleinerung bzw. -erweiterung zur Adressierung von flacheren und tieferen Bindungsfurchen auf der PBD-Oberfläche genutzt werden.

Die gezielte Einführung einer zusätzlichen Methylgruppe in den Aminbaustein 4 ermöglichte hingegen den Zugang zu einem ProM-1-Derivat mit zusätzlicher hydrophober Interaktionsstelle.

Einige dieser ProM-Diprolinmimetika (ProM-1 bis ProM-4) wurden bereits zur Entwicklung eines niedrigmolekularen, hoch selektiven, peptidischen, jedoch voll synthetischen Ena/VASP-EVH1 Inhibitors (PPII-Sekundärstrukturmimetikums) eingesetzt.

**Entwicklung eines Ena/VASP-EVH1 Inhibitors**

Opitz et al. berichteten in einer 2015 erschienenen Publikation (R. Opitz, M. Müller, C. Reuter, M. Barone, A. Soicke, Y. Roske, K. Piotukh, P. Huy, M. Beerbaum, B. Wiesner, M. Beyermann, P. Schmieder, C. Freund, R. Volkmer, H. Oschkinat, H.-G. Schmalz, R. Kühne, PNAS 2015, 112, 5011-5016) von der erfolgreichen Nutzung der ProM-Scaffolds als Diprolinmimetika bei der Entwicklung eines "small-molecule" Ena/VASP-EVH1 Inhibitors (Fig. 5).

Ausgehend von dem im mikromolaren Bereich an die Ena/VASP-EVH1-Domäne bindenden, ActA-abgeleiteten Peptidliganden Ac-SFEFPPPPTEDEL-NH2 (40) erfolgte die systematische Ersetzung aller vier Proline im Kernmotiv (FPPPP) des Liganden durch die rigidifizierten Scaffolds ProM-1 bis ProM-4. Hierbei resultierten die besten Ergebnisse aus der Substitution des ersten Prolinpaares durch ProM-2 (FPPPP) und des zweiten Paares durch ProM-1 (FPPPP). In weiteren Studien wurde zudem gezeigt, dass der Austausch des Phenylalanins (F) durch die unnatürliche Aminosäure L-2-Chlorphenylalanin (L-2Cl-F) zu einem drastischen Affinitätsgewinn führte. Die daraus resultierende Verbindung 41 stellte den ersten, im nanomolaren Bereich an die Ena/VASP-EVH1-Domäne bindenden Peptidliganden mit voll-synthetischem Kernmotiv dar (kanonischer Bindungsmodus).

Für die Entwicklung eines "small-molecules" war es jedoch nötig das Molekulargewicht der Verbindung 41 deutlich zu reduzieren. Interessanterweise war es möglich die flankierenden Epitope (SFE & TEDEL) ohne signifikaten Verlust der Bindungsaffinität von 41 zu entfernen, wohingegen dies beim wt-Liganden 40 zu einem nahezu Komplettverlust der Bindungsfähigkeit führte (siehe 42 & 43). Der Ligand Ac-[L-2Cl-F]-[ProM-2]-[ProM-1]-OH (43) stellte den effizientesten, niedermolekularen Inhibitor zu Adressierung aller drei Ena/VASP-EVH1-Domänen dar. Die Verbindung 43 zeigte eine 180-fach (EVL- und Ena-EVH1) bzw. sogar 280-fach (VASP-EVH1) gesteigerte Bindungsaffinität verglichen mit dem natürlichen, auf die Bindungssequenz reduzierten Peptid 42. Außerdem konnte über Kristallstrukturen der kanonische Bindungsmodus des Liganden 43 nachgewiesen und durch Veresterung der freien Säure eine zellgängige Substanz 44 hergestellt werden.

Die erfolgreiche Entwicklung eines Ena/VASP-EVH1 Inhibitors konnte nicht zuletzt in biologischen Studien bestätigt werden. Es wurde gezeigt, dass die Verbindung 44 das Protein VASP von fokalen Adhäsionen und aus der"leading edge" delokalisiert, die Bildung von Aktinfilamenten dadurch verringert und so die Motilität von stark metastasierenden Kolorektal- und Brustkrebszellen drastisch reduziert.

Zusammenfassend stellen die ProM-Diprolinmimetika nützliche, modular aufgebaute und rational designte Bausteine für die Entwicklung spezifischer Peptidmimetika zur Adressierung von PRM-PBD-Interaktionen dar.

**Aufgabenstellung und Konzeption der vorliegenden Erfindung**

Die vorliegende Erfindung basiert auf den oben genannten Studien im Bereich der PPII-Sekundärsturkturmimetikaentwicklung, z. B. die Herstellung modular aufgebauter Diprolinanaloga in PPII-Konformation (ProMs) und deren Nutzung als Pro-Pro-Äquivalent im Kernmotiv eines PRMs zur Synthese eines hoch selektiven Ena/VASP-EVH1 Inhibitors 43 (Fig. 6).

Mit dem Ziel die Bindungsaffinität des Peptidliganden an die EVH1-Domäne weiter zu erhöhen oder weitere PBDs zu adressieren wurden in den letzten Jahren daher, durch Analyse der erzielten Resultate und weiterer Modeling-Studien, zahlreiche "strukturell optimierte" ProM-Scaffolds synthetisiert (ProM-1 bis ProM-10). Diese sind alle, ausgehend von ProM-1, entweder auf eine Änderung der Stereokonfiguration oder auf die Modifikation des Kohlenstoffgerüstes (Variation von Größe und Substituenten) zurückzuführen, um gezielt weitere Bindungtaschen der Domäne zu adressieren bzw. sterischen Ansprüchen zu genügen.

Ein für diese Arbeit relevantes Beispiel stellt das hergestellte methylsubstituierte ProM-1-Derivat ProM-9 dar.

Als ProM-1-Ersatz im Ena/VASP-EVH1 Inhibitor Ac-[L-2Cl-F]-[ProM-2]-[ProM-1]-OH (43) lieferte es eine um den Faktor 5 - 10 erhöhte Bindungsaffinität.

Der eingefügte Methylsubstituent adressierte hierbei eine hydrophobe Tasche der Domäne und trug somit positiv zur Bindung bei.

Resultierend aus diesen Untersuchungen wuchs das Interesse an ProM-1 bzw. ProM-9 abgeleiteten bicyclischen Strukturen des Typs ProM-15 (Schema 5):

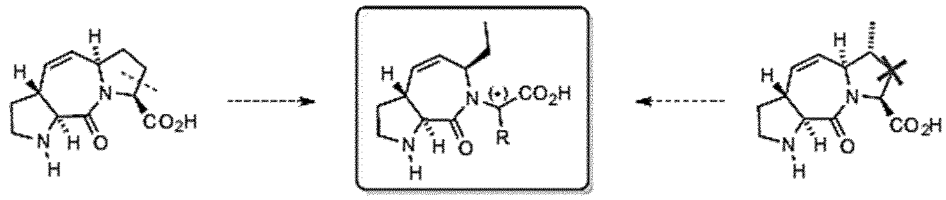

ProM-1 ProM-15 ProM-9

Schema 5: Ableitung des ProM-15-Scaffolds aus den bekannten Strukturen ProM-1 und ProM-9.

Im Falle von R = H kann ProM-15 als ein "Ostring-geöffnetes" ProM-1-Derivat oder als ein, um ein C-Atom reduziertes, ProM-9-Derivat aufgefasst werden. Einerseits zeigten Computer-Modeling-Studien, dass eine gesteigerte Flexibilität des C-Terminus von ProM-1 in 43 eine vielversprechende Option zur Erhöhung der Bindungsaffinität an die Ena/VASP-EVH1-Domäne durch eine mögliche Ausbildung von Wasserstoffbrücken darstellen würde.

Andererseits böte ProM-15 die Möglichkeit, unter Reduktion des Molekulargewichtes, den Methylsubstituenten von ProM-9 zu "imitieren" und somit die zuvor erfolgreich adressierte hydrophobe Bindungstasche ebenfalls zu nutzen. ProM-15 soll dementsprechend die positiven Bindungseigenschaften von ProM-9 mit einer Verringerung des Molekulargewichtes und einer Erhöhung der C-terminalen Flexibilität vereinen.

Nach der etablierten, generellen Synthesestrategie der ProM-Scaffolds lässt sich ProM-15 auf die enantiomerenreinen Säure- und Aminbausteine 3 und 109 zurückführen (Schema 6).

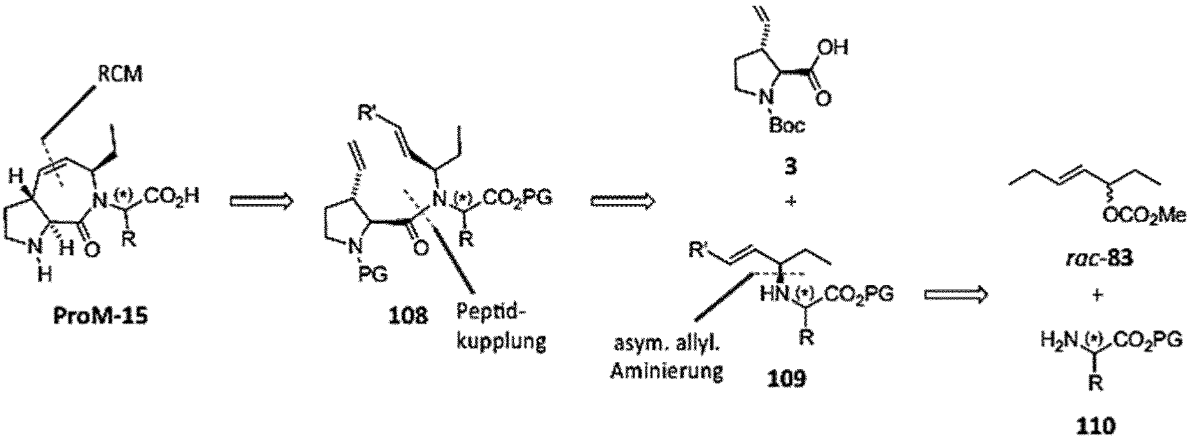


Schema 6: Retrosynthetische Synthese von Diprolinanaloga des Typs ProM-15.

Die Zielstruktur ProM-15 geht durch Ruthenium-katalysierte Ringschlussmetathese (RCM) aus einem Dipeptid des Typs 108 hervor, das wiederum durch Peptidkupplung auf die bereits bekannte "Zaminer-Säure" (3) und ein Allylamin 109 zurückführbar ist. Die stereoselektive Synthese von Allylaminen des Typs 109 stellt dabei eine besondere Herausforderung dar. Hierfür könnte eine Übergangsmetall-katalysierte asymmetrische allylische Aminierung von symmetrisch 1,3-disubstituierten Substraten (zur Vermeidung von Regioisomeren) unter Verwendung von Aminosäure-N-Nukleophilen genutzt werden. Ausgehend von dem racemischen Carbonat rac-83 und einem einfachen, in beiden absoluten Konfigurationen verfügbaren Aminosäureester 110 könnten, unter Einsatz chiraler Liganden, im Prinzip alle vier Stereoisomere gezielt hergestellt werden. Aufbauend auf den wenigen (mäßig erfolgreichen) Literaturbeispielen sollten zunächst verlässliche Bedingungen für die effiziente und stereoselektive Synthese von N-allylierten Aminosäureestern des Typs 112 unter Palladium-Katalyse entwickelt werden (Schema 7).

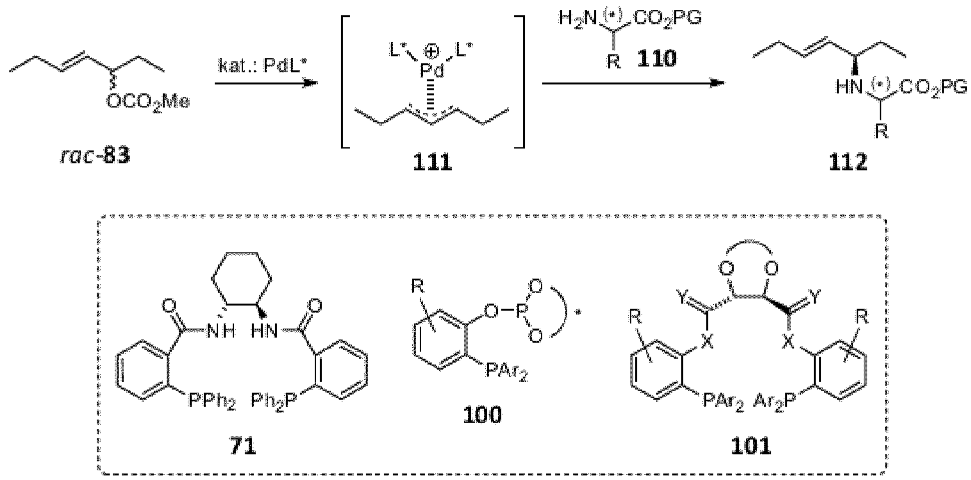


Schema 7: Angestrebte Synthese von N-allylierten Aminosäureestern des Typs 112 unter Verwendung chiraler Liganden.

Hierbei galt es insbesondere, geeignete chirale Liganden zu identifizieren, die hoch selektiv eine Steuerung der Konfiguration des neuen Stereozentrums, unabhängig von der absoluten Konfiguration des eingesetzten Aminosäurebausteins 110, ermöglichen. In diesem Zusammenhang sollten, neben etablierten Liganden wie beispielsweise dem Trost-Liganden, auch die entwickelten Phosphin-Phosphit-Liganden 100 und die C2-symmetrischen Bisphosphin-Liganden des Typs 101 getestet werden, deren modularer Aufbau eine vergleichsweise einfache (individuelle) Strukturoptimierung erlaubt. Der Einsatz diverser Aminosäureester 110 könnte dabei Zugang zu einer Vielzahl von ProM-15-Derivaten liefern, welche durch den zusätzlichen Substituenten weitere Interaktionen mit der Domäne eingehen könnten (Fig. 7).

Die durchgeführten Modeling-Studien eröffneten, dass neben Glycin (R = H), auch Phenylalanin (R = Bn) oder funktionalisierte Aminosäuren wie Glutaminsäure (R = (CH2)2CO2H) von Interesse wären. ProM-15-Derivate basierend auf L-Phenylalanin könnten zusätzliche hydrophobe Wechselwirkungen mit der EVH1-Domäne eingehen. L-Glutaminsäure erschien günstig, da der "TEDEL" Rest des ActA-Peptids imitiert werden würde und somit die Ausbildung von Wasserstoffbrücken zu umgebenden Aminosäuren möglich wäre. Weiterhin könnten ProM-15-Derivate von L-Tyrosin einen Weg zur Adressierung von WW-Domänen mit einem Kernmotiv "PPxY" eröffnen. Zielsetzung war es dementsprechend, nach erfolgreicher Herstellung von Allylaminen des Typs 112, die angestrebten Diprolinanaloga des Typs ProM-15 (z. B. als Bocgeschützte Variante) basierend auf Glycin und beispielsweise L-Phenylalanin zu synthetisieren (Fig. 8).

Die finalen, bereits etablierten Syntheseschritte (Peptidkupplung, Ringschlussmetathese) sollten dabei gegebenenfalls auf die speziellen Systeme optimiert werden. Die Nomenklatur der Zielverbindungen ist wie folgt definiert: N-Terminus-Konfiguration der Zentren a,b,c,d-Aminosäure-[ProM-15]-C-Terminus.

Aufgrund der sehr positiven Resultate bei der Entwicklung eines Ena/VASP-EVH1 Inhibitors wurden nachfolgend in vivo-Experimente angestrebt. Diese erforderten die Bereitstellung große Substanzmengen (>2 g) des ausgewählten Peptid-liganden Ac-[L-2Cl-F]-[ProM-2]-[ProM-1]-NH2 (113), welche über die bisher zum Aufbau des Inhibitors genutzte Festphasenpeptidsynthese nicht zugänglich waren (<5% Ausbeute über 2 Stufen zur freien Säure 43). Daher lag ein weiterer Fokus dieser Erfindung auf der Entwicklung einer effizienten Strategie zur Verknüpfung der ProM-Bausteine in der Flüssigphase und der Etablierung einer Multigrammsynthese des Liganden 113 (Schema 8).

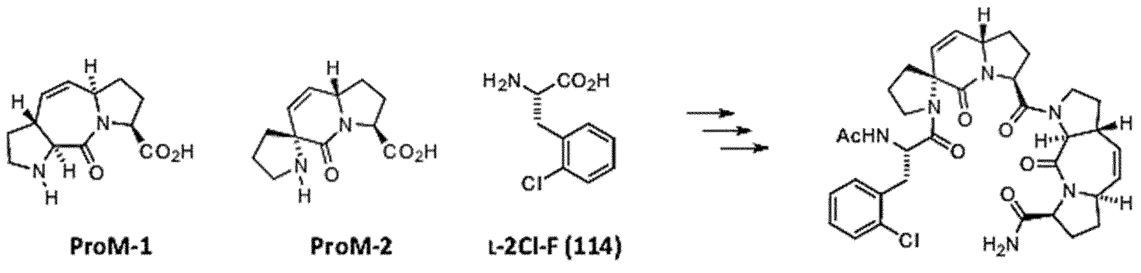

ProM-1 ProM-2 L-2Cl-F (114) 113

Schema 8: Aufbau des Peptidliganden 113 aus den Bausteinen ProM-1, ProM-2 und L-2Cl-F (114).

Neben dem in ausreichenden Mengen bereitgestellten Scaffold ProM-1, sollte ProM-2 hierzu im Multigrammmaßstab über die bekannte Syntheseroute nach Reuter und Soicke hergestellt werden.

Im Zuge dessen galt es, die relativ ineffizienten finalen Schritte der Peptidkupplung (55% Ausbeute) und der Ringschluss-metathese (30 mol% Grubbs II-Katalysator benötigt) nochmals zu untersuchen und zu optimieren.

Des Weiteren bestand die Herausforderung den Peptidliganden 113 mit C-terminaler Amidfunktion herzustellen. Bis dato wurde die aus der Festphasenpeptidsynthese resultierende freie Säure 43 zum Erreichen der Zellgängigkeit des Liganden in den Ethylester 44 überführt. Allerdings wurde erwartet, dass das Amid-Derivat 113 bei ähnlicher Bindungsaffinität und Permeabilität eine erhöhte metabolische Stabilität aufweisen und somit bei den geplanten in vivo-Experimente noch bessere Ergebnisse liefern könnte.

Nicht zuletzt sollte nach erfolgreicher Entwicklung einer Ligandensynthese in der Flüssigphase für den ProM-1 basierten Liganden 113, diese Methodik nachfolgend auch zur Synthese ProM-15 haltiger Liganden 115 genutzt werden (Fig. 9).

Durch abschließende Messung der Bindungsaffiniät der neuen Liganden 115 an die EVH1-Domäne und Kristallisation von 115 mit eben dieser, galt es, die in silico postulierten Effekte einer Substitution von ProM-1 bzw. ProM-9 durch ProM-15 final experimentell zu evaluieren.

## Ergebnisse

### Katalyseergebnisse

Im Rahmen der Erfindung gelang die Entwicklung einer hoch selektiven Methodik zur Synthese von Allylaminen des Typs 112* durch Palladium-katalysierte asymmetrische allylische Aminierung des Carbonates rac-83 und Verwendung diverser $\alpha$-Aminosäureester 110 als N-Nukleophile (Schema 9).

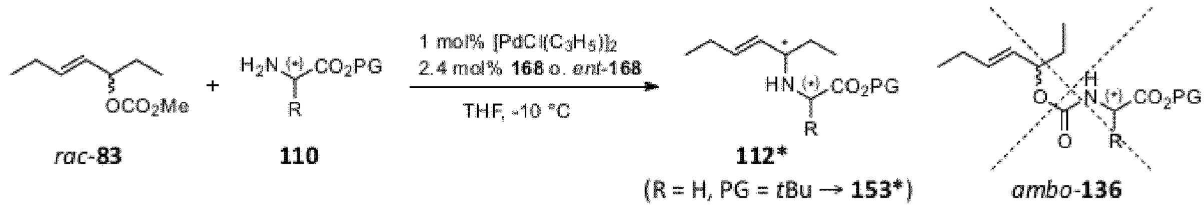


*rac*-**83** **110** **112*** (R = H, PG = *t*Bu → **153***) *ambo*-**136**

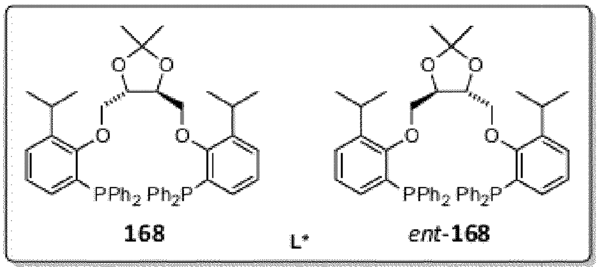

**168** L* *ent*-**168**

Schema 9: Synthese von Allylaminen des Typs 112* bzw 153* durch die in dieser Arbeit entwickelte Palladiumkatalysierte asymmetrische allylische Aminierung des Carbonates rac-83 unter Verwendung diverser a-Aminosäureester 110 als N-Nukleophile. Unter den optimierten Reaktionsbedingungen konnte eine Bildung von Carbamat-Nebenprodukten ambo-136 vollständig unterdrückt werden.

In ersten Experimenten wurden unter Einsatz einer Literaturvorschrift überraschenderweise allylische Carbamate des Typs ambo-136 erhalten, deren Bildung durch eine drastische Konzentrationserhöhung der Reaktionslösung unterdrückt werden konnte. Nach Ligandenscreening und weiteren Optimierungsschritten wurden Bedingungen gefunden, die, unter Einsatz von nur 1 mol% einer Pd-Quelle und 2.4 mol% eines chiralen Liganden (168 bzw. ent-168), die Bildung des Glycin-basierten Allylamins 153* in 95%ee und mit 74 - 84% Ausbeute ermöglichten. Die Steuerung der Konfiguration des neuen Stereozentrums war dabei durch die Wahl des entsprechenden chiralen Liganden möglich (168 → S; ent-168 → R).

Sogar bei Einsatz chiraler Nukleophile 110 konnte die zuvor nachgewiesene substrat-induzierte Diastereoselektivität überschrieben werden. Das Auftreten von matched/mismatched-Kombinationen wurde nicht beobachtet, sodass ein gleichwertiger Zugang zu allen vier Stereoisomeren möglich war. Die erreichten Selektivitäten und Ausbeuten lagen dabei alle in einem exzellenten Bereich und übertreffen die wenigen bekannten Literaturbeispiele für ähnliche Transformationen deutlich.

Die synthetisierten Allylamine und die in der Katalyse erreichten Selektivitäten sind in Fig. 10 nochmals zusammenfassend gezeigt:

**Synthese dipeptidischer Strukturen des Typs 180**

Gemäß der oben vorgestellten, retrosynthetischen Synthese von ProM-15 galt es nun, dipeptidische Strukturen des Typs 180 durch Verknüpfung des Zaminer-Säure-Bausteins (3) und der durch die allylischen Aminierungen hergestellten Allyl-amine 112 zu generieren (Schema 10).

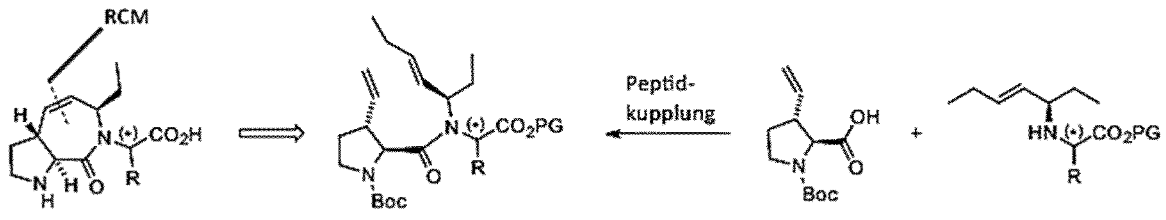


ProM-15 180 3 112

Schema 10: Zur Synthese der Zielstruktur ProM-15 ist die Darstellung dipeptidischer Strukturen des Typs 180 durch Peptidkupplung der Bausteine 3 und 112 wichtig.

Im Rahmen der verschiedenen ProM-Synthesen wurde zur Realisierung solcher Peptidkupplungen in der Regel auf zwei verschiedene Kupplungsreagenzien zurückgegriffen. In den meisten Fällen gelang eine Verknüpfung unter milden Reaktionsbedingungen (bei RT) durch Verwendung von PyBOP (181) in Kombination mit Hünigs-Base (DIPEA).

**Synthese des Dipeptidmimetikums Boc-Gly-[ProM-15]-OMe**

Zur Synthese des Glycin-basierten ProM-15-Derivates musste zunächst der enantiomeren-reine Zaminer-Säurebaustein (3) mit dem durch die Palladium-katalysierte allylische Aminierung in 95%ee zugänglichen Allylamin 153 gekuppelt werden (Schema 11).

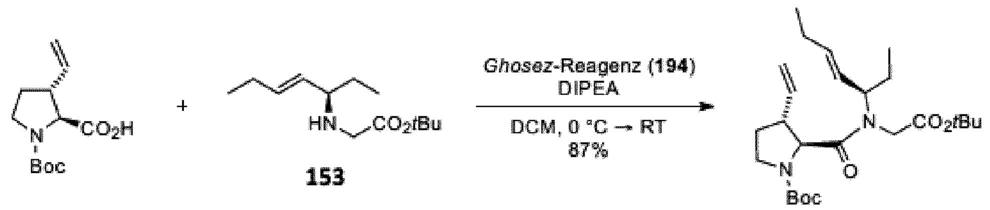


3 153 183

Schema 11: Synthese der dipeptidischen Struktur 183 unter Verwendung von Ghosez-Reagenz.

Durch Anwendung der zuvor entwickelten Kupplungsstrategie gelang die Herstellung des gewünschten Dipeptids 183 nach Aktivierung der Säure 3 durch Ghosez-Reagenz, sowie unter Einsatz von 1.1 Äq. des Amins 153 und DIPEA mit einer sehr guten Ausbeute von 87%. Neben den deutlich milderen Reaktionsbedingungen und der gesteigerten Ausbeute im Vergleich zur vorher beschriebenen Kupplung unter Verwendung des Kupplungsreagenzes HATU, fand hier zudem eine nahezu quantitative Umsetzung des Allylamins 153 statt (bestimmt per DC). Die Abtrennung der geringen Menge des verbleibenden Aminbausteins 153 stellte hiernach kein Problem mehr da und konnte, nach Waschen mit verdünnter HCl, säulenchromatographisch ohne große Verluste erreicht werden.

Das entsprechende Enantiomer ent-183 war unter denselben Bedingungen und in vergleichbarer Ausbeute darstellbar. Nachfolgend wurde dieses zur Optimierung der nächsten Syntheseschritte verwendet, da das zur Herstellung benötigte 2R,3S-Enantiomer der Zaminer-Säure (ent-3), anders als 3 selbst, zum Zeitpunkt der Syntheseentwicklung in großen Mengen zur Verfügung stand.

Die H-NMR-Spektren der beiden Diastereomere unterscheiden sich deutlich voneinander, sodass die Diastereomerenreinheit der selektiv synthetisierten Substanzen eindeutig nachgewiesen werden konnte. Die Bestimmung des Diastereomerenverhältnisses aus zuvor synthetisierten Mischungen der Stereoisomere gelang nicht, da keine vollständig isolierten Signale der einzelnen Diastereomere vorlagen.

Zudem wiesen die Spektren durch das Auftreten von mehrfachen Signalsätzen, die durch 2D H,H-NOESY-Spektroskopie eindeutig auf das Vorliegen von Rotameren zurückgeführt werden konnten (Unterscheidung von NOE- und Austauschsignalen möglich), eine sehr hohe Komplexität auf. Interessanterweise bildeten die Dipeptide 183 bzw. ent-dia-183 jeweils vier Rotamere, wohingegen für alle weiteren, auf anderen Aminosäuren beruhenden Dipeptide im Folgenden jeweils nur zwei Rotamere beobachtet werden konnten. Da für die zwei "Glycin-Protonen" (in $\alpha$-Position zum Stickstoffatom und zum Estercarbonylkohlenstoff) stets sehr unterschiedliche chemische Verschiebungen zu erkennen waren, wird vermutet, dass die zwei zusätzlichen Rotamere auf die Ausbildung einer wahrscheinlich intramolekularen Wasserstoffbrücke zurückzuführen sind.

Im nächsten Syntheseschritt sollte durch Ruthenium-katalysierte Ringschlussmetathese (RCM) die Bildung

der bicyclischen ProM-15-Struktur erfolgen. Bei der Herstellung aller bisherigen ProM-Scaffolds erfolgte die Metathesereaktion unter Verwendung des Grubbs II-Katalysators (208), wobei die benötigte hohe Katalysatorbeladung von 10 - 30 mol% ein bis dato ungelöstes Problem darstellte. Während der in Rahmen dieser Arbeit durchgeführten Nachschubsynthese des Bausteins ProM-2 gelang jedoch die Entwicklung einer leicht optimierten Synthesevorschrift, die eine Verringerung der Grubbs II-Menge erlaubte. Diese verbesserten Bedingungen wurden nun auf das ProM-15-System angewendet, wobei erste Syntheseversuche zunächst mit der in großen Mengen zugänglichen enantiomeren Form des Dipeptids ent-183 erfolgten (Schema 12).

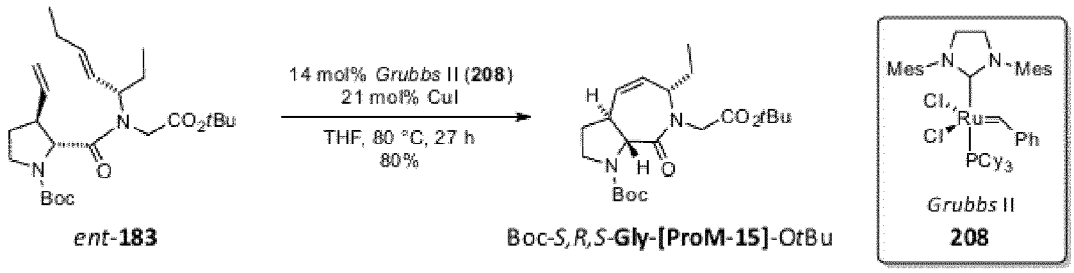


*ent*-**183**

Boc-*S,R,S*-**Gly-[ProM-15]**-O*t*Bu

*Grubbs* II
**208**

Schema 12: RCM von ent-183 zu Boc-S,R,S-Gly-[ProM-15]-OtBu unter Einsatz des Grubbs II-Katalysators (208).

Durch portionsweise Zugabe von insgesamt 14 mol% Grubbs II-Katalysator (208), sowie 21 mol% Kupfer(I)-Iodid (CuI) zum Dipeptid ent-183 und Refluxieren des Reaktionsgemisches in THF für 27 h konnte das gewünschte Produkt in einer guten Ausbeute von 80% isoliert werden. In Anlehnung an die aus der ProM-2 Metathese gewonnenen Erkenntnisse wurde von einer Optimierung mit Grubbs II (208) zur Reduzierung der auch hier notwenigen, hohen Katalysatorbeladung abgesehen und stattdessen nachfolgend ein Screening verschiedener Metathesekatalysatoren angestrebt. Bei der Literaturrecherche zur Identifizierung geeigneter Katalysatorkandidaten wurde festgestellt, dass die Reaktion sehr komplex und eine rationale Wahl des Katalysators in der Regel schwierig ist. Der Erfolg der Metathese hängt auf besondere Weise sehr stark von einem Zusammenspiel aller Reaktionsparameter (Lösungsmittel, Konzentration der Reaktionsmischung, Temperatur, Reaktionszeit) ab, die für jedes Substrat hoch spezifisch angepasst werden müssen.

In der Regel wurden die besten Ergebnisse für sterisch anspruchsvolle Metathesen mit dem Nitro-Grela-Katalysator (209) oder dem Hoveyda II-Katalysator (210) in Toluol bei 70 °C erzielt. Diese Parameter dienten entsprechend als Ausgangspunkt für die hier angestrebte Optimierung der RCM von ent-183, wobei die Substratkonzentration von 0.1 mol/l zunächst beibehalten wurde. Als weiterer Katalysator wurde die von Stewart et al. entwickelte Verbindung 211 getestet, welche insbesondere bei der herausfordernden Bildung von tetra-substituierten Doppelbindungen durch RCM überzeugte.

Die Zugabe des Katalysators erfolgte stets portionsweise, da sich im Zusammenhang mit der Optimierung der ProM-2 Metathese hierbei bessere Ausbeuten erzielen ließen. Eine Menge von 6 mol% wurde als die maximal tolerierbare Katalysatorbeladung definiert.

Die Ergebnisse des Screenings sind in Schema 13 zusammengefasst.

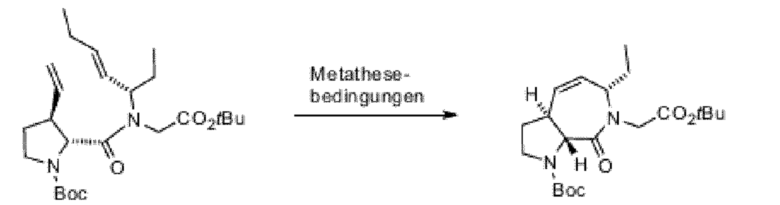


*ent*-**183** Boc-*S,R,S*-**Gly-[ProM-15]**-O*t*Bu

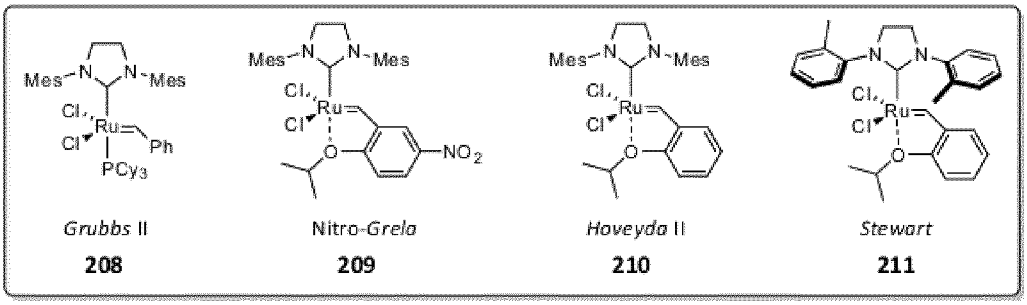

*Grubbs* II **208** Nitro-*Grela* **209** *Hoveyda* II **210** *Stewart* **211**

Schema 13: Screening verschiedener Katalysatoren in der Ringschlussmetathese des Dipeptids ent-183 und Optimierung der Reaktionsbedingungen.

Unter den gewählten Standardbedingungen zeigten die von Bieniek et al. Empfohlenen Nitro-Grela- und Hoveyda II-Katalysatoren und tatsächlich die besten Ergebnisse.

Mit nur 6 mol% Katalysator in einer deutlich verkürzten Reaktionszeit von nur 5 h konnten 78% bzw. 80% des cyclisierten Produktes Boc-S,R,S-Gly-[ProM-15]-OtBu isoliert werden. Eine Verlängerung der Reaktionsdauer auf 22.5 h führte zu keiner deutlichen Steigerung der Ausbeute. Es ist bekannt, dass durch den Wechsel von Toluol auf perfluorierte aromatische Lösungsmittel eine deutliche Erhöhung der Aktivität vieler Metathesekatalysatoren zu beobachten war. Dies konnte hierdurch Verwendung von Hexafluorbenzol (HFB) bestätigt werden. Für die beiden bisher besten Katalysatoren 209 und 210 wurde eine Steigerung der Ausbeute um je circa 10% erreicht, wobei der Hoyveda II-Katalysator mit 91% das beste Ergebnis lieferte. Eine verlängerte Reaktionszeit führte jedoch auch hier nicht zu einer Verbesserung der Ausbeute. Da die Metathesen in deutlich verdünnten Reaktionsmischungen durchgeführt wurden, erfolgte abschließend eine Verdünnung der Reaktionslösung um die Hälfte.

Die hieraus resultierende, minimal gesteigerte Ausbeute stand jedoch in keinem Verhältnis zum Wert des verwendeten Lösungsmittels. Abschließend zeigte sich, dass im Vergleich zu einer dreimaligen portionsweisen Zugabe des Katalysator 210 ä je 2 mol% nach 0 h, 2 h und 3.5 h, gleiche Ergebnisse bei einer Zugabe von 4 mol% zu Beginn und weiteren 2 mol% nach 3.5 h erreicht werden konnten.

Die aus dem Screening erhaltenen besten Reaktionsbedingungen wurden nachfolgend zur Synthese des angestrebten Stereoisomers Boc-R,S,R-Gly-[ProM-15]-OtBu genutzt (Schema 14).

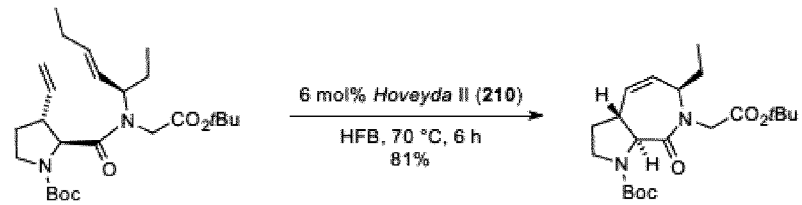


**183** Boc-*R,S,R*-**Gly-[ProM-15]**-O*t*Bu

Schema 14: Synthese von Boc-R,S,R-Gly-[ProM-15]-OtBu durch RCM von 183 unter optimierten Bedingungen.

In dem deutlich größeren Reaktionsansatz wurde das gewünschte Cyclisierungsprodukt zwar mit einer etwas

reduzierten, aber immer noch sehr guten Ausbeute von 81% erhalten. Diese war nicht höher als unter Verwendung von Grubbs II als Katalysator, allerdings konnte eine deutliche Reduzierung der Katalysatormenge von 14 mol% auf 6 mol% und der Reaktionszeit von 27 h auf 6 h, sowie eine drastische Vereinfachung der Reaktionsvorschrift erreicht werden. Nach der säulenchromatographischen Reinigung erfolgte die Entfernung von Rutheniumrückständen durch Rühren über QuadraSil AP, einem Silica-basierten Metallscavenger. Diese finale Aufreinigung wurde bei allen in dieser Arbeit beschriebenen Metathesen abschließend durchgeführt und lieferte die Produkte als nahezu weiße Feststoffe.

Auf dieser Stufe gelang es die beiden Enantiomere Boc-R,S,R-Gly-[ProM-15]-OtBu und Boc-S,R,S-Gly-[ProM-15]-OtBu aus einem Gemisch von n-Heptan/EtOAc = 1/1 zu kristallisieren und somit erstmals Informationen über die Konfiguration des in der Pd-katalysierten allylischen Aminierung erzeugten Stereozentrums zu erhalten. Die Qualität der erzeugten Kristalle reichte hierbei zur eindeutigen Bestimmung der relativen Konfiguration durch Röntgenkristallstrukturanalyse aus.

In Fig. 11 sind die Kristallstrukturen über den entsprechenden Formelbildern dargestellt.

Abschließend ist anzumerken, dass stets versucht wurde nicht umgesetztes Dipeptid 183 bzw. ent-183 zu reisolieren. Interessanterweise gelang dies jedoch in keinem Fall, wobei die stattdessen isolierte Substanz denselben Rf-Wert (0.52, Silica, cHex/EtOAc = 1/1) wie das Substrat aufwies. Eine vollständige Aufklärung der Struktur dieser Verbindung war nicht möglich, jedoch konnte durch NMR-Analytik eindeutig eine Strukturverwandtschaft zum Ursprungssubstrat 183 erkannt werden, da die "charakteristischen" Signale des Dipeptids 183 noch vorhanden waren. Allerdings wurden deutliche Signalverschiebungen im Bereich der Doppelbindungsprotonen registriert, die vermutlich auf eine Isomerisierung des Olefins, eine wohl bekannte Nebenreaktion der RCM, zurückzuführen sind.

Die resultierende Verbindung cyclisierte dabei anscheinend nicht oder deutlich langsamer als die Ursprungsverbindung 183, da keine signifikanten Mengen weiter Nebenprodukte detektiert werden konnten. Ähnliche Beobachtungen bezüglich der Reisolierung von Startmaterial wurden auch bei nachfolgenden Metathesen zur Herstellung von ProM-15-Derivaten gemacht, wobei hier jedoch zusätzlich geringe Mengen eines vom Produkt schwer abzutrennenden Nebenproduktes anfielen.

Für die anstehende Synthese eines ProM-15-haltigen Peptidliganden musste in einem letzten Syntheseschritt Boc-R,S,R-Gly-[ProM-15]-OtBu durch Schutzgruppenmanipulation in einen Methylester umgewandelt werden. Diese Umesterung erfolgte nach einer leicht modifizierten zweistufigen Vorschrift von Reuter.

Unter Einsatz von Thionylchlorid in MeOH wurde zunächst global entschützt und methylverestert. Anschließend erfolgte eine erneut N-terminale Boc-Schützung des Intermediats 213 mit Di-tert-Butyldicarbonat (Boc2O) in wässriger alkalischer Lösung (Schema 15).

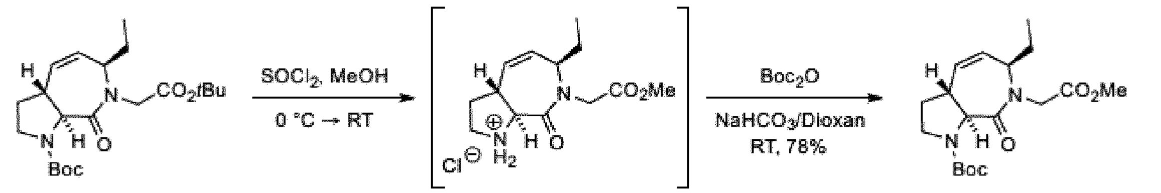


Boc-*R,S,R*-**Gly-[ProM-15]**-O*t*Bu **213** Boc-*R,S,R*-**Gly-[ProM-15]**-OMe

Schema 15: Finale Schutzgruppenmanipulation zur Synthese von Boc-R,S,R-Gly-[ProM-15]-OMe.

Nach säulenchromatographischer Reinigung (Silica, DCM/EtOAc = 1.5/1) wurde die gewünschte finale Zielstruktur Boc-R,S,R-Gly-[ProM-15]-OMe in 78% Ausbeute als farbloses, hoch viskoses Öl erhalten.

Abschließend ist die dreistufige Synthese des anvisierten Diprolinanalogons Boc-R,S,R-Gly-[ProM-15]-OMe, ausgehend von den enantiomerenreinen Bausteinen Zaminer-Säure 3 undAllylamin 153, nochmals übersichtlich zusammengefasst (Schema 16).

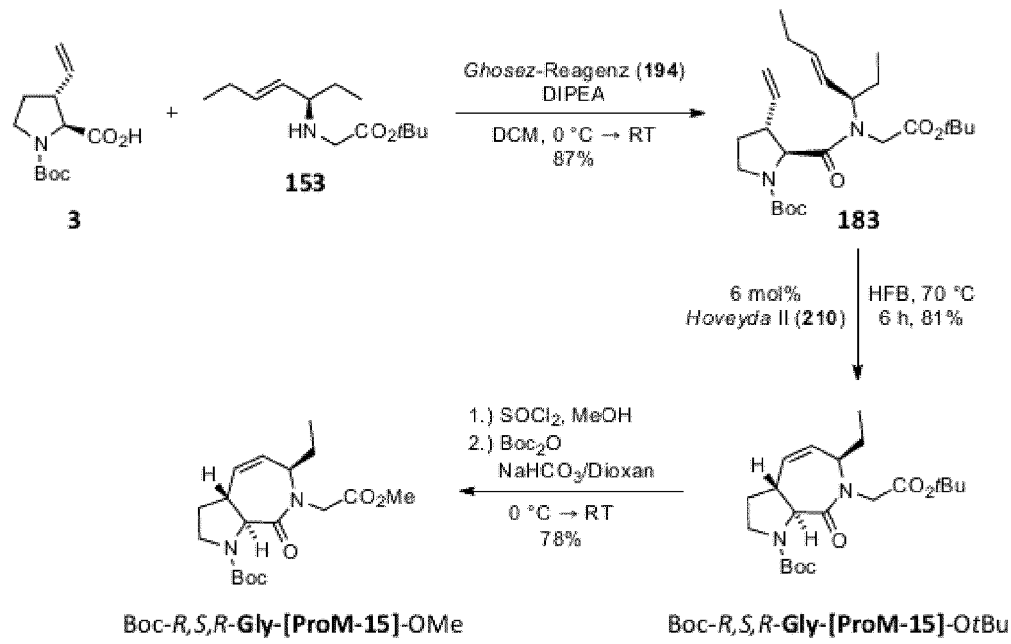


3

153

183

Boc-*R,S,R*-**Gly-[ProM-15]**-OMe

Boc-*R,S,R*-**Gly-[ProM-15]**-O*t*Bu

Schema 16: Synthese von Boc-R,S,R-Gly-[ProM-15]-OMe ausgehend von Zaminer-Säure (3) und dem Allylamin 153

**Synthese des Dipeptidmimetikums Boc-Phe-[ProM-15]-OMe**

Wie oben beschrieben, war neben dem Glycin-basierten R,S,R-Gly-[ProM-15] auch ein auf L-Phenylalanin beruhendes Derivat mit ansonsten gleicher relativer Konfiguration von Interesse. Da zum Zeitpunkt der Syntheseentwicklung jedoch die zur Herstellung dieses Bausteins benötigte 2S,3R-Zaminer-Säure 3 nicht zur Verfügung stand, wurde entschieden zunächst die enantiomere Verbindung S,R,S,R-Phe-[ProM-15]-OMe aus der in reichlichen Mengen verfügbaren 2R,3S-Zaminer-Säure ent-3 zu synthetisieren. Da ferner, durch die zuvor entwickelte Pd-katalysierte allylische Aminierung, alle vier Stereoisomere des Phenylalanin-basierten Allylamins 175* gezielt zugänglich waren, wurden neben der angestrebten Zielstruktur auch die restlichen drei Diastereomere dieser Verbindung hergestellt.

Die Ergebnisse der Peptidkupplung der einzelnen Stereoisomere von 175* mit dem 2R,3S-Enantiomer der Zaminer-Säure ent-3 sind in Schema 17 zusammengefasst:

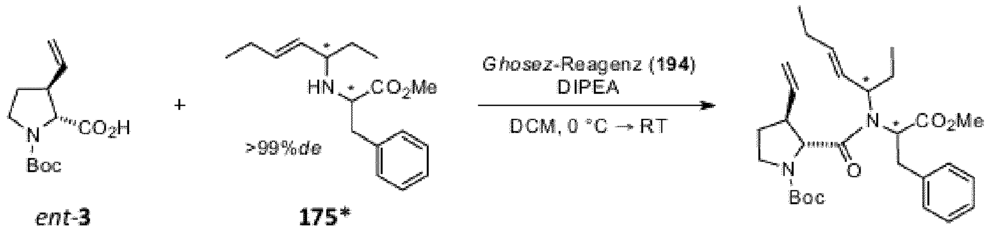


*ent*-**3** **175*** **187***

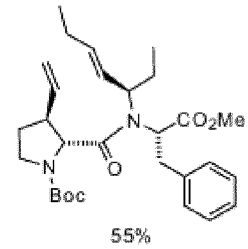

55%

**187-a**

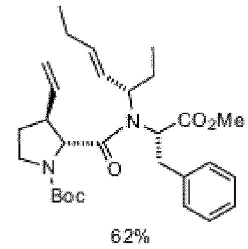

62%

*dia*-**187-b**

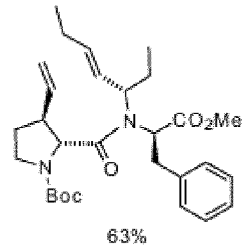

63%

*dia*-**187-c**

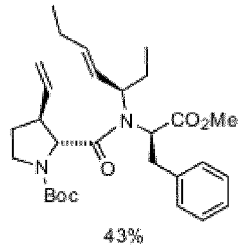

43%

*dia*-**187-d**

Schema 17: Peptidkupplung von Zaminer-Säure (ent-3) mit den diastereomerenreinen Dipeptiden 175* nach Aktivierung mit Ghosez-Reagenz (194). Angegeben sind die isolierten Ausbeuten der vier Diastereomere nach säulenchromatographischer Aufreinigung.

Es gelang dementsprechend die Dipeptide 187-a bis dia-187-d gezielt als enantio- und diastereomerenreine Substanzen herzustellen. Im Folgeschritt konnten die gewünschten Phe-[ProM-15]-Bausteine durch Hoveyda II-vermittelte Ringschlussmetathese erfolgreich hergestellt werden (Schema 18).

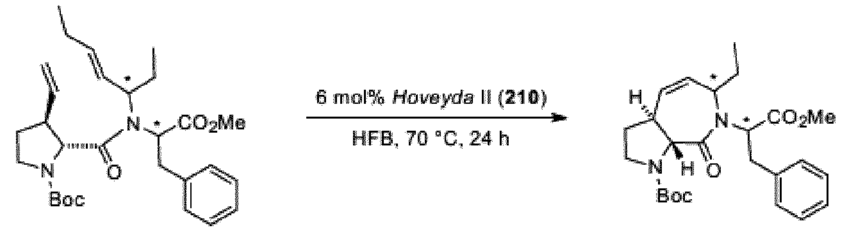


187*  Boc-Phe-[ProM-15]-OMe

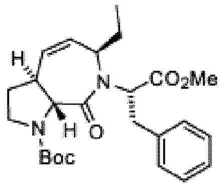

31%

Boc-*S,R,R,S*-Phe-[ProM-15]-OMe

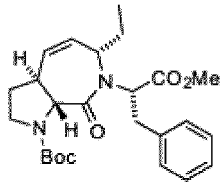

71%

Boc-*S,R,S,S*-Phe-[ProM-15]-OMe

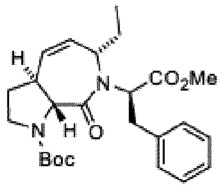

62%

Boc-*S,R,S,R*-Phe-[ProM-15]-OMe

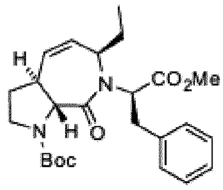

62%

Boc-*S,R,R,R*-Phe-[ProM-15]-OMe

Schema 18: RCM der Dipeptide 187* unter Einsatz von Hoveyda II-Katalysator (210) in Hexafluorbenzol (HFB). Angegeben sind die isolierten Ausbeuten der Cyclisierungsprodukte Boc-Phe-[ProM-15]-OMe nach säulenchromatographischer Aufreinigung und Entfernung von Rutheniumrückständen durch QuadraSil AP.

Unter den zuvor für das Gly-[ProM-15]-Derivat entwickelten Metathesebedingungen gelang die Herstellung alle vier Diastereomere von Phe-[ProM-15] durch Einsatz von 6 mol% Ruthenium-Katalysator 210 in Hexafluorbenzol bei 70 °C in moderaten bis guten Ausbeuten.

Für alle vier Diastereomere gelang es Einkristalle mit ausreichender Qualität zu züchten, um durch Röntgenkristallstrukturanalyse die relative Konfiguration der Stereozentren zu belegen (Fig. 12).

**Synthese des Dipeptidmimetikums Boc-R,S,R,S-Ala-[ProM-15]-OMe**

Nachdem die Kupplung von rac-3 mit dem L-Alanin-basierten Allylamin ambo-171 unter "HATU-Bedingungen" zunächst nicht möglich war, wurde diese anschließend unter Einsatz der neu entwickelten Methodik getestet (Schema 19).

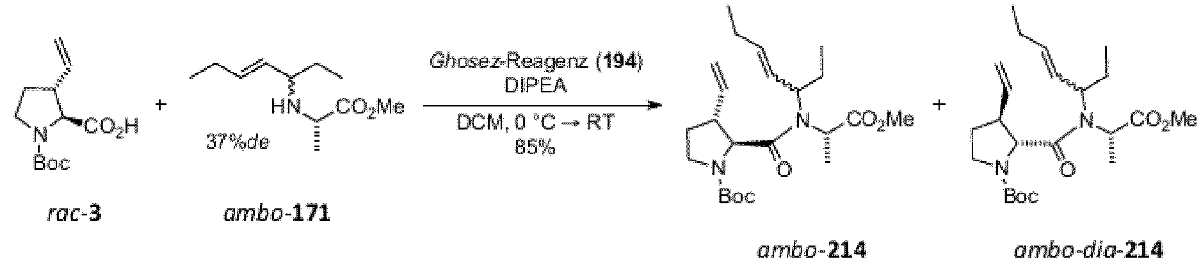


rac-3 ambo-171 ambo-214 ambo-dia-214

Schema 19: Peptidkupplung von racmeischer Zaminer-Säure (rac-3) mit dem Diastereomerengemisch ambo-171.

Tatsächlich gelang auch hier die Synthese der dipeptischen Verbindungen ambo-214 und ambodia-214 in einer sehr guten Ausbeute von 85%. Das Verhältnis der vier Diastereomere konnte an dieser Stelle nicht bestimmt werden, allerdings wurde dünnschicht- und säulenchromatographisch eine teilweise Auftrennung der Stereoisomere beobachtet (3 Spots). Es gelang jedoch zunächst nicht, das trennbare Diastereomerenpaar zu identifizieren. Aus diesem Grund wurde die racemische Zaminer-Säure (rac-3) einzeln mit den beiden, über die Pd-katalysierte allylische Aminierung gezielt zugänglichen, diastereomerenreinen Allylaminen 171 bzw. dia-171 umgesetzt (Schema 20).

Tatsächlich gelang im Falle des R,S-konfigurierten Allylamins 171 eine gute säulenchromatographische Trennung der resultierenden Diastereomere 214-a und dia-214-b, wohingegen die Produkte der Kupplung von rac-3 mit dem S,S-Allylamin dia-171 nicht separiert werden konnten.

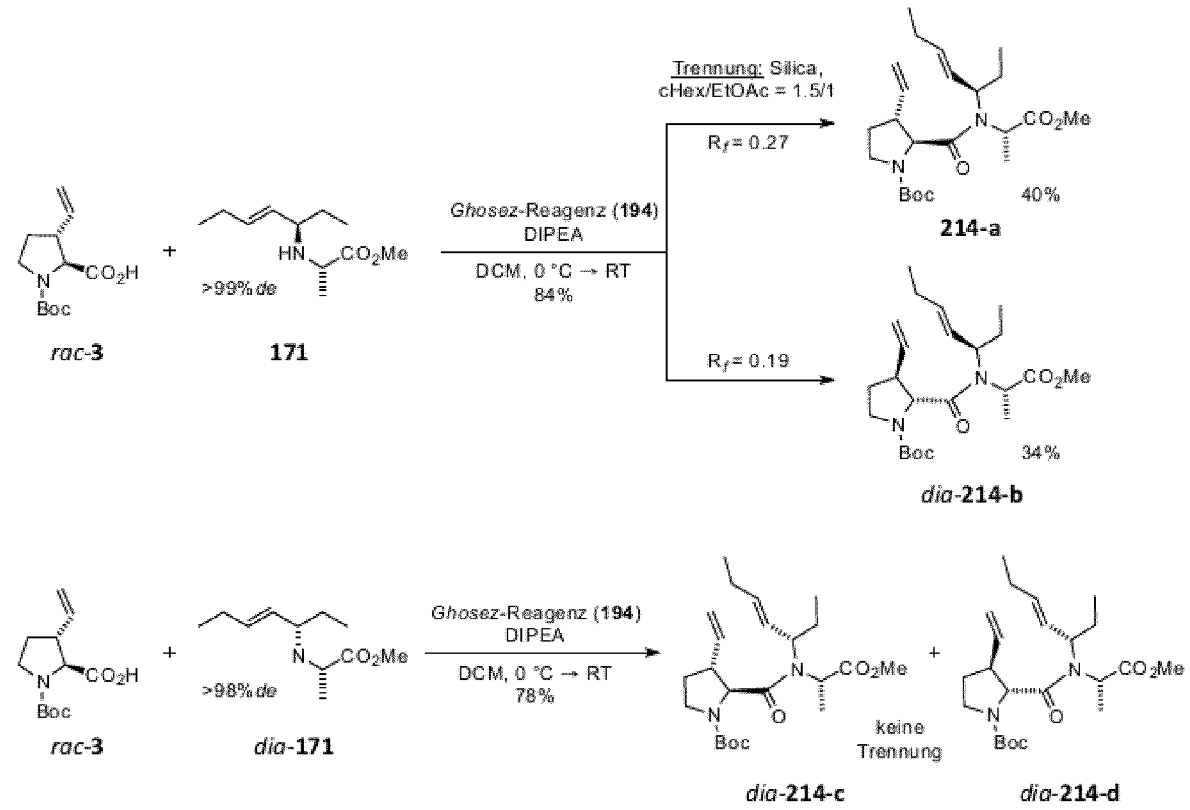


rac-3 171 214-a dia-214-b

rac-3 dia-171 dia-214-c dia-214-d

Schema 20: Peptidkupplung von racemischer Zaminer-Säure (rac-3) mit dem R,S-konfigurierten Allylamin 171 (oben); und mit dem S,S-konfigurierten Allylamin dia-171 (unten).

Die Verbindung dia-214-b lag als Einziges der vier Diastereomere als farbloser Feststoff anstatt als farbloses, viskoses Öl vor. Nach Kristallisation von dia-214-b konnten die relativen Konfigurationen der Stereozentren von 214-a

und dia-214-b daraufhin durch Röntgenkristallstrukturanalyse identifiziert werden (Fig. 13).

Dieser glückliche Zufall eröffnete die Möglichkeit zur Synthese eines weiteren ProM-15-Derivates mit korrekter relativer Konfiguration der Stereozentren. Ringschlussmetathese des Dipeptids 214-a unter den etablierten Bedingungen mit Hoveyda II-Katalysator in HFB bei 70 °C lieferte die gewünschte Zielstruktur Boc-R,S,R,S-Ala-[ProM-15]-OMe nach säulen-chromatographischer Reinigung in 61% Ausbeute (Schema 21).

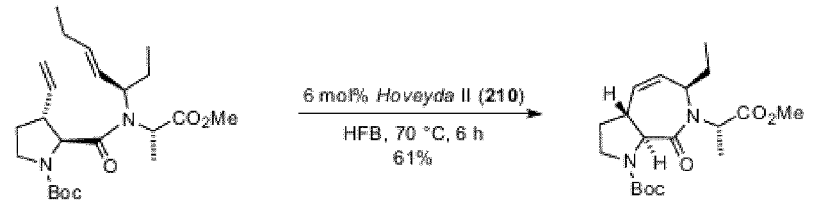


**214-a** Boc-*R,S,R,S*-**Ala-[ProM-15]**-OMe

Schema 21: RCM des Dipeptids 214-a unter Einsatz von Hoveyda II-Katalysator (210) in Hexafluorbenzol (HFB).

Abschließend gelang es hier ebenso die finale Verbindung zu kristallisieren und die relative Konfiguration durch Röntgenkristallstrukturanalyse zu bestätigen (Fig. 14).

### Synthese des Dipeptidmimetikums ProM-2

#### Herstellung von Fmoc-[ProM-2]-OH

Im Zuge der angestrebten Entwicklung einer Ligandensynthese in der Flüssigphase, wurden größere Mengen des Scaffolds ProM-2 benötigt. Die Synthese des literatur-bekannten Fmoc-[ProM-2]-OH erfolgte dabei im Grunde nach den bekannten Vorschriften.

Zum Aufbau der dipeptidischen Struktur ambo-27 wurden die beiden Bausteine 26 und dia-4 unter literaturbekannten Bedingungen mit HATU und DIPEA in NMP bei 85 °C gekuppelt. Die Cyclisierung des Spirobausteins ambo-27 durch Ringschlussmetathese erforderte bis dato stets große Mengen Grubbs II-Katalysator (30 mol%). Eine Steigerung der Ausbeute auf bis zu 75% bei gleichzeitiger Reduzierung der Katalysatormenge um bis zu 50% erreicht werden konnte, wenn die Reaktion in refluxierendem THF durchgeführt und zudem Kupfer(I)-Iodid (CuI) in katalytischen Mengen zugefügt wurde (Schema 22).

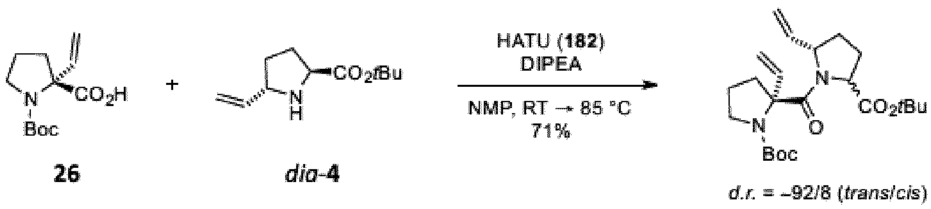


**26** *dia*-**4**

*d.r.* = ~92/8 (*trans*/*cis*)

*ambo*-**27**

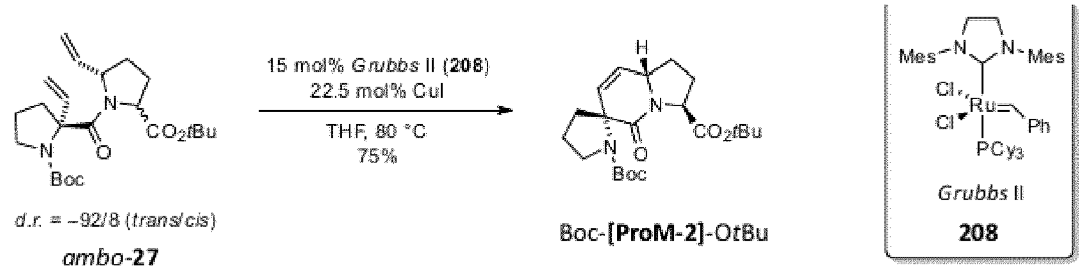


Schema 22: Grubbs II (208)-vermittelte Ringschlussmetathese des Spiro-Dipeptids ambo-27 unter Zusatz katalytischer Mengen CuI.

Die finale Schutzgruppenmanipulation zur Herstellung des Fmoc-[ProM-2]-OH lieferte bis dato bereits gute Ausbeuten von 80-90%. In Zusammenarbeit mit Reuter war es jedoch möglich diese noch zu steigern, indem die N-terminale Fmoc-Schützung nicht nur in gesättigter, sondern durch Zufügen von weiterem festen NaHCO3 in übersättigter wässriger NaHCO3-Lösung durchgeführt wurde. So konnte nach globaler saurer Entschützung mit TFA, basischer Fmoc-Schützung und abschließender säulenchromatographischer Aufreinigung des Rohproduktes mittels eines Reveleris Flash-Säulenchromatographiesystems (DCM/MeOH), das anvisierte Mimetikum Fmoc-[ProM-2]-OH in fast quantitativer Ausbeute isoliert werden (Schema 23).

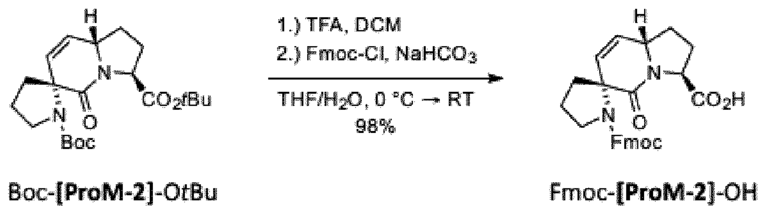


Schema 4.23: Finale Schutzgruppenmanipulation zum Aufbau von Fmoc-[ProM-2]-OH.

**Herstellung von Boc-[ProM-2]-OH**

Zur Optimierung der in dieser Arbeit entwickelten Ligandensynthese in der Flüssigphase bestand die Nachfrage nach einer N-terminal Boc- anstatt Fmoc-geschützten Version der freien Säure von ProM-2. Erste Versuche zur Synthese dieser Verbindung ausgehend von Boc-[ProM-2]-OtBu, unter Anwendung einer ähnlichen Strategie wie zur Herstellung des Fmoc-Derivates, lieferten jedoch keine befriedigenden Ergebnisse. Aus diesem Grund wurde ein Zugang zum methylveresterten Dipeptid 233 entwickelt (Schema 24).

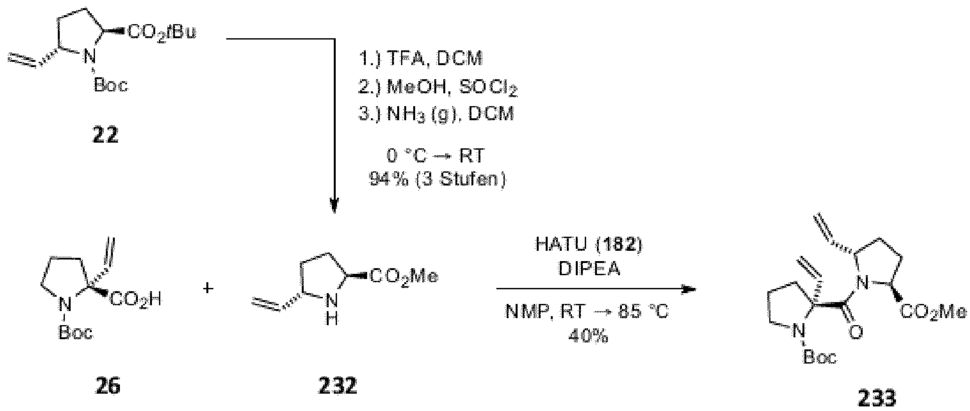


22

26 232 233

Schema 24: Synthese des methylveresterten Spiro-Dipeptids 233 ausgehend von der Spiro-Säure 26 und dem vollständig geschützten trans-5-Vinylprolinbaustein 22.

Unter ähnlichen Bedingungen wie zuvor für das tert-Butylester-Derivat ambo-27, erfolgte die Cyclisierung des Spirobausteins 233 durch portionsweise Zugabe von Grubbs II (208) und CuI zum Reaktionsgemisch. Abweichend wurde jedoch das Lösungsmittel Hexafluorbenzol (HFB) anstatt Toluol verwendet (Schema 25), da durch diesen Wechsel bei der Synthese von ProM-15 signifikant verbesserte Metatheseergebnisse erzielt werden konnten.

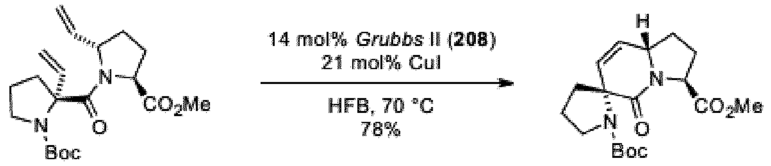


233 Boc-**[ProM-2]**-OMe

Schema 25: Grubbs II (208)-vermittelte RCM des Spiro-Dipeptids 233 unter Zusatz katalytischer Mengen CuI.

Die finale Verseifung der Methylesterfunktionalität konnte problemlos und in quantitativer Ausbeute in wässrig-alkalischem Medium realisiert werden (Schema 26).

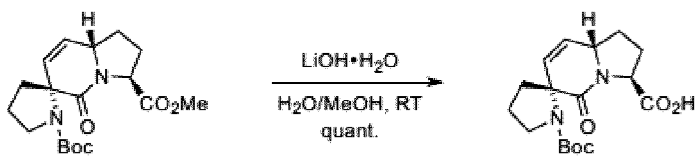


Boc-**[ProM-2]**-OMe Boc-**[ProM-2]**-OH

Schema 26: Verseifung des Methylesters von Boc-[ProM-2]-OMe.

**Ligandensynthese in der Flüssigphase**

**Entwicklung der Kupplungsmethodik** - **"Fmoc-Route"**

Nachdem der entwickelte ProM-basierte Ena/VASP-EVH1 Inhibitor bei in vitro-Studien überzeugte und bemerkenswerte biologische Ergebnisse lieferte, sollten dessen Eigenschaften nachfolgend in vivo untersucht werden. Aus diesem Grund wurden große Substanzmengen (>2 g) des Peptidliganden 113 benötigt. Es galt die bekannten Bausteine Fmoc-[ProM-1]-OH, Fmoc-[ProM-2]-OH und das kommerziell erhältliche L-2-Chlor-phenylalanin (L-2Cl-F) zu nutzen, um eine effiziente Ligandensynthese in der Flüssigphase zu entwickeln und ausreichende Mengen des Inhibitors 113

bereitzustellen (Schema 27).

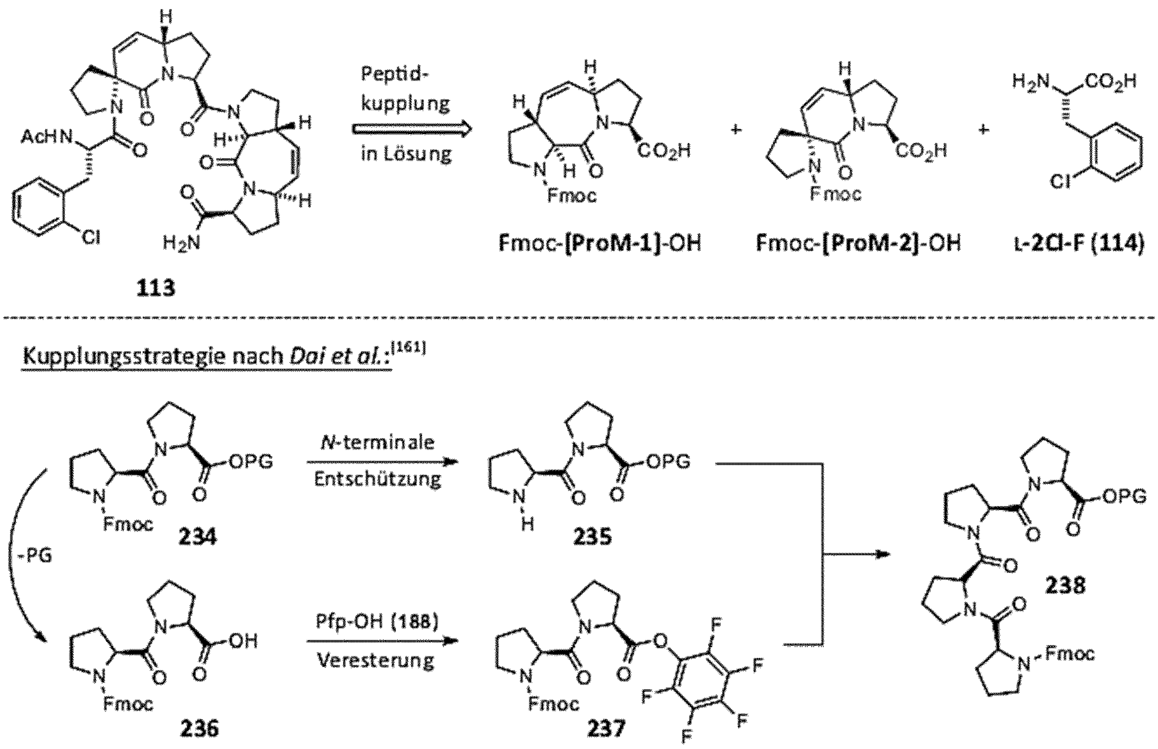


Schema 27: Oben: Retrosynthetische Zerlegung des Liganden 113. Unten: Peptidkupplungsstrategie.

Dieser Strategie folgend bot es sich an, den Liganden wie bei der Festphasenpeptidsynthese vom C-Terminus her aufzubauen. Hierzu wurde der ProM-1-Scaffold zunächst C-terminal geschützt, indem er in den methylveresterten Baustein Fmoc-[ProM-1]-OMe überführt wurde (Schema 28).

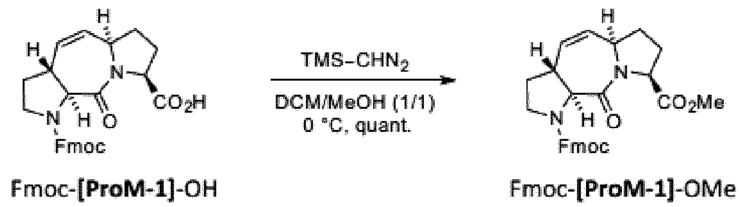


Schema 28: Methylveresterung von Fmoc-[ProM-1]-OH unter Einsatz von TMS-Diazomethan.

Nachdem die einzelnen Ligandenbausteine in guten Ausbeuten und im Multigrammmaßstab bereitgestellt werden konnten, erfolgte anschließend zunächst die Verknüpfung der beiden ProM-Scaffolds nach der durch Dai et al. beschriebenen Methodik (Schema 29).

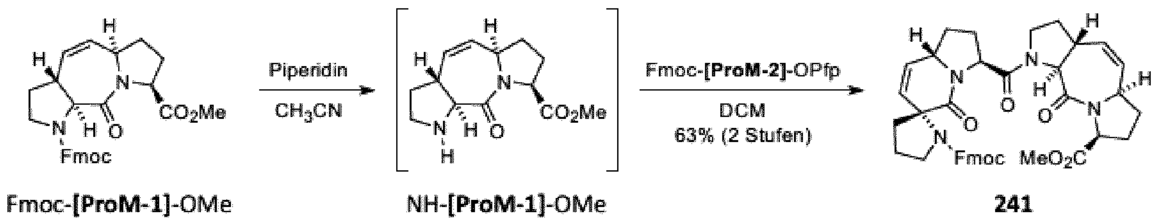

Schema 29: N-terminale Entschützung von Fmoc-[ProM-1]-OMe mit Piperidin und nachfolgende Peptidkupplung des freien Amins NH-[ProM-1]-OMe mit Fmoc-[ProM-2]-OPfp zur Synthese des Tetrapeptids 241.

Die finale Kupplung zum Aufbau des Peptidliganden ambo-242 erfolgte nach identischer Vorgehensweise (Schema 30).

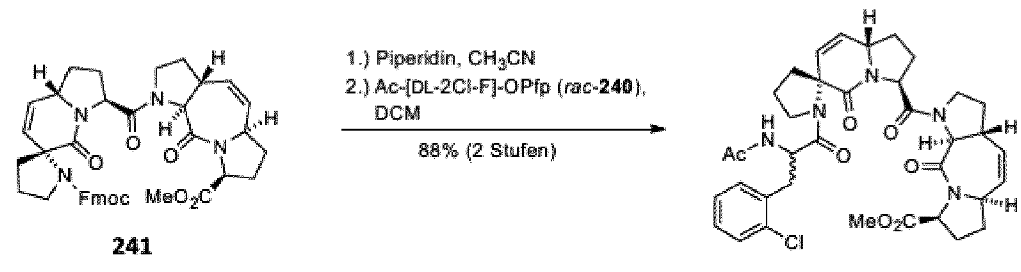


**241**

*ambo*-**242**

Schema 30: Finale Peptidkupplung zum Aufbau des Peptidliganden ambo-242.

Die finale Überführung des Methylesters ambo-242 in das Amid ambo-113 gelang in zwei Stufen und mit einer Gesamtausbeute von 77% (Schema 31).

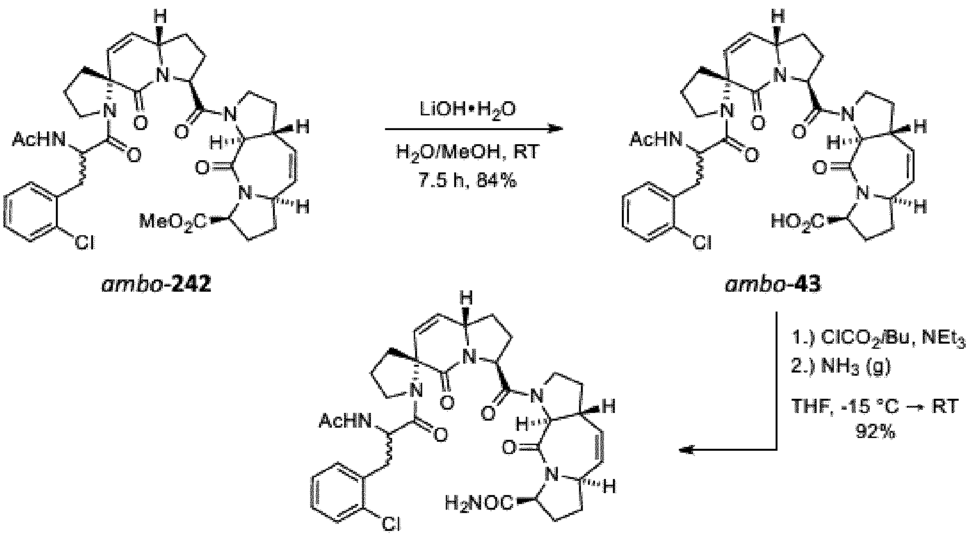


*ambo*-**242**

*ambo*-**43**

*ambo*-**113**

Schema 31: Synthese des Amids ambo-113 durch Verseifung des Methylesters ambo-242 und nachfolgender Amidierung der freien Säure ambo-43.

Zusammenfassend gelang es erstmals die ProM-Bausteine in der flüssigen Phase zu verknüpfen und so die Synthese des Ena/VASP-EVH1 Inhibitors ambo-113 in präparativem Maßstab zu verwirklichen.

**Optimierung der Kupplungsbedingungen - "Boc-Route"**

Obwohl die entwickelte Methodik einen effizienten Zugang zu den Ena/VASP-EVH1 Inhibitoren Ac-[L-2Cl-F]-[ProM-2]-[ProM-1]-OMe/OH/NH2 ambo-242, ambo-43 bzw. ambo-113 bot, lagen verschiedene Gründe für eine Optimierung der Kupplungssequenz vor. Daher wurde versucht, eine möglichst basenfreie Kupplungssequenz zu entwickeln, ohne dabei von der eigentlichen Strategie der Pfp-Ester allzu stark abzuweichen. Dafür bot sich ein "simpler" N-terminaler Schutzgruppenwechsel vom basisch spaltbaren Fluorenylmethoxycarbonyl (Fmoc) zur sauer entfernbaren tert-Butyloxycarbonyl (Boc) Funktionalität an. Die Verknüpfung der ProM-Scaffolds konnte nachfolgend durch eine an die "Fmoc-Route" angelehnte Strategie realisiert werden (Schema 32).

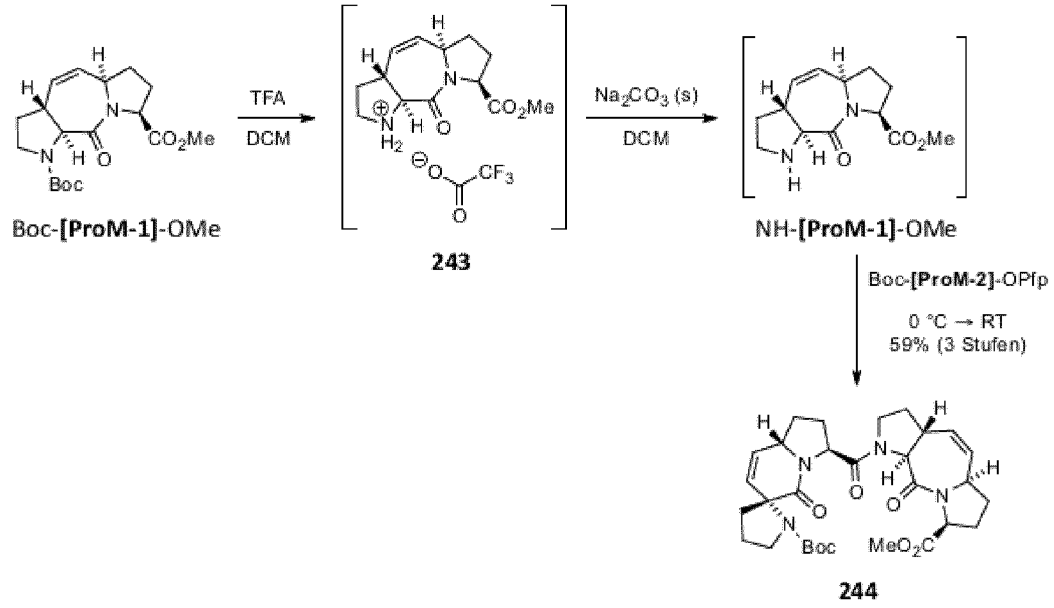


Boc-[ProM-1]-OMe

243

NH-[ProM-1]-OMe

244

Schema 32: N-terminale saure Entschützung von Boc-[ProM-1]-OMe mit TFA, Deprotonierung des resultierenden Ammonium-Kations mit festem Na2CO3 und abschließende Verknüpfung der ProM-Bausteine.

Zudem gelang es einen epimerisierungsfreien Zugang zu Ac-[L-2Cl-F]-[ProM-2]-[ProM-1]-OMe zu ermöglichen. Die entwickelte Methodik wurde an dieser Stelle entsprechend nachvollzogen (Schema 33).

sprechend nachvollzogen (Schema 4.60).

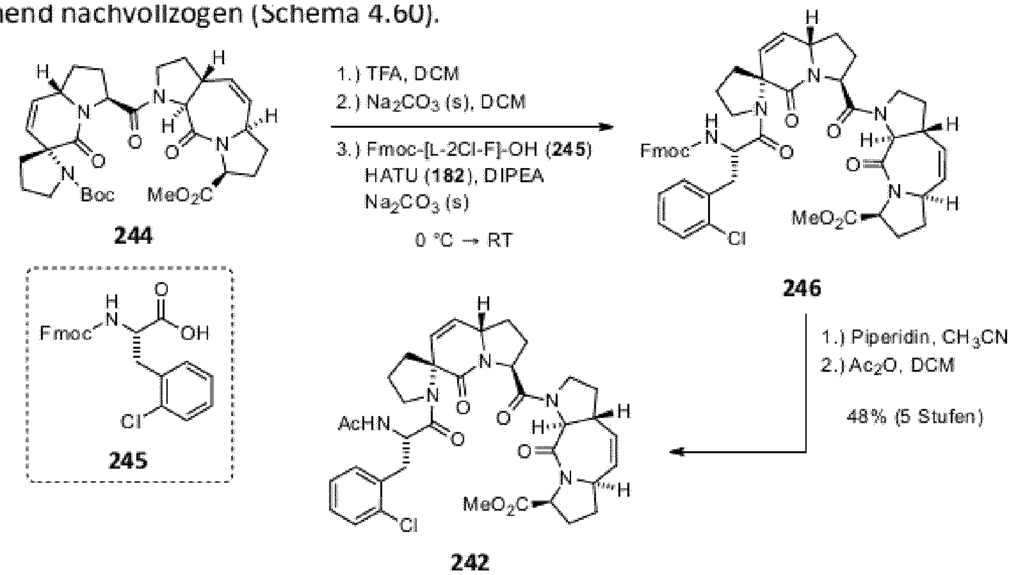


244

246

245

242

Schema 33: Finale Peptidkupplung zum Aufbau des diastereomerenreinen Peptidliganden 242 durch Verknüpfung des Tetrapeptids 244 mit Fmoc-[L-2Cl-F]-OH und nachfolgender Umschützung.

**Synthese ProM-15-haltiger Liganden**

Nach der Entwicklung einer effizienten Ligandensynthese in der Flüssigphase galt es abschließend auch ProM-15-haltige Peptidliganden zu synthetisieren und ihre Bindungsaffinität gegenüber der EVH1-Domäne zu evaluieren. Wie in Kapitel 3 bereits ausführlich dargelegt, sollte der ProM-15-Baustein als ein Ersatz für das bis dato verwendete ProM-1-Scaffold dienen. Es wurde dabei bewusst zunächst das Glycin-Derivat von ProM-15 gewählt, um Einflüsse eines weiteren Substituenten auf die Bindungsaffinität auszuschließen und einen optimalen Vergleich zu den ProM-1- bzw. ProM-9-haltigen Liganden anstellen zu können (Schema 34).

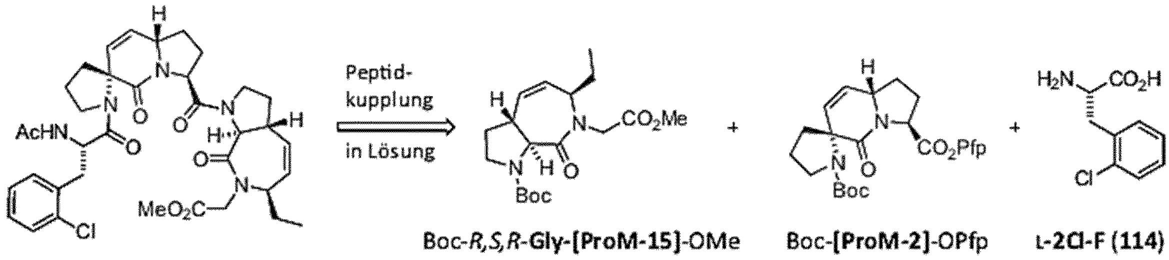


Boc-*R,S,R*-**Gly-[ProM-15]**-OMe + Boc-**[ProM-2]**-OPfp + **L-2Cl-F (114)**

**247**

Schema 34: Retrosynthetische Überlegungen zum Aufbau des ProM-15-haltigen Liganden 247.

Der Aufbau der resultierenden Zielstruktur Ac-[L-2Cl-F]-[ProM-2]-[R,S,R-Gly-ProM-15]-OMe (247) erfolgte zunächst durch die Verknüpfung der beiden ProM-Scaffolds (Schema 35).

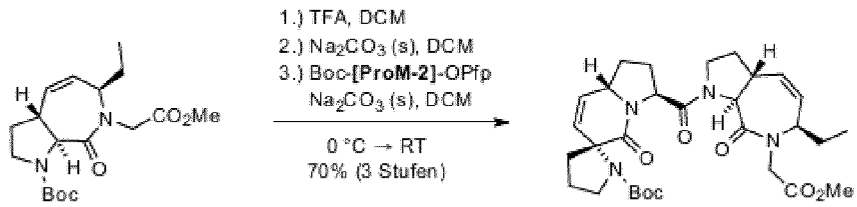


Boc-*R,S,R*-**Gly-[ProM-15]**-OMe **248**

Schema 35: Verknüpfung der ProM-Scaffolds zum Aufbau des Tetrapeptids 248 unter etablierten Bedingungen.

Das angestrebte Tetrapeptid 248 konnte komplikationslos unter den etablierten Bedingungen hergestellt und nach säulenchromatographischer Aufreinigung als weißer Feststoff in einer Ausbeute von 70% über 3 Stufen isoliert werden. Auch an dieser Stelle wurden die im NMR sichtbaren, doppelten Signalsätze durch H,H-NOESY-Spektroskopie auf das Vorliegen von Rotameren zurückgeführt. Insbesondere die zwei Signale des C! -Protons im ProM-2-Scaffold (4.79 ppm & 4.93 ppm) ließen sich eindeutig als Austauschsignale identifizieren.

Eine Diastereomerenreinheit des Peptids 248 kann somit auch hier als sehr wahrscheinlich angenommen werden und untermauert nochmals die Vermutung, dass die Kupplung unter den gewählten Bedingungen epimerisierungsfrei verläuft.

Die Synthese des ProM-15-Liganden 247 fiel zeitlich mit der Entdeckung der 2-Chlorphenylalanin-Racemisierung zusammen, wobei zu diesem Zeitpunkt nicht ausgemacht werden konnte, ob eine zeitnahe Lösung für diese Problematik zu finden wäre. Um im Rahmen dieser Arbeit dennoch Material für die angestrebten biologischen Untersuchungen (Bindungsmessungen zur, und Kristallisierung mit der EVH1-Domäne), bei denen das Vorliegen eines Diastereomerengemisches ohne Bedeutung war, zur Verfügung zu stellen, wurde das Tetrapeptid 248 zunächst mit dem racemischen Aktivester rac-240 gekuppelt (Schema 36).

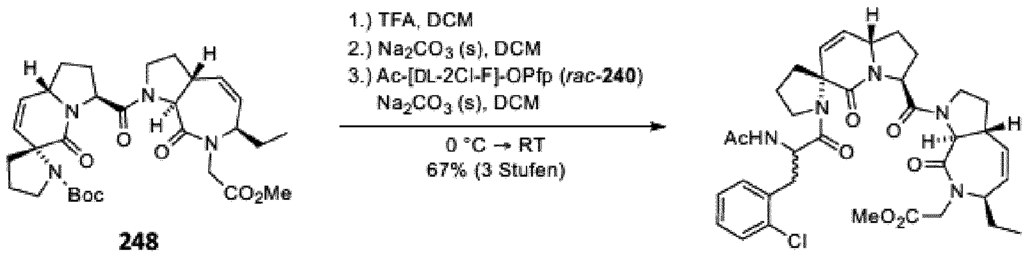


**248**

*ambo*-**247**

Schema 36: Finale Peptidkupplung zum Aufbau des Peptidliganden ambo-247 durch Verknüpfung des Tetrapeptids 248 mit racemischem Ac-[L-2Cl-F]-OPfp (rac-240).

Nach identischer Methodik wie für den ersten Kupplungsschritt gelang die Synthese des finalen Liganden ambo-247 als Diastereomerengemisch in einer vergleichbaren Ausbeute von 67% über 3 Stufen. Ein Diastereomerenverhältnis konnte aus dem komplexen NMR-Spektrum nicht bestimmt werden. Zudem waren die 1H- und 13C-NMR-Spektren von ambo-247 und der später synthetisierten Reinsubstanz 247, wie schon im Falle der ProM-1-Liganden ambo-242 und 242, deckungsgleich.

Neben dem Methylester ambo-247 sollte auch die freie Säure der Verbindung ambo-249 für die biologischen Untersuchungen bereitgestellt werden. Diese Verseifung gelang problemlos unter Einsatz von Lithiumhydroxid in wässrig-methanolischer Lösung bei RT in nur 4 h (Schema 37).

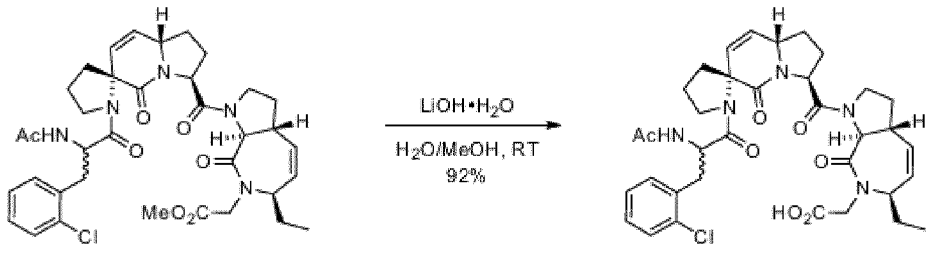


*ambo*-**247**

*ambo*-**249**

Schema 37: Verseifung des Methylesters ambo-247 zur Synthese der freien Säure ambo-249.

Das gewünschte Produkt ambo-249 wurde in einer Ausbeute von 92% als nahezu weißer Feststoff erhalten, der keiner weiteren Aufreinigung mehr bedurfte. Es konnte im Rahmen dieser Arbeit schlussendlich dennoch die Synthese des diastereomerenreinen Liganden 247 realisiert werden (Schema 38).

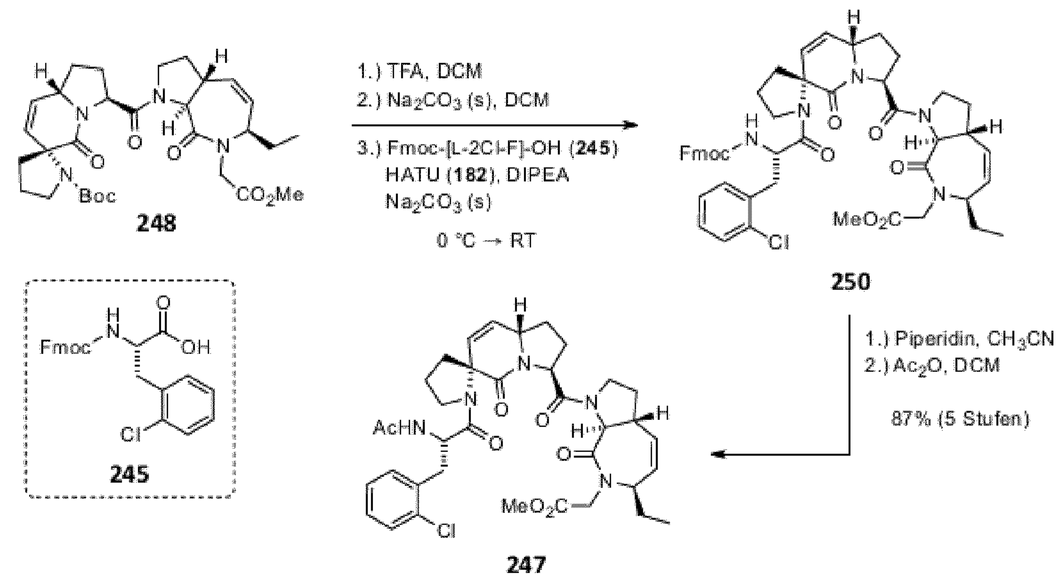


248

250

245

247

Schema 38: Finale Peptidkupplung zum Aufbau des diastereomerenreinen Peptidliganden 247 durch Verknüpfung des Tetrapeptids 248 mit Fmoc-[L-2Cl-F]-OH (245) und nachfolgender Umschützung.

Wie bereits für den ProM-1-Liganden 242 beschrieben, erfolgte auch hier nach N-terminaler Entschützung des Tetrapeptids 248 anschließend die HATU-vermittelte Kupplung mit Fmocgeschütztem L-2-Chlorphenylalanin 245. Die Abtrennung des entstehenden Harnstoffderivates war auch in diesem Fall säulenchromatographisch nicht möglich, stellte jedoch hier ebenso wenig ein Problem für die nachfolgende Umschützung dar und konnte schlussendlich komplikationslos vom finalen Liganden 247 abgetrennt werden. Die abschließende Acetylierung des Liganden erfolgte mit Essigsäureanhydrid, wonach die finale Zielverbindung dieser Arbeit, der Ena/VASP-EVH1 Inhibitor 247, diastereomerenrein in einer Ausbeute von 87% über 5 Stufen als leicht bräunlicher Feststoff isoliert werden konnte. Die deutlich verbesserte Ausbeute im Vergleich zur Synthese des ProM-1-Liganden 242 (48%) war hierbei auf einen Waschschritt mit Acetonitril zurückzuführen.

Das der Reaktion beigefügte Na2CO3 schien größere Mengen des freien Amins des entschützten Tetrapeptids 248 zu binden, die entsprechend für die Kupplung nicht mehr zur Verfügung standen. Durch Einsatz von Acetonitril konnte dies unterbunden und die Ausbeute deutlich erhöht werden. Dieser Waschschritt sollte folglich zukünftig bei allen Kupplungen durchgeführt werden, um mögliche Ausbeuteverluste zu minimieren.

**Biologische Untersuchungen**

Nach der erfolgreichen Synthese verschiedener neuer Ena/VASP-EVH1-Liganden galt es abschließend, deren Bindungsaffinität gegenüber der EVH1-Domäne zu evaluieren. Als Referenzsubstanz diente dabei Ligand Ac-[L-2Cl-F]-[ProM-2]-[ProM-1]-OEt.

Die Bindungsdaten der Liganden zur Domäne wurde mittels isothermaler Titrationskalorimetrie (ITC) oder Fluoreszenztitration (FT) bestimmt und in Form der Dissoziationskonstante (Kd) bzw. der freien Enthalpie (∆G0) dargestellt. Hierbei drückt sich eine stärkere Bindung des Liganden zur Domäne durch einen kleineren Kd-Wert bzw. entsprechend einen stärker negativen ∆G0-Wert aus. Der Zusammenhang beider Parameter ist in Gleichung (a) wiedergegeben:

$$K_\mathrm{d} = \frac{[Ligand][Domäne]}{[Komplex]} = e^{\frac{\Delta G^0}{RT}} \qquad \text{(a)}$$

Erste Bindungsstudien wurden für den im Rahmen dieser Arbeit per Flüssigphasenpeptidsynthese hergestellten EVH1-Ligand 113 angestellt, der verglichen mit dem "Ausgangs-ligand" 44, C-terminal eine Amidfunktion aufwies. Es wurde erwartet, dass durch diese Veränderung eine Verbindung mit ähnlicher Bindungsaffinität wie der bis dato genutzte Ethylester 44 generiert würde, welche jedoch eine höhere metabolische Stabilität aufweisen könnte. Entsprechend galt es zunächst die Affinität des Amid-Derivates 113 gegenüber der EVH1-Domäne zu untersuchen (Tabelle 1).

**Tabelle 1**: Bindungsaffinitäten verschiedener ProM-1-basierter Liganden bezüglich der EnaH-EVH1-Domäne. Angegeben sind die per isothermaler Titrationskalorimetrie (ITC) bestimmten Dissoziationskonstanten (Kd) mit den entsprechenden Standardfehlern in Klammern:

| # | Ligand | ITC $K_d$ [$\mu$M] |
|---|---|---|
| 1 | Ac-[L-2Cl-F]-**[ProM-2]-[ProM-1]**-OEt **(44)** | 7.8 (0.4) |
| 2 | Ac-[L-2Cl-F]-**[ProM-2]-[ProM-1]**-OMe **(242)** | 6.8 (0.7) |
| 3 | Ac-[L-2Cl-F]-**[ProM-2]-[ProM-1]**-$NH_2$ **(113)** | 7.0 (0.7) |

Hierbei wurde festgestellt, dass die Änderung der C-terminalen Maskierung, wie in silico erwartet, keinen Einfluss auf die Bindungsaffinität hatte. Sowohl der während der Flüssigphasenpeptidsynthese zuerst anfallende Methylester 242, als auch das daraus hergestellte finale Amid-Derivat 113, wiesen nahezu identische Bindungsaffinitäten wie der Ethylester 44 auf.

Da weiterführende Untersuchungen außerdem eröffneten, dass die Zellpermeabilitäten, aber auch die metabolischen Stabilitäten aller drei Derivate 44, 242 und 113 kaum unterschiedlich waren, wurde für zukünftige Arbeiten entschieden, von der finalen Umfunktionalisierung des Methylesters 242 zum Amid 113 abzusehen und die Synthesesequenz somit um zwei Stufen zu verkürzen.

Wie bereits dargelegt, ließen durchgeführte Computer-Modeling-Studien vermuten, dass der ProM-15-basierte Peptidligand 247, bei dem der ProM-1-Scaffold gegen einen ProM-15-Baustein ersetzt wurde, eine deutlich höhere Bindungsaffinität zur EVH1-Domäne aufweisen könnte. Durch seine ausgeprägte C-terminale Flexibilität sollte das ProM-15-Derivat 247 zur Ausbildung einer zusätzlichen Wasserstoffbrücke in der Lage sein und somit einen Affinitätsgewinn liefern. Ferner basierte das ProM-15-Design auf dem ProM-1-verwandten, allerdings durch Einführung einer zusätzlichen Methylgruppe "strukturell optimierten" ProM-9-Scaffold. Dieser adressierte mit drastischem Affinitätsgewinn eine hydrophobe Tasche der Domäne, die durch den flexiblen Ethylsubstituenten in ProM-15 ebenfalls angesprochen werden sollte. Durch Kombination mit der zusätzlichen Wasserstoffbrücke wurde entsprechend eine weitere Steigerung der Bindungsaffinität an die EVH1-Domäne erwartet, welche die des bereits fortgeschrittenen ProM-9-basierten Liganden nochmals übertreffen könnte.

Nach der erfolgreichen Synthese des ProM-15-Scaffolds und ferner des Liganden Ac-[L-2Cl-F]-[ProM-2]-[R,S,R-Gly-ProM-15]-OMe (247) konnten diese in silico postulierten Effekte nun abschließend experimentell untersucht werden.

In Tabelle 2 sind die Bindungsaffinitäten der relevanten Liganden zusammenfassend dargestellt.

Tatsächlich konnte durch Substitution von ProM-1 durch ProM-15 ein bemerkenswerter Affinitätsgewinn um den Faktor 9 - 10, verglichen mit den ProM-1-basierten Vorgängerliganden 44 und 242, erreicht werden. Der nanomolare Kd-Wert liegt hierbei allerdings in einem ähnlichen Bereich wie für den ProM-9-haltigen Liganden 251, womit der erwartete zusätzliche Affinitätsgewinn zunächst nicht erreicht wurde.

**Tabelle 2:** Bindungsaffinitäten verschiedener ProM-basierter Liganden bezüglich der EnaH-EVH1-Domäne. Angegeben sind die per Fluoreszenztitration (FT) bestimmten Dissoziationskonstanten (Kd) und die freien Enthalpien ($\Delta$G0) mit den entsprechenden Standardfehlern in Klammern:

| # | Ligand | FT | |
|---|---|---|---|
| | | $K_d$ [$\mu$M] | $\Delta G^0$ [kJ/mol] |
| 1 | Ac-[L-2Cl-F]-**[ProM-2]-[ProM-1]**-OEt **(44)** | 4.1 (0.3) | -30.8 (0.2) |
| 2 | Ac-[L-2Cl-F]-**[ProM-2]-[ProM-1]**-OMe **(242)** | 4.4 (0.7) | -30.6 (0.4) |
| 3 | Ac-[L-2Cl-F]-**[ProM-2]-[*R,S,R*-Gly-ProM-15]**-OMe **(247)** | 0.47 (0.03) | -36.1 (0.1) |
| 4 | Ac-[L-2Cl-F]-**[ProM-2]-[ProM-9]**-OEt **(251)** | 0.38 (0.05) | -36.6 (0.3) |

Die Bindungsaffinitäten weiterer ProM-basierter Liganden gegen die EnaH-EVH1-Domäne wurden getestet. Insbesondere wurden weitere Derivate auf Basis der ProM-15, ProM-17 und ProM-21-Scaffolds analysiert. Kombinationen zwischen ProM-1 oder ProM-2 zusammen mit ProM-15, ProM-17 oder ProM-21 zeigen besonders gute Affinitäten gegen die EnaH-EVH1-Domäne und weisen höhere Affinitäten zum bevorzugten Target auf, als die ProM-2-ProM-1-Derivate der früheren Generationen.

**Tabelle 3:** Bindungsaffinitäten weiterer ProM-basierter Liganden bezüglich der EnaH-EVH1-Domäne. Angegeben sind die per Fluoreszenztitration (FT) bestimmten Dissoziationskonstanten (Kd).

| **Ligand** | **$K_D$** |
|---|---|
| Ac-[2Cl-Phe]-[ProM-2]-[ProM-15]-OMe | $K_D$ = 0.47 μM |
| Ac-[2Cl-Phe]-[ProM-1]-[ProM-15]-OMe | $K_D$ = 0.9 μM |
| Ac-[2Cl-Phe]-[ProM-2]-[ProM-17]-OMe | $K_D$ = 0.54 μM |
| Ac-[2Cl-Phe]-[ProM-1]-[ProM-17]-OMe | $K_D$ = 2.2 μM |
| Ac-[2Cl-Phe]-[ProM-2]-[ProM-21]-OMe | $K_D$ = 0.26 μM |
| Ac-[2Cl-Phe]-[ProM-1]-[ProM-21]-OMe | $K_D$ = 2.1 μM |

Es gelang ferner Ac-[L-2Cl-F]-[ProM-2]-[R,S,R-Gly-ProM-15]-OMe (247) im Komplex mit EnaH-EVH1 zu kristallisieren und eine Kristallstruktur des Ligand-Domäne Komplexes zu erhalten. Fig. 15 zeigt eine Überlagerung der beiden Liganden 247 und 252 an der Proteinoberfläche, die Rückschlüsse auf die Bindungssituation zulässt.

Wie in Fig. 15 links zu erkennen, bindet der ProM-15-Ligand 247 kanonisch in der Bindungsfurche und die Strukturen der Liganden 247 und 252 liegen nahezu identisch auf der Oberfläche. Interessanterweise ist jedoch eine deutlich andere Ausrichtung der C-terminalen Carbonylfunktion zu erkennen (roter Pfeil), wobei diese im Falle der ProM-15-Verbindung 247 in Richtung der Proteinoberfläche zeigt, wohingegen sie beim ProM-9-Liganden 252 von dieser weg orientiert vorliegt.

Das rechte Bild zeigt einen detaillierteren Aufblick auf ProM-15 und ProM-9. Überraschenderweise weist die Ethylgruppe von ProM-15 entlang des Pyrrolidinrings von ProM-9 und nicht, wie erwartet, in Richtung des ProM-9-Methylsubstituenten (roter Pfeil). ProM-15 formt entsprechend den ursprünglichen Prolinring nach, anstatt die durch ProM-9 adressierte hydrophobe Tasche ebenfalls anzusprechen. Schlussfolgernd aus diesen Beobachtungen kann der erreichte drastische Affinitätsgewinn des ProM-15-Liganden 247 im Grunde nicht auf dieselben Effekte wie bei ProM-9 zurückgeführt werden, obwohl er im gleichen Bereich liegt. Da eine Adressierung der hydrophoben Region durch den Ethylsubstituenten scheinbar nicht erfolgt, ist der C-Terminus vermutlich hauptsächlich für den Affinitätsgewinn verantwortlich. Ein genauerer Blick auf die Elektronendichte des Liganden 247 in der Bindungstasche ist in Fig. 16 gezeigt.

Die Form der Elektronendichte um das C-terminale Carbonylsauerstoff lässt vermuten, dass die Carbonylfunktion in der gezeigten Orientierung auf dem Epitop liegt und entsprechend auf das Stickstoffatom des darunter befindlichen Tryptophan 23 (Trp23) zeigt. Messungen des Abstandes beider Atome eröffneten, dass die Entfernung zwischen diesen beiden Funktionalitäten optimal zur Ausbildung einer Wasserstoffbrücke über ein Wassermolekül wäre, was zudem dem gewonnenen Affinitätsgewinn entsprechen würde. Nicht zuletzt wurde durch das in silico-Design von ProM-15 mit seiner größeren C-terminalen Flexibilität genau auf die Bildung einer solchen Wasserstoffbrücke spekuliert, weshalb bis dato vermutet wird, dass diese Interaktion für den drastischen Affinitätsgewinn verantwortlich ist. Bis zur Fertigstellung dieser Arbeit war es noch nicht möglich die Kristallstruktur vollständig zu lösen, aber erste Verfeinerungen scheinen die Anwesenheit eines Wassermoleküls zwischen Trp23 und der Carbonylfunktion zu belegen und die postulierte Vermutung somit zu stützen. Detaillierte Auswertungen sind entsprechend in Arbeit und sollen alsbald kommuniziert werden.

Im Sinne der konzeptionellen Idee scheint der ProM-15-Baustein die bisher vielversprechendste strukturelle Optimierung des Ausgangsscaffolds ProM-1 darzustellen. Obwohl die in silico vorhergesagte Adressierung der hydrophoben Bindungstasche durch den Ethylsubstituenten anscheinend nicht erfolgte, war es vermutlich erstmals möglich durch die erhöhte C-terminale Flexibilität eine zusätzliche Wasserstoffbrücke mit der Domäne auszubilden. Hieraus resultierte ein signifikanter Affinitätsgewinn um den Faktor 9 - 10 und ein im (höheren) nanomolaren Bereich bindender Ligand 247. Aufgrund des einfachen modularen Aufbaus von ProM-15 sollte die neu entwickelte Palladium-katalysierte allylische Aminierung eine simple Modifikation des Ethylsubstituenten erlauben und somit die Adressierung der bis dato nicht angesprochenen hydrophoben Bindungstasche ebenfalls ermöglichen. Hierdurch könnte die ursprünglich angedachte Kombination der Bindungseffekte dennoch erreicht und ein weiterer Affinitätsgewinn an die EVH1-Domäne generiert werden.

## Zusammenfassung

Polyprolin-Typ2-Helix (PPII-Helix) vermittelte Protein-Protein-Wechselwirkungen zwischen sogenannten prolinreichen Motiven (PRMs) und den korrespondierenden Bindungsdomänen (PBDs) spielen bei vielen essentiellen biologischen Prozessen eine maßgebliche Rolle, weshalb deren Modulation von großem wissenschaftlichen und pharmakologischen Interesse ist. In diesem Kontext entwickelten die Erfinder rational designte, modular aufgebaute Diprolinanaloga in PPII-Konformation (ProMs), deren Nutzung als Pro-Pro-Äquivalent im Kernmotiv eines PRMs die gezielte Inhibition der oben genannten Interaktionen ermöglichte. Die vorliegende Arbeit liefert einen Beitrag zur Optimierung

des aus diesen Studien resultierenden, hoch selektiven Ena/VASP-EVH1 Inhibitors 43 (Schema 39).

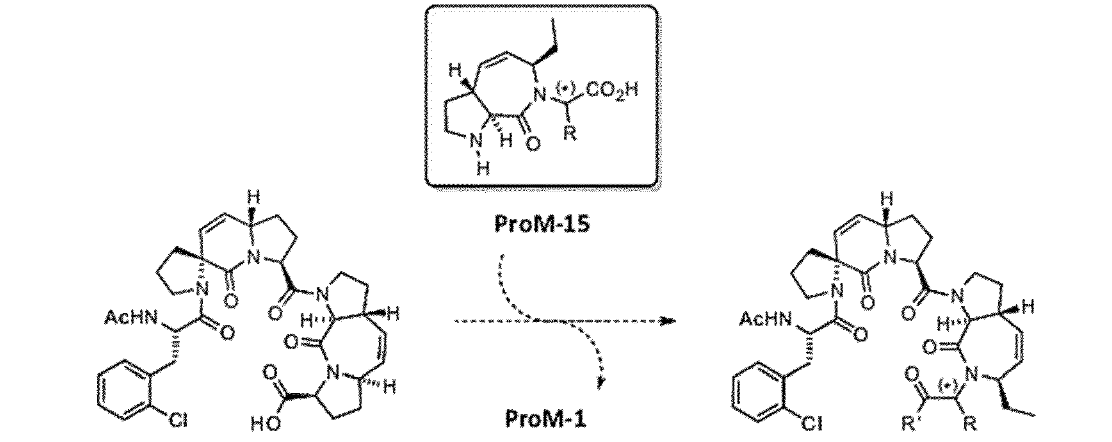

ProM-15

ProM-1

Ac-[L-2Cl-F]-[ProM-2]-[ProM-1]-OH (43)    Ac-[L-2Cl-F]-[ProM-2]-[ProM-15]-R′ (115)

Schema 39: Strukturelle Weiterentwicklung des Ena/VASP-EVH1 Inhibitors 43.

Die Substitution des Scaffolds ProM-1 durch die bicyclische Struktur ProM-15 sollte zu einem C-terminal signifikant flexibleren Peptidmimetikum 115 führen, das zusätzliche hydrophobe und hydrophile Wechselwirkungen mit der EVH1-Domäne ausbilden und somit eine deutlich gesteigerte Bindungsaffinität an diese aufweisen könnte. Neben der größeren Flexibilität bietet der in silico entworfene ProM-15-Baustein eine weitere variable Interaktionsstelle "R" an, über welche ebenfalls Wechselwirkungen mit der Domäne möglich sind, die aber auch eine Option zur Adressierung neuer PBDs darstellt.

Einer bereits etablierten, bewährten Synthesestrategie zum Aufbau von ProM-Bausteinen folgend, konnte ProM-15 durch Ringschlussmetathese und Peptidkupplung auf den bereits bekannten Säurebaustein 3 und die bis dato wenig erforschten Allylamine 112 zurückgeführt werden (Schema 40).

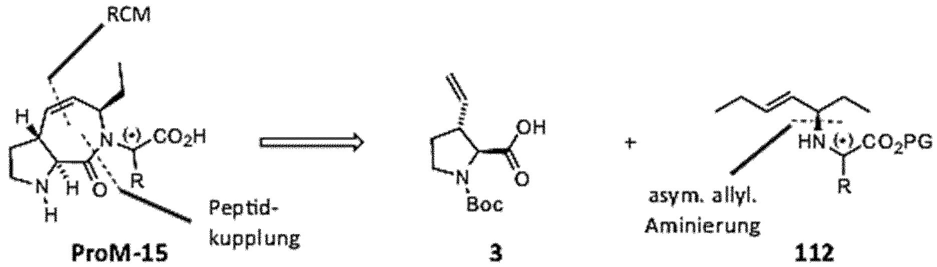


ProM-15    3    112

Schema 40: Retrosynthetische Überlegungen zum Aufbau des ProM-15-Scaffolds.

## Palladium-katalysierte asymmetrische allylische Aminierungen

Die stereoselektive Synthese N-allylierter Aminosäureester vom Typ 112 stellte entsprechend eine Herausforderung und die wesentliche Voraussetzung zur Bereitstellung von ProM-15 dar, welche durch die Entwicklung einer neuartigen, hoch selektiven, asymmetrischen allylischen Aminierung unter Verwendung des racemischen Carbonates rac-83 und diverser α-Aminosäureester 110 erfolgreich realisiert werden konnte (Schema 41).

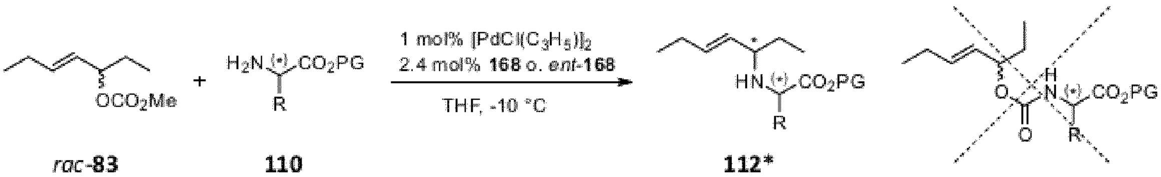


*rac*-**83** **110** **112*** *ambo*-**136**

(R = H, PG = *t*Bu → **153***)

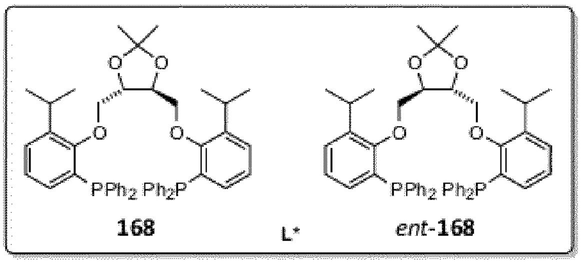

**168** L* *ent*-**168**

Schema 41: Synthese von Allylaminen des Typs 112* bzw 153* durch die in dieser Arbeit entwickelte Palladium-katalysierte asymmetrische N-Allylierung von α-Aminosäureestern 110. Unter den optimierten Reaktionsbedingungen konnte eine Bildung von Carbamat-Nebenprodukten ambo-136 vollständig unterdrückt werden.

Nach Optimierung der Reaktionsbedingungen gelang unter Einsatz von nur 1 mol% einer Palladium-Quelle und 2.4 mol% der chiralen Bisphosphinliganden 168 bzw. ent-168 die Synthese von insgesamt 19 (neuen) Allylaminen 112* und deren erstmalige vollständige Charakterisierung (Fig. 17). Die gefundenen Bedingungen ermöglichten nicht nur die Herstellung der Glycin-basierten Verbindung 153* in 95%ee, sondern auch das Erzielen exzellenter Selektivitäten und guter Ausbeuten bei Verwendung chiraler Nukleophile 110, wobei die wenigen in der Literatur beschriebenen Beispiele für ähnliche Transformationen deutlich übertroffen wurden.

**Stereoselektive Synthese neuer Dipeptidmimetika**

Ausgehend von den stereoselektiv hergestellten Allylaminen 112* gelang nachfolgend die Synthese von sieben neuen ProM-15-Scaffolds unter Anwendung der etablierten Strategie, wobei die finalen Schritte auf das neue System adaptiert bzw. optimiert wurden.

Schema 42 zeigt exemplarisch die Synthese des Glycin-basierten ProM-15 mit anvisierter absoluter Konfiguration, sowie die weiteren neuen Dipeptidanaloga:

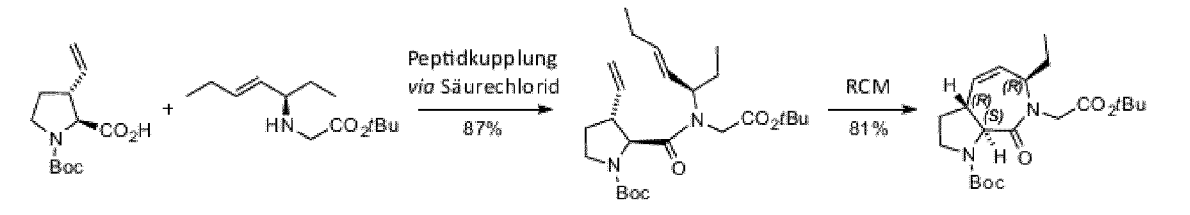


3 153 183 Boc-*R*,*S*,*R*-**Gly-[ProM-15]**-O*t*Bu

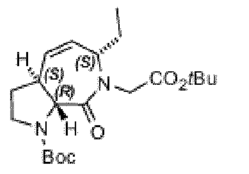

Boc-*S*,*R*,*S*-**Gly-[ProM-15]**-O*t*Bu

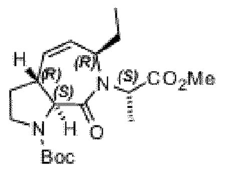

Boc-*R*,*S*,*R*-**Ala-[ProM-15]**-OMe

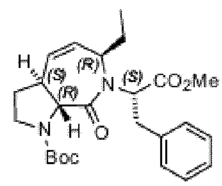

-*S*,*R*,*R*,*S*-

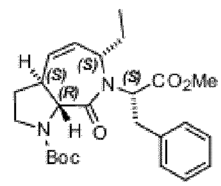

-*S*,*R*,*S*,*S*-

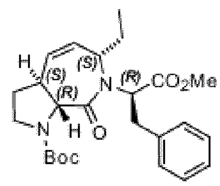

-*S*,*R*,*S*,*R*-

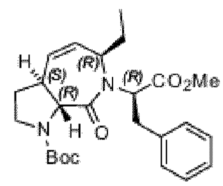

-*S*,*R*,*R*,*R*-

Boc-**Phe-[ProM-15]**-OMe

Schema 42: Synthesestrategie zum Aufbau von ProM-15 und die hierdurch in dieser Arbeit hergestellten Strukturen.

Da die Peptidkupplung von 3 und 153 unter den bis dato genutzten "Standardbedingungen" nur unbefriedigende Ergebnisse lieferte und im Zuge der Verwendung sterisch noch anspruchsvollerer Allylamine 112* mit R ≠ H (siehe Schema 5.2) vollkommen versagte, stellte die Realisierung dieser Verknüpfung ein herausforderndes Problem dar. Die Lösung der Kupplungsproblematik gelang erst nach intensiver Untersuchung diverser Aktivierungsreagenzien und präziser Interpretation der Ergebnisse. Die gefundenen Bedingungen (Kupplung via des Säurechlorids von 3) ermöglichten nicht nur die Synthese des Dipeptids 183 in exzellenter Ausbeute, sondern erlaubten zudem die sehr erfolgreiche Verknüpfung der Zaminer-Säure (3) mit den sterisch gehinderten Aminbausteinen 112* (R ≠ H). Der finale Ringschluss wurde ebenfalls durch Variation diverser Reaktionsparameter deutlich optimiert, sodass die sterisch anspruchsvolle Metathese in allen Fällen mit nur 6 mol% Katalysator in moderaten bis guten Ausbeuten realisiert werden konnte.

Alle neu hergstellen ProM-15-Scaffolds konnten abschließend außerdem kristallisiert und die relative Konfiguration der Stereozentren durch Röntgenkristallstrukturanalyse belegt werden.

**Ligandensynthese in der Flüssigphase**

Im Rahmen dieser Erfindung konnte zusätzlich die Synthese der finalen ProM-basierten PPII-Peptidmimetika in der Flüssigphase realisiert werden. Das Interesse an einer solchen, im Multigrammmaßstab durchführbaren Syntheseroute beruhte auf der Notwendigkeit große Substanzmengen für angestrebte in vivo-Experimente bereitzustellen, die über die bisher genutzte Festphasenpeptidsynthese nicht zugänglich waren. Es konnte ausgehend vom kommerziell erhaltlichen L-2-Chlorphenylalanin (114) und den bereits bekannten Bausteinen Fmoc-[ProM-2]-OH (dessen Synthese ebenfalls optimiert wurde) und Fmoc-[ProM-1]-OH eine effiziente Kupplungsmethodik basierend auf Pentafluorphenolestern entwickelt werden (Schema 43).

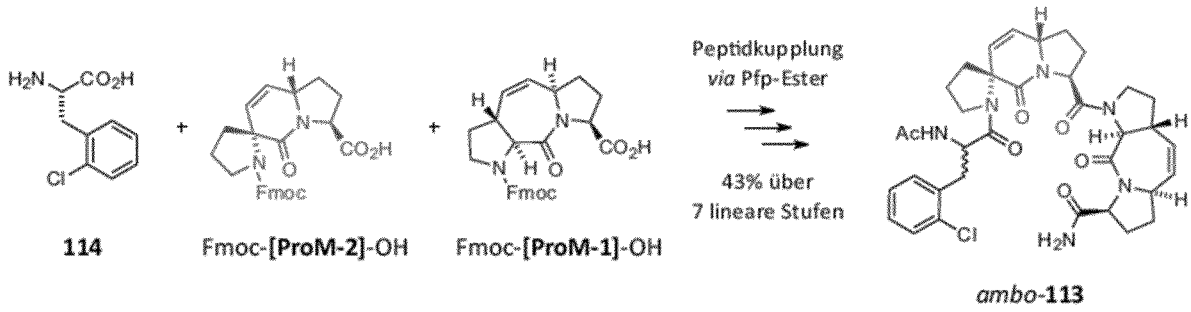


114 Fmoc-[ProM-2]-OH Fmoc-[ProM-1]-OH *ambo*-113

Schema 43: Aufbau des Peptidliganden ambo-113 ausgehend von L-2-Chlorphenylalanin (114) und den beiden ProMScaffolds Fmoc-[ProM-2]-OH und Fmoc-[ProM-1]-OH.

Der Aufbau des Liganden 113 erfolgte unter milden Bedingungen, nach Methylveresterung des ProM-1-Scaffolds und Bereitstellung der aktivierten Kupplungspartner, vom C-Terminus aus, wonach final umfunktionalisiert wurde. Allerdings erwies sich die gefundene Methodik aus mehreren Gründen als nicht optimal, wobei die Epimerisierung des 2-Chlorphenyl-alanin-Stereozentrums den größten Nachteil darstellte. Nichtsdestotrotz konnten, bei einer Gesamtausbeute von 43% über sieben lineare Stufen ausgehend vom ProM-1-Baustein, zunächst insgesamt circa 2.5 g des Peptidliganden ambo-113, wenn auch als Diastereomerengemisch, synthetisiert und für die angestrebten in vivo-Experimente bereitgestellt werden.

Anschließend gelang eine erste Optimierung der Synthesesequenz, die eine Umstellung auf Boc-Schutzgruppen und eine Kombination aus Pfp-Ester- und HATU-Aktivierung umfasste, wodurch der gewünschte Ligand nun diastereomerenrein zugänglich war. Die verbesserte Methodik wurde abschließend auch zur Herstellung eines ProM-15-haltigen Liganden 247 genutzt, dessen Synthese in Schema 44 dargestellt ist:

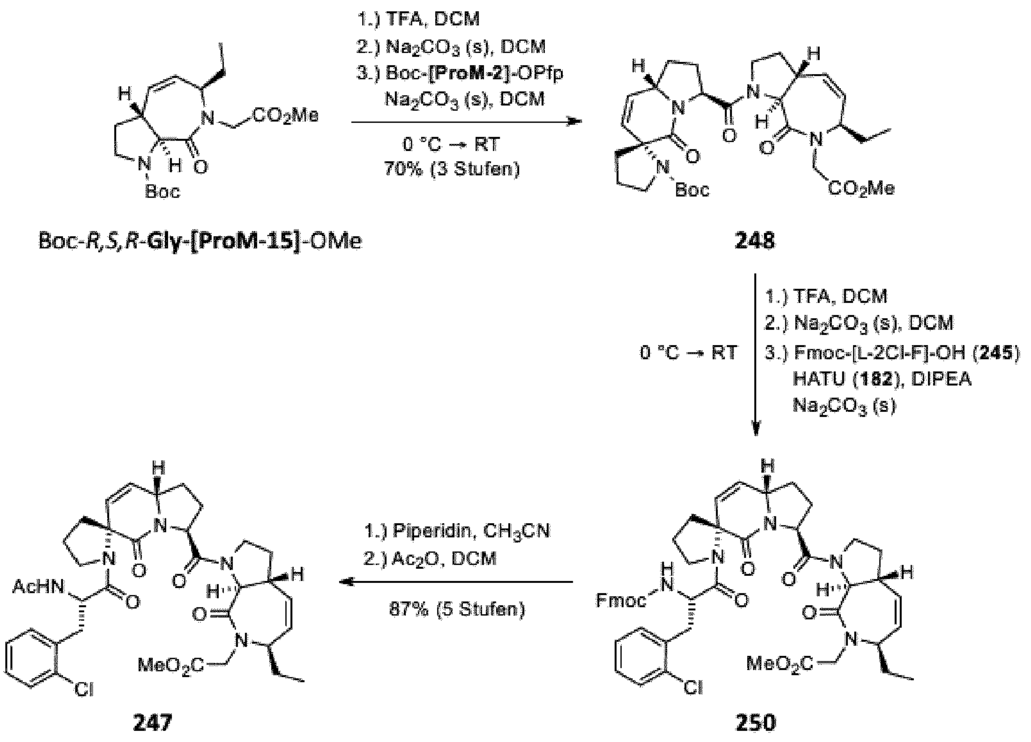


Boc-*R,S,R*-Gly-[ProM-15]-OMe 248 247 250

Schema 44: Synthese des Liganden Ac-[L-2Cl-F]-[ProM-2]-[R,S,R-Gly-ProM-15]-OMe (247) unter optimierten Kupplungsbedingungen.

**Biologische Untersuchungen des ProM-15-haltigen Liganden 247**

Wie einleitend zu diesem Kapitel bereits angedeutet, ließen durchgeführte Computer-Modeling-Studien vermuten, dass ein ProM-15-basierter Peptidligand durch seine ausgeprägte C-terminale Flexibilität eine deutlich höhere Bindungsaffinität zur EVH1-Domäne aufweisen könnte, als die starre, ProM-1-enthaltende Vorgängerversion. Ferner flossen Erkenntnisse aus dem ProM-1-verwandten, allerdings "strukturell optimierten" ProM-9-Scaffold in das ProM-15-Design mit ein (Schema 45).

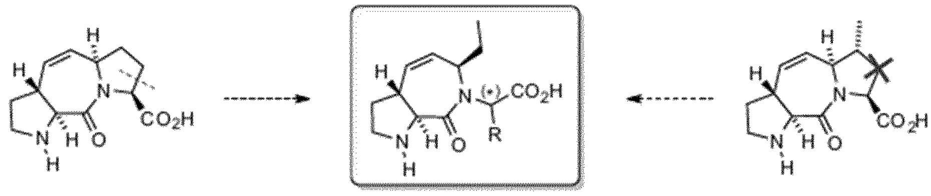

ProM-1 ProM-15 ProM-9

Schema 45: Ableitung des ProM-15-Scaffolds aus den bekannten Strukturen ProM-1 und ProM-9.

Durch Einführung einer zusätzlichen Methylgruppe konnte mit ProM-9 eine hydrophobe Tasche der Domäne angesprochen und somit ein Peptidligand mit beeindruckend gesteigerter Bindungsaffinität erzeugt werden.

Der flexible Ethylsubstituent in ProM-15 sollte diese Interaktionsfläche ebenfalls adressieren und durch Kombination mit einer zusätzlich neu ausgebildeten Wasserstoffbrücke zu einem weiteren Affinitätsgewinn führen.

Nachdem höchst erfolgreich ein Zugang zu ProM-15 gefunden und der Peptidligand 247 synthetisiert wurde, konnten die in silico postulierten Effekte im FMP in Berlin experimentell überprüft werden.

Die neue Verbindung Ac-[L-2Cl-F]-[ProM-2]-[R,S,R-Gly-ProM-15]-OMe (247) wies, verglichen mit den ProM-1-basierten Vorgängerliganden 44 bzw. 242, tatsächlich eine, um den Faktor 9 - 10, eindrucksvoll gesteigerte Bindungsaffinität zur EVH1-Domäne auf und lag somit im gleichen Bereich, wie die bis dato bestbindende ProM-9-Version 251.

Aufgrund der Ausrichtung der C-terminalen Carbonylfunktion wurde der drastische Affinitätsgewinn auf die Ausbildung der angestrebten Wasserstoffbrücke zurückgeführt, wenngleich die zweite Interaktion des Ethylsubstituenten mit der hydrophoben Tasche der Domäne nicht erreicht werden konnte. Obwohl die Kombination beider Effekte scheiterte, scheint ProM-15 im Sinne der konzeptionellen Idee trotzdem die bisher vielversprechendste strukturelle Optimierung des ProM-1-Scaffolds darzustellen. Die Ausbildung der neuen Wasserstoffbrücke und der dadurch erreichte signifikante Affinitätsgewinn können zunächst als großer Erfolg angesehen werden, wobei die im Rahmen dieser Arbeit entwickelte Palladium-katalysierte allylische Aminierung eine Derivatisierung von ProM-15 in einfacher Weise erlauben sollte (Schema 46).

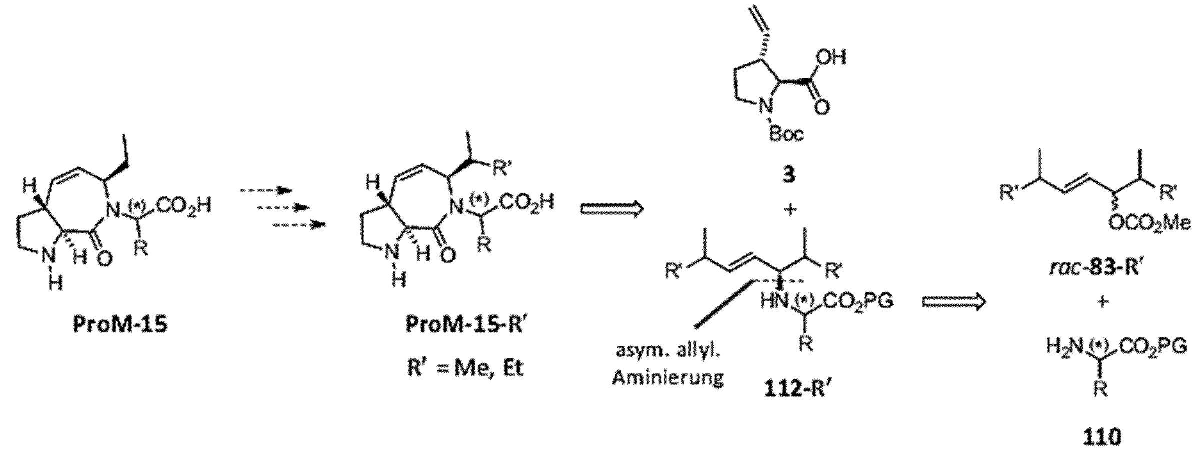


ProM-15 ProM-15-R' R' = Me, Et 3 112-R' rac-83-R' 110

Schema 46: Eine Weiterentwicklung des ProM-15-Scaffolds könnte durch eine Verzweigung des Ethylsubstituenten erfolgen. Diese Derivatisierung sollte in einfacher Weise durch Verwendung eines leicht modifizierten Katalysesubstrates rac-83-R' zugänglich sein.

Durch Wahl eines entsprechend modifizierten Katalysesubstrates rac-83-R' sollte eine gezielte Einführung verschiedener Substituenten gelingen. Als sinnvolle Strukturvorschläge könnten die neuen ProM-15-R'-Derivate beispielsweise eine verzweigte iso-Propyl- oder iso-Butyl-Gruppe tragen, wodurch die Adressierung der hydrophoben Tasche ermöglicht werden sollte

**Synthese des Peptidliganden Ac-[L-2Cl-F]-[ProM-2]-[R,S,R-Gly-ProM-15]-OMe (247)**

Synthese von Boc-[ProM-2]-[R,S,R-Gly-ProM-15]-OMe (248)

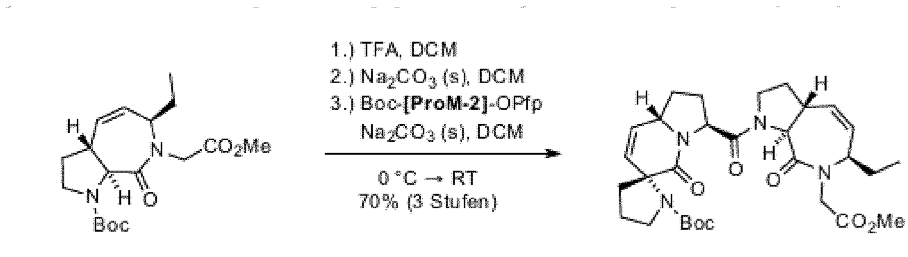


Boc-*R,S,R*-Gly-[ProM-15]-OMe 248

Unter nicht inerten Bedingungen wurde eine Lösung von 220 mg (0.624 mmol, 1 Äq.) Boc-R,S,R-Gly-[ProM-15]-OMe in 1.4 ml DCM bei 0 °C tropfenweise mit 0.72 ml (9.36 mmol, 15 Äq.) TFA versetzt. Das Eisbad wurde entfernt, 1.5 h bei RT gerührt, nachfolgend alle flüchtigen Bestandteile bei vermindertem Druck entfernt, zweimal aus DCM wieder eingeengt und schlussendlich im Vakuum getrocknet. Der erhaltene Rückstand wurde in 12.5 ml DCM aufgenommen, mit 3 Spateln festem Na2CO3 versetzt (Feststoff verklebt etwas) und 5 Min. gerührt. Danach wurde filtriert, das Lösungsmittel bei vermindertem Druck entfernt und im Vakuum getrocknet.

Der nun erhaltene Rückstand wurde unter Argon-atmosphäre in 12.5 ml abs. DCM aufgenommen, erneut 3 Spatel festes Na2CO3 zugegeben und anschließend 376 mg (0.749 mmol, 1.2 Äq.) Boc-[ProM-2]-OPfp zugefügt. Nach Rühren über Nacht (15 h) bei RT wurde filtriert und das Lösungsmittel bei vermindertem Druck entfernt. Durch säulenchromatographische Aufreinigung an Kieselgel (DCM/MeOH = 20/1) und Trocknen im Vakuum bei 60 °C konnten 250 mg (0.438 mmol, 70% über 3 Stufen) des gewünschten Tetrapeptids 248 als weißer Feststoff isoliert werden.

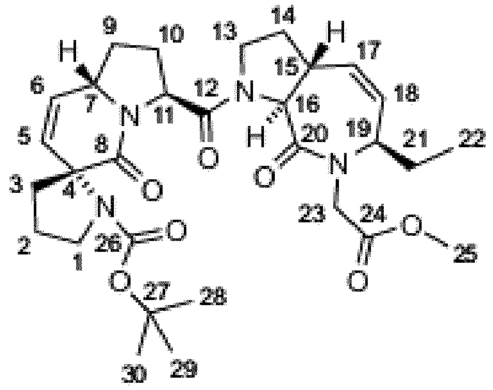

$C_{30}H_{42}N_4O_7$

M: 570.68 g/mol.

DC: Rf = 0.22 (Silica, DCM/MeOH = 20/1), KMnO4-Reagenz.

1H-NMR (500 MHz, CDCl3, Rotamerengemisch95 ): δ [ppm] = 1.01 (t,3J = 7.2 Hz, 3H, H-22) 1.35, 1.44 (2 × s, 9H, H-28/29/30); 1.52 - 1.63 (m, 1.55H, H-9rot1/21); 1.69 - 1.82 (m, 2.45H, H-2/9rot2/21'); 1.84 - 1.93 (m, 2H, H-3/14); 1.95 - 2.03 (m, 1H, H-2'); 2.12 - 2.22 (m, 3H, H-9'/10/14'); 2.26 - 2.31 (m, 1H, H-3'); 2.40 - 2.46 (m, 1H, H-10'); 2.94 - 3.03 (m, 1H, H-15); 3.43 - 3.52 (m, 1H, H-1); 3.56 - 3.70 (m, 4.45H, H-1'rot2/13/25); 3.73 - 3.78 (m, 1.55H, H-1'rot1/23); 4.24 (Ψ t, J = 8.8 Hz, 0.45H, H-13'rot2); 4.30 (Ψ t, J = 8.7 Hz, 0.55H, H-13'rot1); 4.35 - 4.39 (m, 1H, H-7); 4.68 - 4.81 (m, 2.55H, H-11rot1/19/23'); 4.93 (Ψ t, J = 8.1 Hz, 0.45H H-11rot2); 5.11 (Ψ t, J = 11.9 Hz, 1H, H-16); 5.56 - 5.59 (m, 1H, H-17); 5.74 - 5.79 (m, 1.1H, H-5rot1/6rot1); 5.83-5.89 (m, 1.9H, H-5rot2/6rot2/18).

13C-NMR (125 MHz, CDCl3, Rotamerengemisch95 ): δ [ppm] = 11.7 (C-22); 22.7, 23.1 (C-2); 26.2, 26.2 (C-21); 28.3 (C-10); 28.5, 28.7 (C-28/29/30); 31.1, 31.1 (C-14); 31.4, 31.5 (C-9); 38.6, 39.8 (C-3); 42.3 (C-15); 43.2 (C-23); 47.6, 47.7 (C-13); 48.1, 48.5 (C-1); 52.1, 52.1 (C-25); 55.4, 55.5 (C-19); 56.3, 56.3 (C-11); 58.9, 59.0 (C-7); 60.3, 60.3 (C-16); 64.1, 64.3 (C-4); 79.5, 79.7 (C-27); 121.9, 123.1 (C-5 o. C-6); 130.7, 130.7, 130.8 (C-17/18); 133.0, 133.6 (C-5 o. C-6); 154.2, 154.4 (C-26); 168.2, 168.4 (C-8); 170.4, 170.4 (C-24); 171.7, 171.9 (C-12); 172.2 (C-20).

HR/MS (ESI): berechnet für: [M+H]+ 571.3126; gefunden: 571.3130; berechnet für [M+Na]+ 593.2946; gefunden: 593.2943.

**Synthese von Fmoc-[L-2Cl-F]-[ProM-2]-[R,S,R-Gly-ProM-15]-OMe (250)**

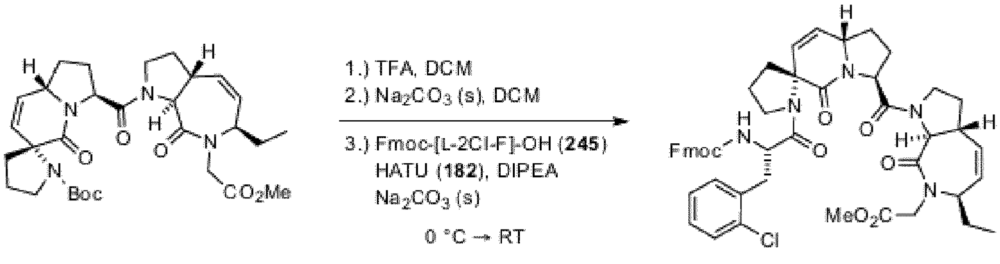


**248** **250**

1.) Synthese des Aktivesters: Unter Argonatmosphäre wurde eine Lösung von 318 mg (0.753 mmol, 2 Äq.) Fmoc-[L-2Cl-F]-OH (245) und 358 mg (0.942 mmol, 2.5 Äq.) HATU in 4.7 ml abs. DCM bei RT mit 0.13 ml (0.753 mmol, 2 Äq.) DIPEA versetzt und 45 Min. gerührt (trübe, leicht gelbliche Lösung).

2.) Synthese des Liganden 250: Unter nicht inerten Bedingungen wurden 215 mg (0.377 mmol, 1 Äq.) des Tetrapeptids 248 in 0.87 ml DCM gelöst und bei 0 °C tropfenweise mit 0.44 ml (5.65 mmol, 15 Äq.) TFA versetzt. Das Eisbad wurde entfernt und 1 h bei RT gerührt, nachfolgend alle flüchtigen Bestandteile bei vermindertem Druck entfernt, zweimal aus DCM wieder eingeengt und schlussendlich im Vakuum getrocknet. Der erhaltene Rückstand

wurde in 5 ml DCM aufgenommen, mit 3 Spateln festem Na2CO3 versetzt (Feststoff verklebt etwas) und 5 Min. gerührt. Danach wurde filtriert, der bräunliche Filterkuchen mit wenig CH3CN nachgewaschen (braune Verfärbung löst sich), das Lösungsmittel bei vermindertem Druck entfernt und im Vakuum getrocknet. Der nun erhaltene Rückstand wurde unter Argonatmosphäre in 4.7 ml abs. DCM aufgenommen und mit wenigen Tropfen trockenem CH3CN versetzt (gerade so viel, dass sich der polare Feststoff löst). Danach wurden zunächst 3 Spatel festes Na2CO3 und anschließend die Lösung des zuvor synthetisierten Aktivesters (siehe oben) zugegeben.

Nach Rühren über Nacht bei RT wurde das Na2CO3 über wenig Silica abfiltriert (Eluent: DCM/MeOH = 15/1), das Lösungsmittel bei vermindertem Druck entfernt und der Rückstand säulenchromatographisch an Kieselgel (DCM/MeOH = 15/1) aufgereinigt. Nach Trocknen im Vakuum konnten 486 mg (150%) eines leicht bräunlichen Feststoffes isoliert werden.

Es war an dieser Stelle nicht möglich, das gewünschte Produkt 250 vollständig aufzureinigen. Sowohl die entstehende Harnstoffspezies (Rf = 0.35, Silica, DCM/MeOH = 15/1, KMnO4), als auch Spuren von DIPEA (Rf = 0.22, Silica, DCM/MeOH = 15/1, KMnO4) konnten säulenchromatographisch nicht abgetrennt werden. Da diese Verunreinigungen jedoch keinen Einfluss auf die Folgechemie hatten, wurden zur Vermeidung von Ausbeuteverlusten keine hiermit verunreinigten Produktfraktionen verworfen. Das 1H-NMR-Spektrum des bräunlichen Feststoffes zeigte die zu erwartenden charakteristischen Signale des Liganden 250, sowie die Signale der genannten Verunreinigungen. Aus diesem Grund wurde hier auf eine vollständige Charakterisierung und Bestimmung der Ausbeute verzichtet. Für den Folgeschritt wurde eine quantitative Reaktion angenommen.

$C_{49}H_{52}ClN_5O_8$, M: 874.42 g/mol.

DC: Rf = 0.29 (Silica, DCM/MeOH = 15/1), KMnO4-Reagenz.

HR/MS (ESI): berechnet für: [M+H]+ 874.3577; gefunden: 874.3591; berechnet für [M+Na]+ 896.3397; gefunden: 896.3403.

**Synthese von Ac-[L-2Cl-F]-[ProM-2]-[R,S,R-Gly-ProM-15]-OMe (247)**

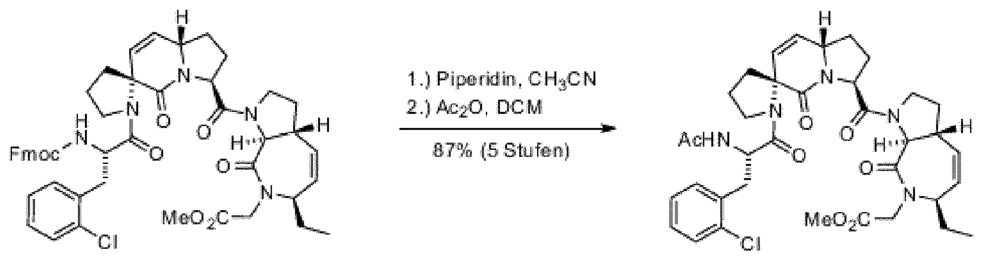


250 247

Unter Argonatmosphäre wurde der zuvor synthetisierte Ligand 250 (6.19.2) in 6.8 ml trockenem CH3CN gelöst und bei RT tropfenweise mit 1.7 ml Piperidin versetzt (entspricht 20% Piperidin in CH3CN).

Nach 1 h Rühren bei RT erfolgte ein Lösungsmittelwechsel an der Schlenk-Line: Zunächst wurden CH3CN und Piperidin vorsichtig entfernt und bis zur Trockenheit eingeengt. Danach wurde der Rückstand in ~2 ml DCM aufgenommen, erneut vom Lösungsmittel und Piperidinresten befreit und diese Prozedur ein weiteres Mal wiederholt. Anschließend wurde der Rückstand unter Argonatmosphäre in 15.3 ml abs. DCM aufgenommen und bei RT tropfenweise mit 1.7 ml Essigsäureanhydrid versetzt (entspricht 10% Ac2O in DCM). Nach Rühren über Nacht wurden alle flüchtigen Bestandteile bei vermindertem Druck entfernt, nochmals aus DCM wieder eingeengt und das Rohprodukt nachfolgend säulenchromatographisch an Kieselgel (DCM/MeOH = 15/1) aufgereinigt. Nach Trocknen im Vakuum konnten 227 mg (0.327 mmol, 87% über 5 Stufen) des gewünschten Liganden 247 als leicht bräunlicher Feststoff isoliert werden.

$C_{36}H_{44}ClN_5O_7$, M: 694.22 g/mol.

DC: Rf = 0.17 (Silica, DCM/MeOH = 15/1), KMnO4-Reagenz.

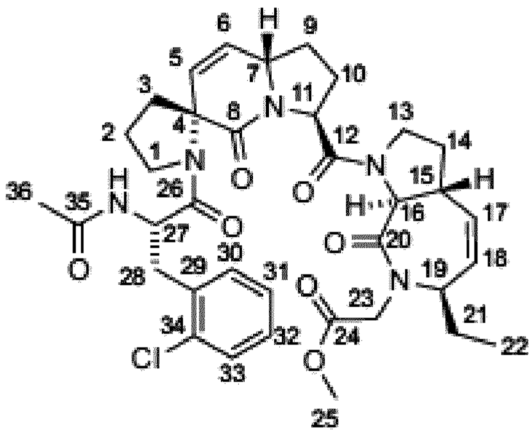

1H-NMR (600 MHz, CDCl3, Rotamerengemisch98 ): δ [ppm] = 0.98 - 1.02 (m, 3H, H-22); 1.54 - 1.62 (m, 1H, H-21); 1.68 - 2.22 (m, 12H, H-2/3/9/10/14/21736); 2.29 (Ψ dt, J = 12.2 Hz, J = 5.9 Hz, 0.3H, H-3'rot2); 2.38 (Ψ dt, J = 12.9 Hz, J = 6.4 Hz, 0.7H, H-3'rot1); 2.45 - 2.50 (m, 0.7H, H-10'rot1); 2.53 - 2.58 (m, 0.3H, H-10'rot2); 2.85 (dd,2J = 14.2 Hz,3J = 10.7 Hz, 0.7H, H-28rot1); 2.92 - 3.03 (m, 1.3H, H-15/28rot2); 3.14 (dd,2J = 13.0 Hz,3J = 6.1 Hz, 0.3H, H-28'rot2);3.36 (dd,2J = 14.2 Hz,3J = 3.8 Hz, 0.7H, H-28'rot1); 3.44 - 3.48 (m, 0.3H, H-1 rot2); 3.54 - 3.61 (m, 1H, H-13); 3.67, 3.67 (2 × s, 3H, H-25); 3.76 - 3.80 (m, 1H, H-23); 3.89 - 3.96 (m, 1.7H, H-1rot1/1'); 4.10 (Ψ t, J = 9.0 Hz, 0.3H, H-13'rot2); 4.19 (Ψ t, J = 8.8 Hz, 0.7H, H-13'rot1); 4.32 - 4.36 (m, 0.3H, H-7rot2); 4.38 - 4.41 (m, 0.7H, H-7rot1); 4.67 - 4.76 (m, 2.6H, H-5rot2 o.6rot2/19/23'/27rot2); 4.83 (Ψ t, J = 8.3 Hz, 0.3H, H-11rot2); 4.96 (Ψ t, J = 8.1 Hz, 0.7H, H-11rot1); 5.06 (d,3J = 12.1 Hz, 0.3H, H-16rot2); 5.11 -5.17 (m, 1.4H, H-16rot1/27rot1); 5.54-5.89 (m, 3.7H, H-5rot1/6 o. H-5/6rot1 und H-17/18); 6.13 (d,3J = 10.3 Hz, 0.3H, NHrot2); 6.17 (d,3J = 8.8 Hz, 0.7H, NHrot1); 7.13 - 7.34 (m, 4H, H-30/31/32/33).

13C-NMR (150 MHz, CDCl3, Rotamerengemisch98 ): δ [ppm] = 11.7, 11.7 (C-22); 22.2, 24.1 (C-2); 23.0, 23.0 (C-36); 26.2 (C-21); 28.2, 28.5 (C-10); 31.0, 31.0, 31.2, 31.4 (C-9/14); 36.0, 37.9 (C-28); 38.2, 41.1 (C-3); 42.3, 42.3 (C-15); 43.2 (C-23); 47.5 (C-13); 48.7, 48.8 (C-1); 50.5, 50.7 (C-27); 52.1 (C-25); 55.4, 55.6 (C-19); 56.4, 57.0 (C-11); 59.2, 59.6 (C-7); 60.2, 60.3 (C-16); 64.7, 65.5 (C-4); 123.1, 125.6 (C-5 o. C-6); 126.6, 126.8, 128.6, 128.6, 129.4, 129.6 (C-31/32/33); 130.0, 130.6, 130.8, 130.8, 131.3 (C-5 o. C-6/17/18); 131.8, 132.0 (C-30); 134.4, 134.6, 134.7, 135.4 (C-29/34); 166.7, 167.1 (C-8); 169.7, 170.2, 170.4, 171.3, 171.7 (C-12/24/26/35); 172.1, 172.2 (C-20).

HR/MS (ESI): berechnet für: [M+H]+ 694.3002; gefunden: 694.3014; berechnet für [M+Na]+ 716.2821; gefunden: 716.2824.

**Weitere Synthese der erfindungsgemäßen Liganden**

Im Zuge dieser Arbeiten wurde eine verbesserte Methodik zum Zusammenbau der ProM-Bausteine entwickelt, die nicht nur weniger Schritte benötigt, sondern vor allem die Produkte in höherer Ausbeute und Reinheit liefert. Die neue verbesserte Ligandensynthese unterscheidet sich von den bisherigen Methoden darin, dass die Substanzen von "links nach rechts" ausgerichtet sind bzw. hergestellt werden. Zunächst wird N-Fmoc-geschützes (L)-2-Chlorphenylalanin mit dem "ersten" (zentralen) ProM-Baustein verknüpft, bevor nach Esterhydrolyse der "rechte" (C-terminale) ProM-Baustein angehängt wird. Abschließend wird die Fmoc-Gruppe gegen die Acetylgruppe (oder auch gegen andere Acylreste) ausgetauscht.

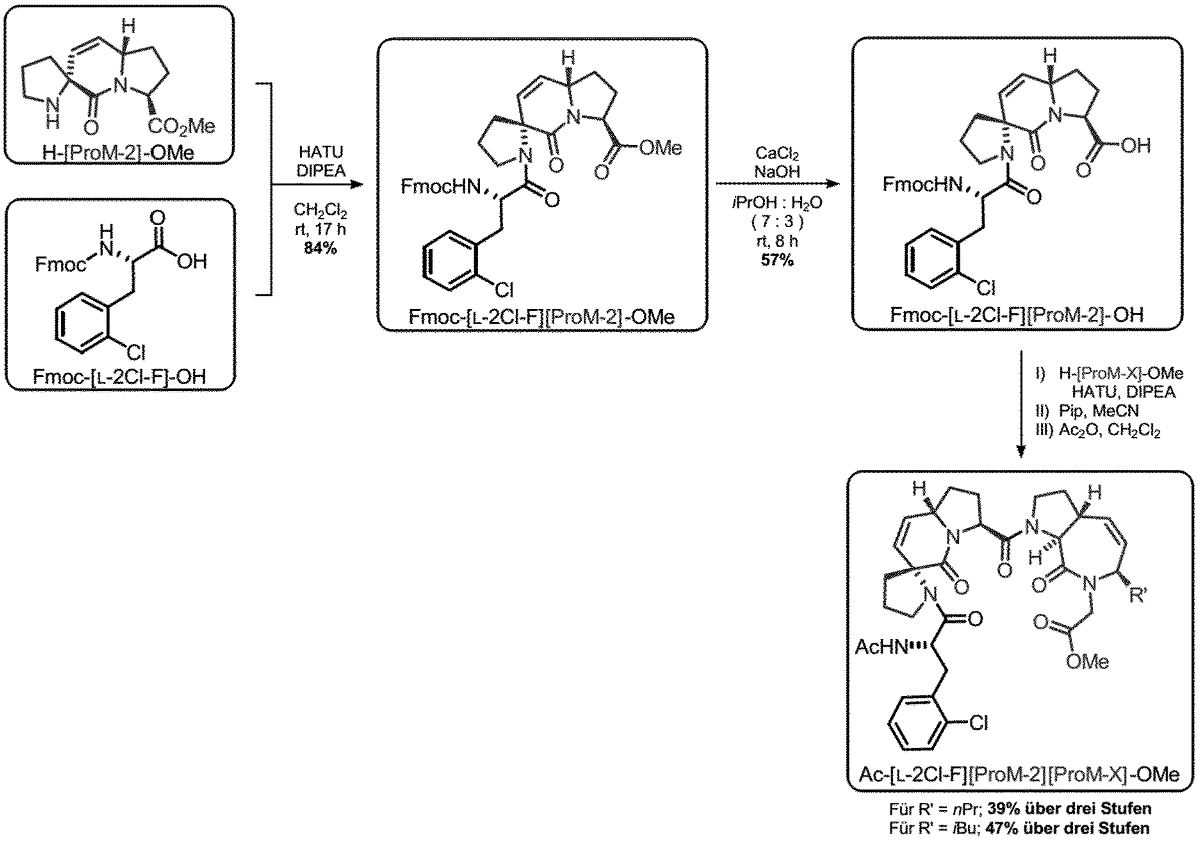


**Synthese des ProM-21-Bausteins:**

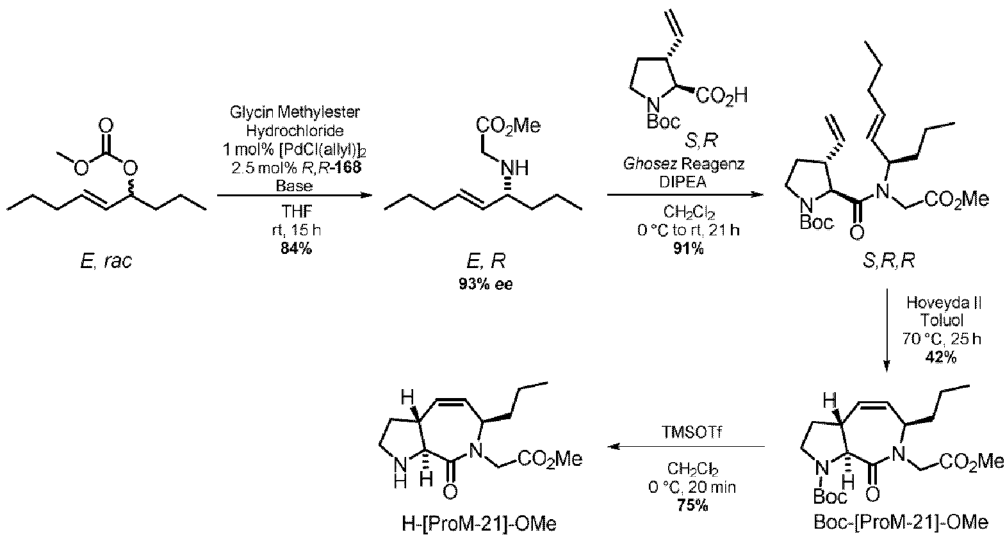


**Charakteristische Daten von H-[ProM-17]-OMe**

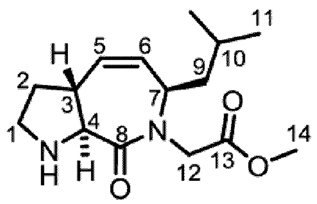

H-[ProM-17]-OMe

$C_{15}H_{24}N_2O_3$.

280.368 g/mol.

**TLC:** ($SiO_2$, $CH_2Cl_2$/MeOH 20:1): $R_f$ = 0.13.

**$^1$H-NMR:** (500 MHz, $CDCl_3$) δ = 5.90 (dt, $^3J$ = 11.1, $^4J$ = 2.4 Hz, 1H, H5), 5.49 (dt, $^3J$ = 11.1, $^4J$ = 3.0 Hz, 1H, H6), 4.75 - 4.70 (m, 1H, H7), 4.46 (d, $^3J$ = 11.9 Hz, 1H, H4), 4.41 (d, $^3J$ = 17.5 Hz, 1H, H12a), 3.99 (d, $^3J$ = 17.6 Hz, 1H, H12b) 3.71 (s, 3H, H14), 3.22 (dd, $^3J$ = 9.5, $^3J$ = 4.7 Hz, 2H, H1), 3.11 (bs, 1H, -N*H*), 2.62 - 2.55 (m, 1H, H3), 2.23 - 2.18 (m, 1H, H2a), 1.81 - 1.72 (m, 1H, H2b), 1.63 (dp, $^3J$ = 13.2, $^3J$ = 6.5 Hz, 1H, H10), 1.55 - 1.42 (m, 2H, H9), 0.96 (Ψdd, $^3J$ = 15.8, $^3J$ = 6.5 Hz, 6H, H11).

**$^{13}$C-NMR:** (125 MHz, $CDCl_3$) δ = 174.3 (s, C8), 170.3 (s, C13), 131.9 (d, C5), 130.3 (d, C6), 62.1 (d, C4), 52.3 (q, C14), 51.7 (d, C7), 45.4 (d, C3), 45.2 (t, C1), 43.7 (t, C12), 42.1 (t, C9), 32.5 (t, C2), 25.1 (d, C10), 23.2 (q, C11a), 21.8 (q, C11b).

**FT-IR:** (ATR) ν [$cm^{-1}$] = 3371 (b), 2954 (m), 2871 (w), 1751 (m), 1657 (s), 1455 (m), 1436 (m), 1384 (m), 1302 (w), 1260 (m), 1201 (s), 1177 (s), 1125 (m), 1076 (w), 1042 (w), 1008 (m), 938 (w), 913 (w), 823 (w), 775 (m), 734 (w), 690 (m), 635 (m), 561 (m).

**GC-MS:** m/z (%) = 280 (12), 251 (5), 223 (17), 195 (44), 179 (5), 158 (100), 137 (5), 122 (44), 106 (24), 91 (19), 69 (29), 41 (26).

**HR-MS (ESI):**

| Exakte Masse [u] | Gemessene Masse [u] | Fehler [ppm] |
|---|---|---|
| 281.1859 $[M+H]^+$ | 281.1859 $[M+H]^+$ | + 0.06 |
| 303.1679 $[M+Na]^+$ | 303.1678 $[M+Na]^+$ | - 0.23 |

**$[\alpha]_\lambda^{20}$:** (0.50 g/100 ml in $CHCl_3$): $[\alpha]_{365}$ = -278.6 °, $[\alpha]_{436}$, = -175.6 °, $[\alpha]_{546}$ = -103.5 °, $[\alpha]_{579}$ = - 90.8 °, $[\alpha]_{589}$ = -88.1 °.

**Charakteristische Daten von H-[ProM-21]-OMe**

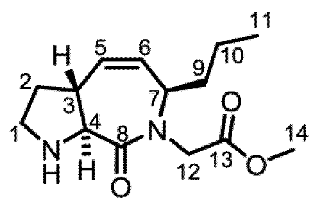

H-[ProM-21]-OMe

$C_{14}H_{22}N_2O_3$.

266.341 g/mol.

**TLC:** ($SiO_2$, CyHex/EtOAc/MeOH 2:2:1): $R_f$ = 0.10.

**$^1$H-NMR:** (600 MHz, $CDCl_3$) δ = 5.90 (dt, $^3J$ = 11.1 Hz, $^4J$ = 2.4 Hz, 1H, H5), 5.52 (dt, $^3J$ = 11.1 Hz, $^4J$ = 3.0 Hz, 1H, H6), 4.65 -4.62 (m, 1H, H7), 4.44 - 4.35 (m, 2H, H4 und H12a), 4.04 (d, $^3J$ = 17.6 Hz, 1H, H12b), 3.71 (s, 3H, H14), 3.23 - 3.17 (m, 2H, H1), 2.60 - 2.54 (m, 1H, H3), 2.22 -2.17 (m, 1H, H2a), 1.75 (tt, $^3J$ = 12.3 Hz, $^3J$ = 9.5 Hz, 1H, H2b), 1.70 - 1.50 (m, 2H, H9), 1.50 - 1.30 (m, 2H, H10), 0.98 (t, $^3J$ = 7.3 Hz, 3H, H11).

**$^{13}$C-NMR:** (125 MHz, $CDCl_3$) δ = 174.4 (s, C8), 170.4 (s, C13), 132.0 (d, C5), 130.1 (d, C6), 62.1 (d, C4), 53.8 (d, C7), 52.3 (q, C14), 45.4 (d, C3), 45.1 (t, C1), 43.7 (t, C12), 35.2 (t, C9), 32.6 (t, C2), 20.3 (t, C10), 13.9 (q, C11).

**FT-IR:** (ATR) ν [$cm^{-1}$] = 3321 (b), 3020 (w), 2956 (w), 2873 (w), 1751 (m), 1651 (s), 1454 (m), 1436 (m), 1384 (m), 1301 (w), 1253 (m), 1202 (s), 1178 (s), 1163 (s), 1114 (m), 1078 (w), 1047 (w), 1009 (m), 943 (w), 909 (w), 890 (w), 845 (w), 783 (m), 732 (m), 689 (m), 638 (m), 558 (m), 485 (m).

**GC-MS:** m/z (%) = 266 (27), 237 (16), 223 (49), 209 (10), 195 (53), 179 (5), 165 (10), 144 (100), 122 (31), 108 (26), 94 (15), 80 (24), 56 (6), 41 (11).

**HR-MS (ESI):**

| Exakte Masse [u] | Gemessene Masse [u] | Fehler [ppm] |
|---|---|---|
| 267.1703 $[M+H]^+$ | 267.1704 $[M+H]^+$ | + 0.24 |
| 289.1522 $[M+Na]^+$ | 289.1523 $[M+Na]^+$ | + 0.20 |

**Charakteristische Daten von Ac-[I-2CI-F][ProM-1][ProM-15]-OMe**

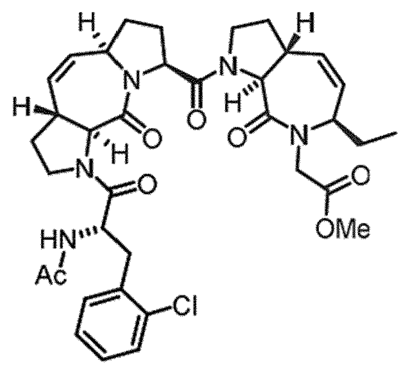

Ac-[L-2Cl-F][ProM-1][ProM-15]-OMe

$C_{36}H_{44}N_5O_7Cl_1$.

694.226 g/mol.

**$^{13}$C-NMR:** (125 MHz, $CDCl_3$) δ = 172.2, 171.2, 171.0, 170.9, 170.8, 170.5, 170.5, 169.6, 168.8, 168.7, 135.2, 134.8, 134.7, 134.6, 132.2, 132.0, 130.8, 130.7, 130.6, 130.0, 129.5, 129.5, 129.7, 128.8, 128.6, 128.0, 127.9, 126.9, 126.8, 62.8, 62.3, 60.3, 60.2, 59.1, 59.0, 58.0, 55.5, 55.4, 52.1, 51.6, 50.5, 47.9, 47.7, 46.9, 43.2, 42.4, 42.0, 40.6, 38.6, 37.0, 33.4, 33.2, 31.8, 31.1, 29.9, 27.3, 26.2, 23.5, 23.2, 22.8, 14.3, 11.8, 11.8.

**HR-MS (ESI):**

| Exakte Masse [u] | Gemessene Masse [u] | Fehler [ppm] |
|---|---|---|
| 694.3002 $[M+H]^+$ | 694.3012 $[M+H]^+$ | + 1.46 |
| 716.2821 $[M+Na]^+$ | 716.2823 $[M+Na]^+$ | + 0.33 |

**Charakteristische Daten von Ac-[I-2CI-F][ProM-1][ProM-17]-OMe**

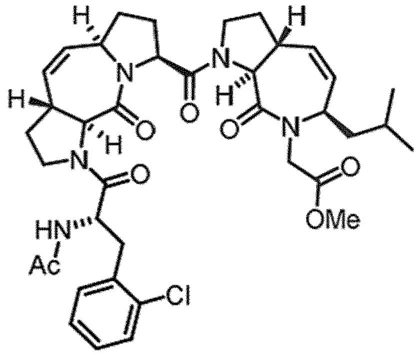

Ac-[L-2Cl-F][ProM-1][ProM-17]-OMe

$C_{38}H_{48}N_5O_7Cl_1$.

722.280 g/mol.

**$^{13}$C-NMR:** (125 MHz, $CDCl_3$) $\delta$ = 172.3, 171.1, 171.0, 170.9, 170.7, 170.0, 168.9, 168.9, 134.7, 134.6, 134.5, 132.2, 132.1, 130.9, 130.8, 130.7, 130.6, 129.9, 129.5, 129.5, 129.2, 128.9, 128.6, 128.1, 128.1, 127.0, 126.9, 62.8, 62.3, 60.3, 59.2, 58.1, 52.2, 51.6, 50.7, 48.0, 47.7, 47.0, 46.1, 43.3, 42.4, 42.2, 40.6, 38.6, 36.8, 33.4, 33.2, 32.1, 31.8, 31.1, 31.0, 29.9, 27.25 , 25.1, 23.6, 23.1, 22.7, 22.1, 21.7, 14.3.

**HR-MS (ESI):**

| Exakte Masse [u] | Gemessene Masse [u] | Fehler [ppm] |
|---|---|---|
| 722.3315 $[M+H]^+$ | 722.3319 $[M+H]^+$ | + 0.53 |
| 744.3134 $[M+Na]^+$ | 744.3132 $[M+Na]^+$ | - 0.26 |

**Charakteristische Daten von Ac-[I-2Cl-F][ProM-2][ProM-17]-OMe**

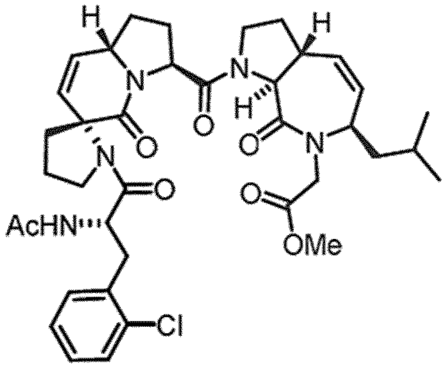

Ac-[L-2Cl-F][ProM-2][ProM-17]-OMe

$C_{38}H_{48}N_5O_7Cl_1$.

722.280 g/mol.

**$^{13}$C-NMR:** (125 MHz, $CDCl_3$) $\delta$ = 172.3, 172.2, 171.6, 171.6, 171.1, 170.5, 170.46, 170.2, 169.4, 168.2, 167.2, 166.7, 135.4, 134.9, 134.7, 134.6, 132.2, 132.1, 131.8, 131.5, 131.0, 130.9, 130.7, 130.1, 129.6, 129.4, 128.5, 126.8, 126.6, 125.5, 123.1, 77.4, 77.0, 65.5, 64.6, 60.3, 60.2, 59.6, 59.2, 57.1, 56.4, 52.2, 52.1, 51.5, 51.5, 50.6, 50.4, 48.7, 47.5, 43.3, 42.4, 42.4, 42.2, 41.2, 38.3, 38.0, 36.2, 31.5, 31.0, 29.8, 28.2, 25.1, 24.1, 23.4, 23.3, 23.2, 22.8, 22.2, 21.9, 21.9, 14.3.

**HR-MS (ESI):**

| Exakte Masse [u] | Gemessene Masse [u] | Fehler [ppm] |
|---|---|---|
| 722.3315 $[M+H]^+$ | 722.3320 $[M+H]^+$ | + 0.75 |
| 744.3134 $[M+Na]^+$ | 744.3132 $[M+Na]^+$ | -0.28 |

**Charakteristische Daten von Ac-[I-2Cl-F][ProM-1][ProM-21]-OMe**

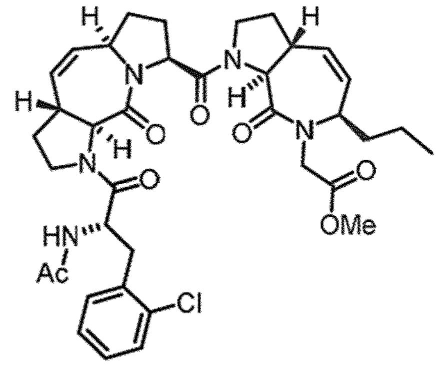

Ac-[L-2Cl-F][ProM-1][ProM-21]-OMe

$C_{37}H_{46}N_5O_7Cl_1$.

708.253 g/mol.

**$^{13}$C-NMR:** (125 MHz, $CDCl_3$) $\delta$ = 172.2, 171.1, 171.0, 170.9, 170.5, 170.5, 169.6, 168.8, 168.7, 168.7, 135.1, 134.8, 134.7, 134.6, 132.2, 132.0, 130.9, 130.8, 130.8, 130.7, 130.0, 129.5, 129.5, 129.2, 128.8, 128.6, 128.0, 127.9, 126.9, 126.8, 77.4, 62.8, 62.3, 60.2, 59.1, 59.0, 59.0, 58.1, 58.0, 53.6, 52.1, 52.1, 51.6, 50.5, 47.9, 47.7, 46.9, 43.3, 42.4, 42.0, 40.6, 37.0, 35.4, 33.3, 33.2, 31.8, 31.1, 31.0, 29.9, 27.3, 23.5, 23.2, 23.1, 20.4, 14.0.

**HR-MS (ESI):**

| Exakte Masse [u] | Gemessene Masse [u] | Fehler [ppm] |
|---|---|---|
| 708.3158 $[M+H]^+$ | 708.3165 $[M+H]^+$ | + 0.95 |
| 730.2978 $[M+Na]^+$ | 730.2972 $[M+Na]^+$ | - 0.75 |

**Charakteristische Daten von Ac-[I-2Cl-F][ProM-2][ProM-21]-OMe**

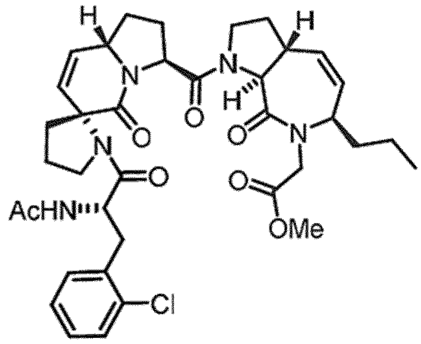

Ac-[L-2Cl-F][ProM-2][ProM-21]-OMe

$C_{37}H_{46}N_5O_7Cl_1$.

708.253 g/mol.

**$^{13}$C-NMR:** (125 MHz, $CDCl_3$) $\delta$ = 172.2, 172.1, 171.7, 171.5, 171.3, 171.1, 170.4, 170.2, 169.3, 167.1, 166.6, 135.4, 134.9, 134.6, 134.5, 132.0, 131.8, 131.5, 131.1, 130.6, 130.1, 129.5, 129.4, 128.5, 126.7, 126.5, 125.5, 123.0, 77.4, 77.2, 76.9, 65.5, 64.6, 60.3, 60.2, 59.6, 59.2, 57.0, 56.4, 56.3, 53.7, 53.6, 52.9, 52.2, 52.1, 50.6, 50.3, 48.7, 48.7, 47.5, 43.2, 43.1, 42.3, 41.5, 41.2, 38.5, 38.3, 38.0, 36.2, 35.3, 32.1, 31.4, 31.2, 31.1, 29.8, 28.6, 28.2, 24.1, 23.3, 23.2, 22.8, 22.2, 20.3, 19.8, 14.0, 14.0.

**HR-MS (ESI):**

| Exakte Masse [u] | Gemessene Masse [u] | Fehler [ppm] |
|---|---|---|
| 708.3158 $[M+H]^+$ | 708.3162 $[M+H]^+$ | + 0.56 |
| 730.2978 $[M+Na]^+$ | 730.2974 $[M+Na]^+$ | - 0.44 |

## Patentansprüche

1. Verbindung gemäß Formel I:

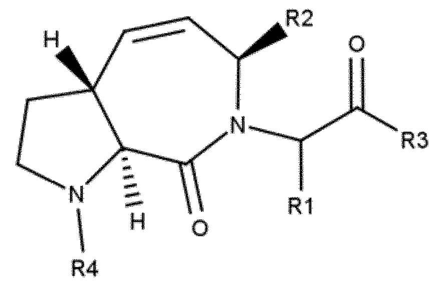

wobei

R1: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise einen R-Rest einer Aminosäure, wobei die genannten Reste optional substituiert sind,
R2: Alkyl, Aryl, Alkoxyalkyl, Cycloalkyl, Mercaptoalkyl, Sulfidoalkyl, Arylalkyl, Fluoralkyl
R3: OH, Alkyl, O-Alkyl, O-Aryl, NRR' (R, R': H oder Alkyl), NROR' (R, R': H, Alkyl), Heteroaryl,
R4: H, Alkyl, Acyl, Aryl, Heteroaryl, Cycloalkyl, Alkylheteroaryl, Alkylcarbonyl, Arylalkyl, Arylcarbonyl, Alkoxycarbonyl, Aminocarbonyl, Aryloxycarbonyl, Alkylsulfonyl, Arylsulfonyl, Aminoacyl und/oder Peptidyl, oder R5, wobei die genannten Reste optional substituiert sind,
R5:

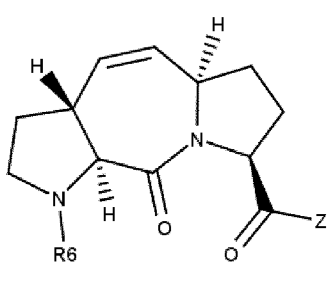

or

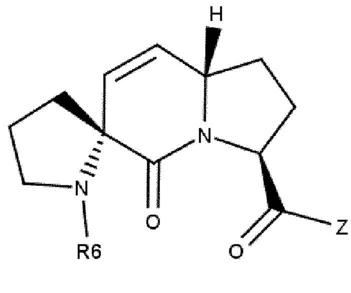

Z: Bindung zur Struktur gemäß Formel I,
R6: H, Alkyl, Aryl, Cycloalkyl, Alkylcarbonyl, Arylcarbonyl, Alkoxycarbonyl, Arylalkyl, Aminocarbonyl, Aryloxycarbonyl, Alkylsulfonyl, Arylsulfonyl, Aminoacyl und/oder Peptidyl, wobei die genannten Reste optional substituiert sind, oder

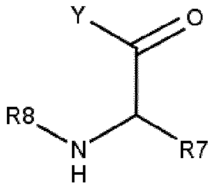

Y: Bindung zur Struktur gemäß R5,
R7: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise ein R-Rest einer Aminosäure, wobei die genannten Reste optional substituiert sind,
R8: H, Alkyl, Aryl, Acyl, Heteroaryl, Cycloalkyl, Alkyl-Heteroaryl, Alkylcarbonyl, Arylalkyl, Arylcarbonyl, Alkoxycarbonyl, Aminocarbonyl, Aryloxycarbonyl, Alkylsulfonyl, Arylsulfonyl, Aminoacyl und/oder Peptidyl, wobei die genannten Reste optional substituiert sind.

**2.** Verbindung nach Anspruch 1 gemäß Formel II-a oder Formel II-b:

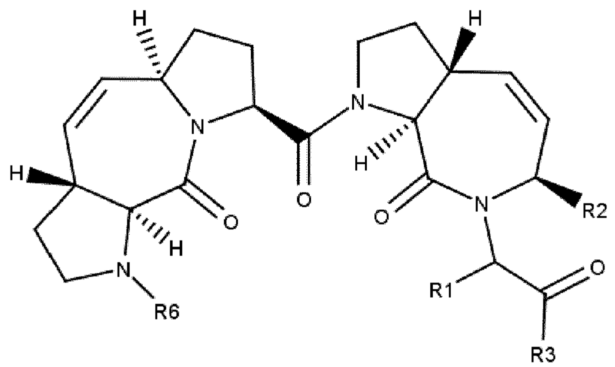

Formel II-a

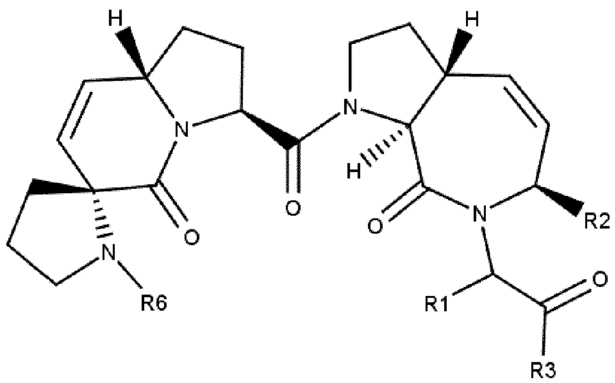

Formel II-b

wobei

R1: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise einen R-Rest einer Aminosäure, wobei die genannten Reste optional substituiert sind,
R2: Alkyl, Aryl, Alkoxyalkyl, Cycloalkyl, Mercaptoalkyl, Sulfidoalkyl, Arylalkyl, Fluoralkyl,
R3: OH, Alkyl, O-Alkyl, O-Aryl, NRR‘ (R, R‘: H oder Alkyl), NROR‘ (R, R‘: H, Alkyl), Heteroaryl,
R6: H, Alkyl, Aryl, Cycloalkyl, Alkylcarbonyl, Arylcarbonyl, Alkoxycarbonyl, Arylalkyl, Aminocarbonyl, Aryloxycarbonyl, Alkylsulfonyl, Arylsulfonyl, Aminoacyl und/oder Peptidyl, wobei die genannten Reste optional substituiert sind, oder

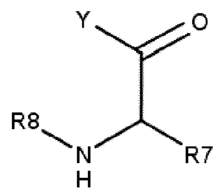

Y: Bindung zur Struktur gemäß Formel II-a oder II-b,
R7: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise ein R-Rest einer Aminosäure, wobei die genannten Reste optional substituiert sind,
R8: H, Alkyl, Aryl, Acyl, Heteroaryl, Cycloalkyl, Alkyl-Heteroaryl, Alkylcarbonyl, Arylalkyl, Arylcarbonyl, Alkoxycarbonyl, Aminocarbonyl, Aryloxycarbonyl, Alkylsulfonyl, Arylsulfonyl, Aminoacyl und/oder Peptidyl, wobei die genannten Reste optional substituiert sind.

3. Verbindung nach einem der vorhergehenden Ansprüche, **dadurch gekennzeichnet, dass** R1: ein R-Rest einer Aminosäure, der sich von einer natürlichen (kanonischen) oder unnatürlichen D- oder L-konfigurierten Aminosäure ableitet.

4. Verbindung nach dem vorhergehenden Anspruch, **dadurch gekennzeichnet, dass**

R2: $CH_2OMe$, $CH_2SMe$, $CH_2CH_2OR$, $CH_2CH_2SR$ (R: C1-C4 Alkyl oder H), benzyl, $CH_2$-benzyl, oder $CH_2CH_2$-benzyl,
oder
R2: C1-C4 Alkyl, vorzugsweise Methyl, Ethyl, Propyl, *Iso*-Propyl, Butyl, *Iso*-Butyl, *Sec*-Butyl,
oder
R2: Methyl, Propyl, *Iso*-Propyl, Butyl, *Iso*-Butyl, *Sec*-Butyl.

5. Verbindung nach einem der vorhergehenden Ansprüche, **dadurch gekennzeichnet, dass** wenn R3: OH, die Verbindung als Salz vorliegt.

6. Verbindung nach einem der vorhergehenden Ansprüche, **dadurch gekennzeichnet, dass** R4, R6: können gleich oder unterschiedlich sein, H, Alkyl, Aryl, Acyl, Peptidyl, Alkylsulfonyl, Arylsulfonyl, Heteroaryl, wobei die genannten Reste optional substituiert sind.

7. Verbindung nach Anspruch 2, **dadurch gekennzeichnet, dass**

R6:

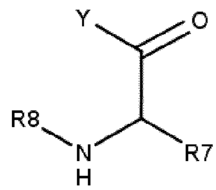

Y: Bindung zur Struktur gemäß Formel II-a oder II-b,
R7: H, Alkyl, Aryl, Heteroaryl, Cycloalkyl, Hydroxyalkyl, Arylalkyl, Alkylheteroaryl, Aminoalkyl, Aminocarbonyl, Alkylaminocarbonyl, Alkylcarbonyl, Mercaptoalkyl, Sulfidoalkyl, vorzugsweise ein R-Rest einer Aminosäure, wobei die genannten Reste optional substituiert sind,
R8: H, Alkyl, Aryl, Acyl, Peptidyl, Alkylsulfonyl, Arylsulfonyl, Heteroaryl, wobei die genannten Reste optional substituiert sind.

8. Verbindung nach Anspruch 2, **dadurch gekennzeichnet, dass**
R7: CH2-aryl ist, insbesondere substituiertes -CH2-Phenyl, wobei der Substituent an der Ortho-Position der Phenylgruppe positioniert ist, wobei der Substituent bevorzugt Halogen ist, insbesondere Cl.

**9.** Verbindung nach Anspruch 2, **dadurch gekennzeichnet, dass** R6: N-Acyl-(2-chlorphenyl)alaninoyl.

**10.** Verbindung nach einem der vorhergehenden Ansprüche, wobei optional substituiert bedeutet ein oder mehrere Wasserstoffatome der Kohlenwasserstoffgruppe durch einen oder mehrere Substituenten ersetzt sind, ausgewählt aus der Gruppe bestehend aus Halogen (vorzugsweise Fluor, Chlor, Brom und lod), -OH, -CN, -SH, Amin (vorzugsweise -NH2, -NHCH3), -NO2, und -Alkyl, wobei Alkyl gegebenenfalls mit einem oder mehreren Substituenten optional substituiert ist; wobei vorzugsweise 1, 2, 3 oder 4 optionale Substituenten vorhanden sind.

**11.** Verbindung nach einem der vorhergehenden Ansprüche, gemäß Formel III:

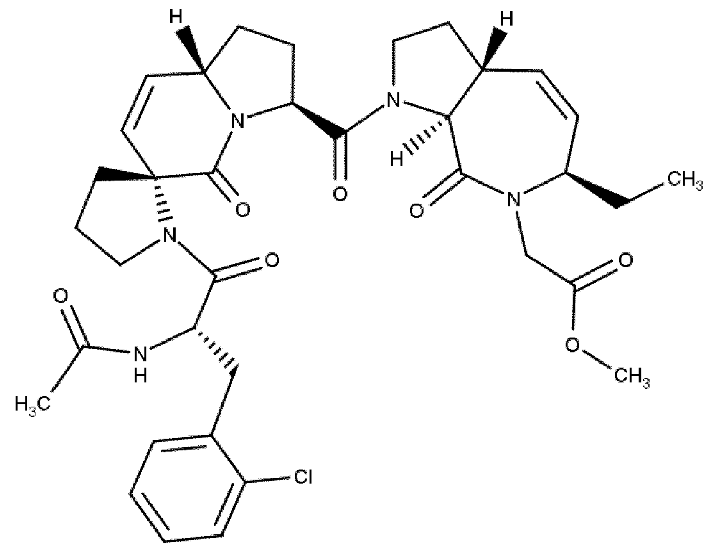

Formel III

**12.** Verbindung nach einem der vorhergehenden Ansprüche, gemäß Formel IV, V, VI, VII oder VIII:

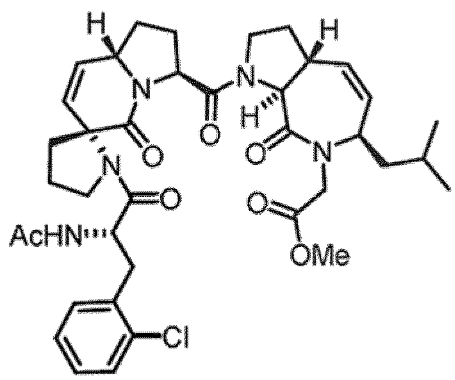

Formel IV

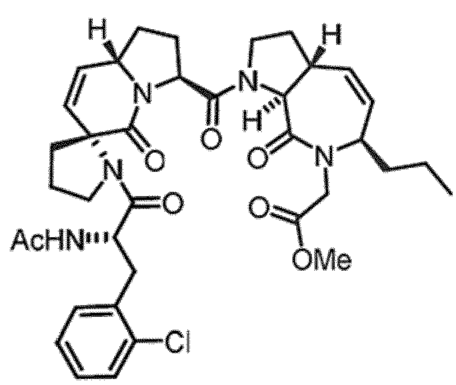

Formel V

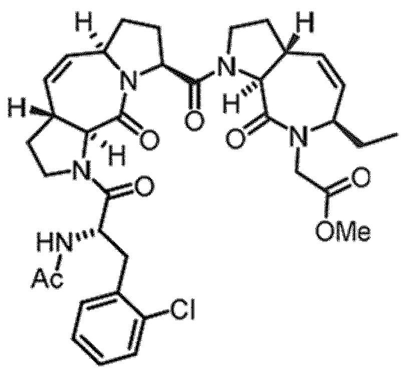

Formel VI

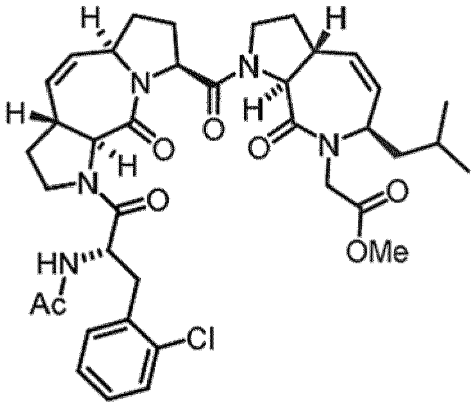

Formel VII

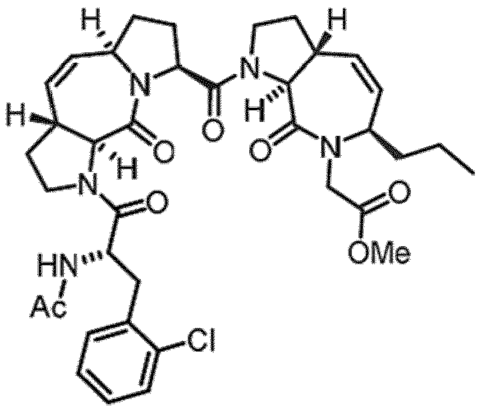

Formel VIII

**13.** Pharmazeutische Zusammensetzung umfassend eine Verbindung nach einem der vorhergehenden Ansprüche, bevorzugt mit einem pharmazeutisch annehmbaren Träger.

**14.** Verbindung nach einem der vorhergehenden Ansprüche zur Verwendung als Medikament zur Behandlung von Tumorerkrankungen, wobei die Tumorerkrankung vorzugsweise Brustkrebs ist.

**15.** Verbindung oder Zusammensetzung nach einem der vorhergehenden Ansprüche zur Verwendung als Medikament zur Behandlung von Tumorerkrankungen, **dadurch gekennzeichnet, dass** die Verbindung die Metastasierung eines Tumors inhibiert.

**Claims**

**1.** A compound according to formula I:

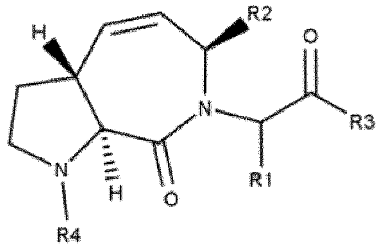

wherein

R1: H, alkyl, aryl, heteroaryl, cycloalkyl, hydroxyalkyl, arylalkyl, alkylheteroaryl, aminoalkyl, aminocarbonyl, alkylaminocarbonyl, alkylcarbonyl, mercaptoalkyl, sulfidoalkyl, preferably an R residue of an amino acid, wherein said residues are optionally substituted,
R2: alkyl, aryl, alkoxyalkyl, cycloalkyl, mercaptoalkyl, sulfidoalkyl, arylalkyl, fluoroalkyl
R3: OH, alkyl, O-alkyl, O-aryl, NRR' (R, R': H or alkyl), NROR' (R, R': H, alkyl), heteroaryl,
R4: H, alkyl, acyl, aryl, heteroaryl, cycloalkyl, alkylheteroaryl, alkylcarbonyl, arylalkyl, arylcarbonyl, alkoxycarbonyl, aminocarbonyl, aryloxycarbonyl, alkylsulfonyl, arylsulfonyl, aminoacyl and/or peptidyl, or R5, wherein said residues are optionally substituted,
R5:

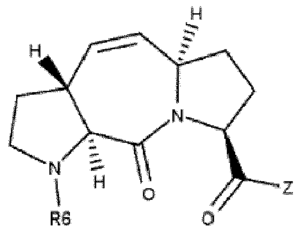

or

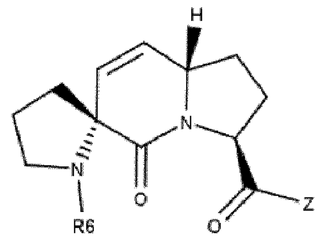

Z: bond to the structure according to formula I,
R6: H, alkyl, aryl, cycloalkyl, alkylcarbonyl, arylcarbonyl, alkoxycarbonyl, arylalkyl, aminocarbonyl, aryloxycarbonyl, alkylsulfonyl, arylsulfonyl, aminoacyl and/or peptidyl, wherein said residues are optionally substituted, or

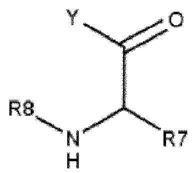

Y: bond to the structure according to R5,
R7: H, alkyl, aryl, heteroaryl, cycloalkyl, hydroxyalkyl, arylalkyl, alkylheteroaryl, aminoalkyl, aminocarbonyl, alkylaminocarbonyl, alkylcarbonyl, mercaptoalkyl, sulfidoalkyl, preferably an R residue of an amino acid, wherein said residues are optionally substituted,
R8: H, alkyl, aryl, acyl, heteroaryl, cycloalkyl, alkylheteroaryl, alkylcarbonyl, arylalkyl, arylcarbonyl, alkoxycarbonyl, aminocarbonyl, aryloxycarbonyl, alkylsulfonyl, arylsulfonyl, aminoacyl and/or peptidyl, wherein said residues are optionally substituted.

**2.** The compound according to claim 1 according to formula II-a or formula II-b:

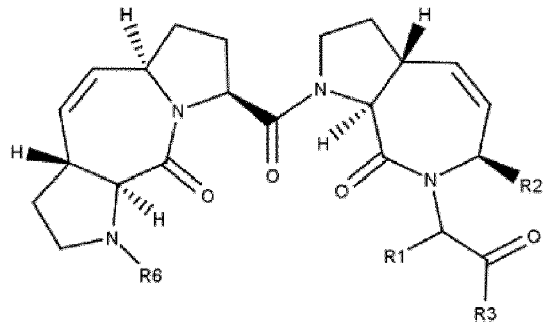

formula II-a

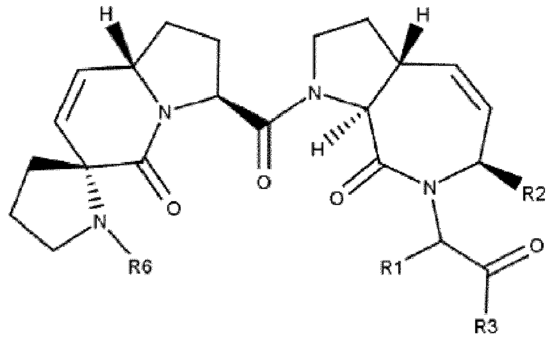

formula II-b

wherein

R1: H, alkyl, aryl, heteroaryl, cycloalkyl, hydroxyalkyl, arylalkyl, alkylheteroaryl, aminoalkyl, aminocarbonyl, alkylaminocarbonyl, alkylcarbonyl, mercaptoalkyl, sulfidoalkyl, preferably an R residue of an amino acid, wherein said residues are optionally substituted,
R2: alkyl, aryl, alkoxyalkyl, cycloalkyl, mercaptoalkyl, sulfidoalkyl, arylalkyl, fluoroalkyl,
R3: OH, alkyl, O-alkyl, O-aryl, NRR' (R, R': H or alkyl), NROR' (R, R': H, alkyl), heteroaryl,
R6: H, alkyl, aryl, cycloalkyl, alkylcarbonyl, arylcarbonyl, alkoxycarbonyl, arylalkyl, aminocarbonyl, aryloxycarbonyl, alkylsulfonyl, arylsulfonyl, aminoacyl and/or peptidyl, wherein said residues are optionally substituted, or

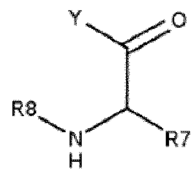

Y: bond to the structure according to formula II-a or II-b,
R7: H, alkyl, aryl, heteroaryl, cycloalkyl, hydroxyalkyl, arylalkyl, alkylheteroaryl, aminoalkyl, aminocarbonyl, alkylaminocarbonyl, alkylcarbonyl, mercaptoalkyl, sulfidoalkyl, preferably an R residue of an amino acid, wherein said residues are optionally substituted,
R8: H, alkyl, aryl, acyl, heteroaryl, cycloalkyl, alkylheteroaryl, alkylcarbonyl, arylalkyl, arylcarbonyl, alkoxycarbonyl, aminocarbonyl, aryloxycarbonyl, alkylsulfonyl, arylsulfonyl, aminoacyl and/or peptidyl, wherein said residues are optionally substituted.

3. The compound according to any one of the preceding claims, **characterized in that** R1: an R residue of an amino acid which is derived from a natural (canonical) or unnatural D- or L-configured amino acid.

4. The compound according to the preceding claim, **characterized in that**

R2: $CH_2OMe$, $CH_2SMe$, $CH_2CH_2OR$, $CH_2CH_2SR$ (R: C1-C4 alkyl or H), benzyl, $CH_2$ benzyl, or $CH_2CH_2$ benzyl, or

R2: C1-C4 alkyl, preferably methyl, ethyl, propyl, iso-propyl, butyl, iso-butyl, sec-butyl,
or
R2: methyl, propyl, iso-propyl, butyl, iso-butyl, sec-butyl.

**5.** The compound according to any one of the preceding claims, **characterized in that** when R3: OH, the compound is in the form of a salt.

**6.** The compound according to any one of the preceding claims, **characterized in that** R4, R6: can be the same or different, H, alkyl, aryl, acyl, peptidyl, alkylsulfonyl, arylsulfonyl, heteroaryl, where said residues are optionally substituted.

**7.** The compound according to claim 2, **characterized in that** R6:

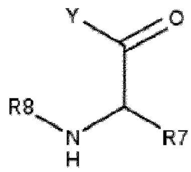

Y: bond to the structure according to formula II-a or II-b,
R7: H, alkyl, aryl, heteroaryl, cycloalkyl, hydroxyalkyl, arylalkyl, alkylheteroaryl, aminoalkyl, aminocarbonyl, alkylaminocarbonyl, alkylcarbonyl, mercaptoalkyl, sulfidoalkyl, preferably an R residue of an amino acid, wherein said residues are optionally substituted,
R8: H, alkyl, aryl, acyl, peptidyl, alkylsulfonyl, arylsulfonyl, heteroaryl, wherein said residues are optionally substituted.

**8.** The compound according to claim 2, **characterized in that** R7: is CH2-aryl, in particular substituted -CH2-phenyl, wherein the substituent is positioned in the ortho position of the phenyl group, preferably wherein the substituent is halogen, in particular Cl.

**9.** The compound according to claim 2, **characterized in that** R6: N-acyl-(2-chlorophenyl)alaninoyl.

**10.** The compound according to any one of the preceding claims, wherein optionally substituted means one or more hydrogen atoms of the hydrocarbon group are replaced by one or more substituents selected from the group consisting of halogen (preferably fluorine, chlorine, bromine and iodine), -OH, -CN , -SH, amine (preferably -NH2, -NHCH3), -NO2, and -alkyl, wherein alkyl is optionally substituted with one or more substituents; preferably wherein 1, 2, 3 or 4 optional substituents are present.

**11.** The compound according to any one of the preceding claims, according to formula III:

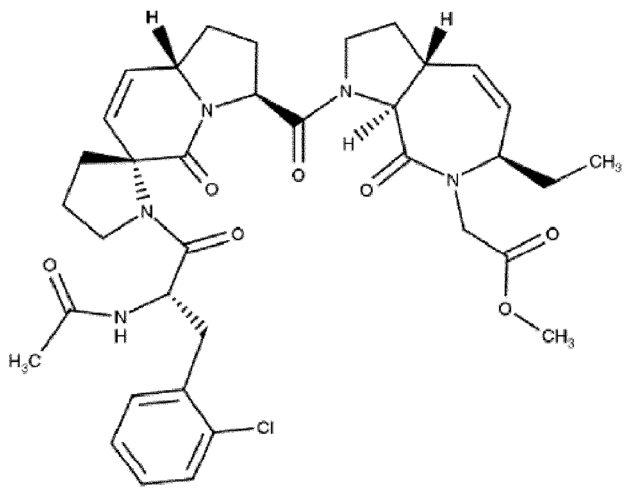

formula III

**12.** The compound according to any one of the preceding claims, according to formula IV, V, VI, VII or VIII:

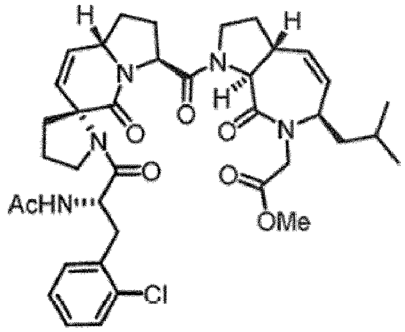

formula IV

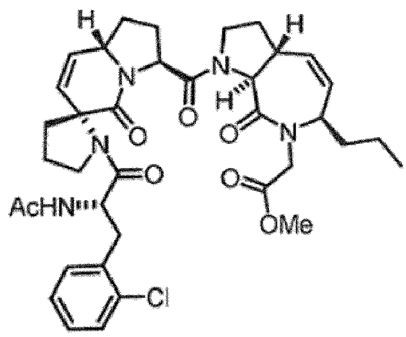

formula V

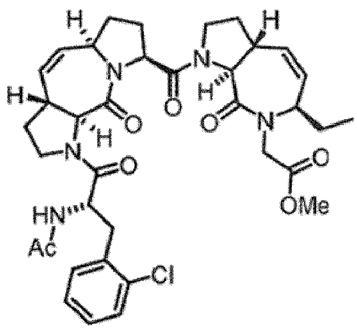

formula VI

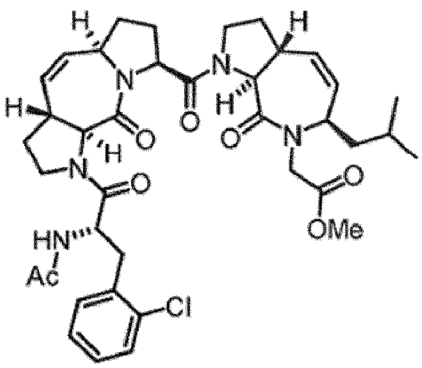

formula VII

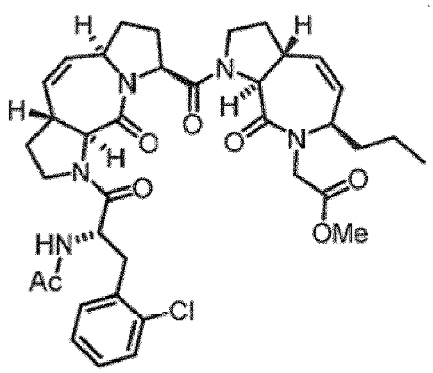

formula VIII

**13.** A pharmaceutical composition, comprising a compound according to any one of the preceding claims, preferably with a pharmaceutically acceptable carrier.

**14.** The compound according to any one of the preceding claims for use as a medicament for the treatment of tumor diseases, preferably wherein the tumor disease is breast cancer.

**15.** The compound or composition according to any one of the preceding claims for use as a medicament for the treatment of tumor diseases, **characterized in that** the compound inhibits metastasis of a tumor.

## Revendications

**1.** Composé selon la formule I :

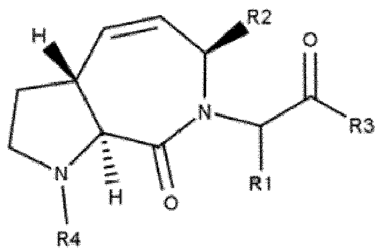

où

R1 : H, alkyle, aryle, hétéroaryle, cycloalkyle, hydroxyalkyle, arylalkyle, alkylhétéroaryle, aminoalkyle, aminocarbonyle, alkylaminocarbonyle, alkylcarbonyle, mercaptoalkyle, sulfidoalkyle, de préférence un radical R d'un acide aminé, dans lequel les radicaux cités sont éventuellement substitués,
R2 : alkyle, aryle, alcoxyalkyle, cycloalkyle, mercaptoalkyle, sulfidoalkyle, arylalkyle, fluoroalkyle
R3 : OH, alkyle, O-alkyle, O-aryle, NRR' (R, R' : H ou alkyle), NROR' (R, R' : H, alkyle), hétéroaryle,
R4 : H, alkyle, acyle, aryle, hétéroaryle, cycloalkyle, alkylhétéroaryle, alkylcarbonyle, arylalkyle, arylcarbonyle, alcoxycarbonyle, aminocarbonyle, aryloxycarbonyle, alkylsulfonyle, arylsulfonyle, aminoacyle et/ou peptidyle, ou R5, dans lequel les radicaux mentionnés sont éventuellement substitués,
R5 :

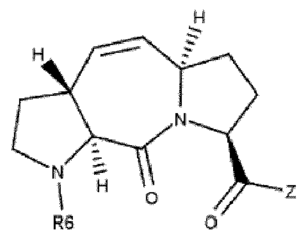

ou

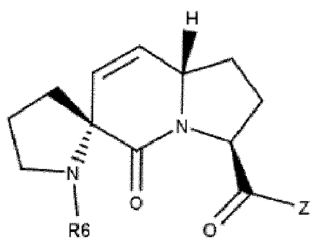

Z : liaison à la structure selon la formule I,

R6 : H, alkyle, aryle, cycloalkyle, alkylcarbonyle, arylcarbonyle, alcoxycarbonyle, arylalkyle, aminocarbonyle, aryloxycarbonyle, alkylsulfonyle, arylsulfonyle, aminoacyle et/ou peptidyle, dans lequel les radicaux mentionnés sont éventuellement substitués, ou

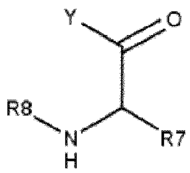

Y : liaison à la structure selon R5,

R7 : H, alkyle, aryle, hétéroaryle, cycloalkyle, hydroxyalkyle, arylalkyle, alkylhétéroaryle, aminoalkyle, aminocarbonyle, alkylaminocarbonyle, alkylcarbonyle, mercaptoalkyle, sulfidoalkyle, de préférence un radical R d'un acide aminé, dans lequel les radicaux cités sont éventuellement substitués,

R8 : H, alkyle, aryle, acyle, hétéroaryle, cycloalkyle, alkylhétéroaryle, alkylcarbonyle, arylalkyle, arylcarbonyle, alcoxycarbonyle, aminocarbonyle, aryloxycarbonyle, alkylsulfonyle, arylsulfonyle, aminoacyle et/ou peptidyle, dans lequel les radicaux mentionnés sont éventuellement substitués.

**2.** Composé selon la revendication 1 selon la formule II-a ou la formule II-b :

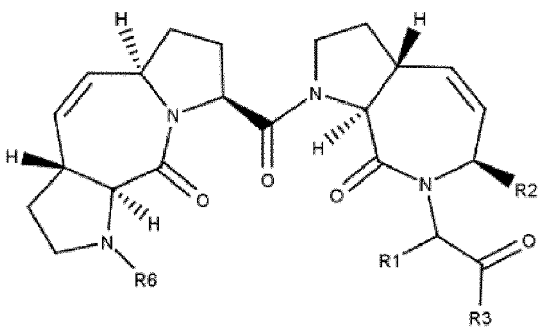

Formule II-a

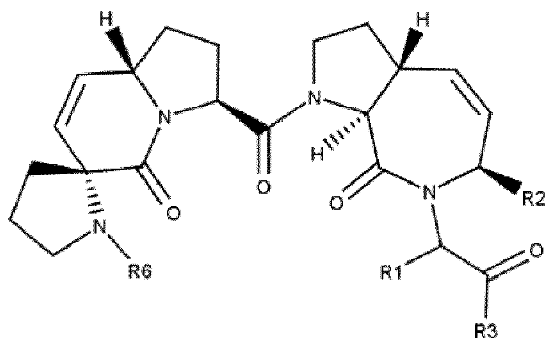

Formule II-b

où

R1 : H, alkyle, aryle, hétéroaryle, cycloalkyle, hydroxyalkyle, arylalkyle, alkylhétéroaryle, aminoalkyle, aminocarbonyle, alkylaminocarbonyle, alkylcarbonyle, mercaptoalkyle, sulfidoalkyle, de préférence un radical R d'un acide aminé, dans lequel les radicaux cités sont éventuellement substitués,

R2 : alkyle, aryle, alcoxyalkyle, cycloalkyle, mercaptoalkyle, sulfidoalkyle, arylalkyle, fluoroalkyle,

R3 : OH, alkyle, O-alkyle, O-aryle, NRR' (R, R' : H ou alkyle), NROR' (R, R' : H, alkyle), hétéroaryle,

R6 : H, alkyle, aryle, cycloalkyle, alkylcarbonyle, arylcarbonyle, alcoxycarbonyle, arylalkyle, aminocarbonyle, aryloxycarbonyle, alkylsulfonyle, arylsulfonyle, aminoacyle et/ou peptidyle, dans lequel les radicaux mentionnés sont éventuellement substitués, ou

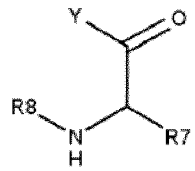

Y : liaison à la structure selon la formule II-a ou II-b,
R7 : H, alkyle, aryle, hétéroaryle, cycloalkyle, hydroxyalkyle, arylalkyle, alkylhétéroaryle, aminoalkyle, aminocarbonyle, alkylaminocarbonyle, alkylcarbonyle, mercaptoalkyle, sulfidoalkyle, de préférence un radical R d'un acide aminé, dans lequel les radicaux cités sont éventuellement substitués,
R8 : H, alkyle, aryle, acyle, hétéroaryle, cycloalkyle, alkylhétéroaryle, alkylcarbonyle, arylalkyle, arylcarbonyle, alcoxycarbonyle, aminocarbonyle, aryloxycarbonyle, alkylsulfonyle, arylsulfonyle, aminoacyle et/ou peptidyle, dans lequel les radicaux mentionnés sont éventuellement substitués.

3. Composé selon l'une des revendications précédentes, **caractérisé en ce que** R1 : un radical R d'un acide aminé dérivé d'un acide aminé naturel (canonique) ou non naturel en configuration D ou L.

4. Composé selon la revendication précédente, **caractérisé en ce que**

R2 : $CH_2OMe$, $CH_2SMe$, $CH_2CH_2OR$, $CH_2CH_2SR$ (R : alkyle en C1-C4 ou H), benzyle, $CH_2$-benzyle ou $CH_2CH_2$-benzyle,
ou
R2 : alkyle en C1-C4, de préférence méthyle, éthyle, propyle, iso-propyle, butyle, *iso*-butyle, *sec*-butyle,
ou
R2 : méthyle, propyle, *iso*-propyle, butyle, *iso*-butyle, *sec*-butyle

5. Composé selon l'une des revendications précédentes, **caractérisé en ce que** lorsque R3 : OH, le composé est sous forme de sel.

6. Composé selon l'une des revendications précédentes, **caractérisé en ce que** R4, R6 : peuvent être identiques ou différents, H, alkyle, aryle, acyle, peptidyle, alkylsulfonyle, arylsulfonyle, hétéroaryle, dans lequel les radicaux mentionnés sont éventuellement substitués.

7. Composé selon la revendication 2, **caractérisé en ce que** R6 :

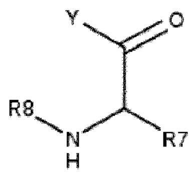

Y : liaison à la structure selon la formule II-a ou II-b,
R7 : H, alkyle, aryle, hétéroaryle, cycloalkyle, hydroxyalkyle, arylalkyle, alkylhétéroaryle, aminoalkyle, aminocarbonyle, alkylaminocarbonyle, alkylcarbonyle, mercaptoalkyle, sulfidoalkyle, de préférence un radical R d'un acide aminé, dans lequel les radicaux cités sont éventuellement substitués,
R8 : H, alkyle, aryle, acyle, peptidyle, alkylsulfonyle, arylsulfonyle, hétéroaryle, dans lequel les radicaux mentionnés sont éventuellement substitués.

8. Composé selon la revendication 2, **caractérisé en ce que** R7 est aryle en CH2, en particulier phényle en CH2 substitué, dans lequel le substituant est positionné en position ortho du groupe phényle, dans lequel le substituant est de préférence un halogène, en particulier Cl.

9. Composé selon la revendication 2, **caractérisé en ce que** R6 : N-acyl-(2-chlorophényl)alaninoyle.

10. Composé selon l'une des revendications précédentes, dans lequel éventuellement substitué signifie qu'un ou plusieurs atomes d'hydrogène du groupe hydrocarbure sont remplacés par un ou plusieurs substituants choisis dans

le groupe constitué de l'halogène (de préférence fluor, chlore, brome et iode), -OH, - CN, -SH, amine (de préférence -NH2, -NHCH3), -NO2 et -alkyle, dans lequel l'alkyle est éventuellement substitué par un ou plusieurs substituants ; dans lequel il y a, de préférence, 1, 2, 3 ou 4 substituants optionnels.

**11.** Composé selon l'une des revendications précédentes, selon la formule III :

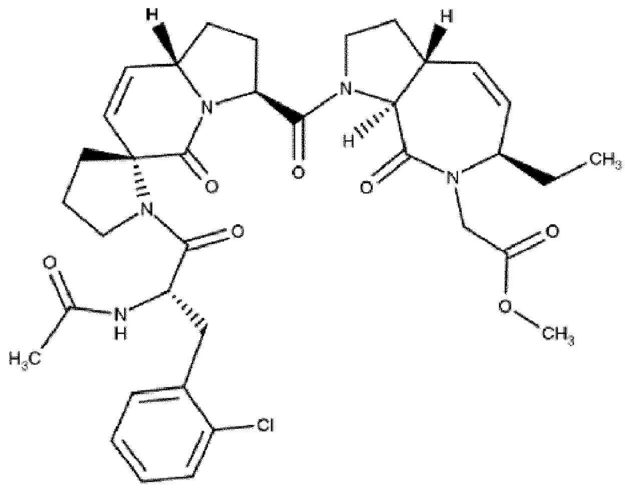

Formule III

**12.** Composé selon l'une des revendications précédentes, selon la formule IV, V, VI, VII ou VIII :

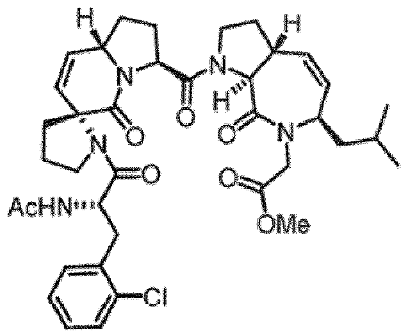

Formule IV

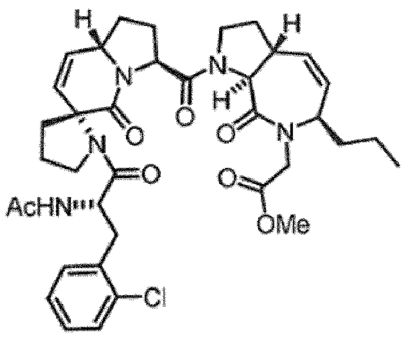

Formule V

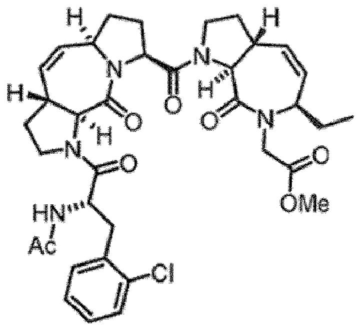

Formule VI

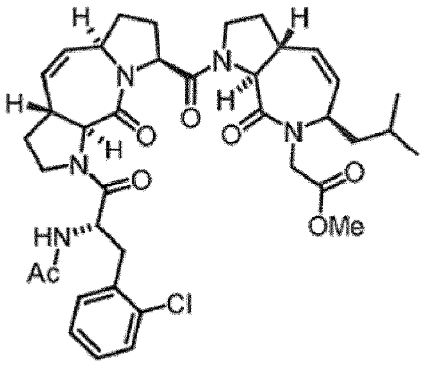

Formule VII

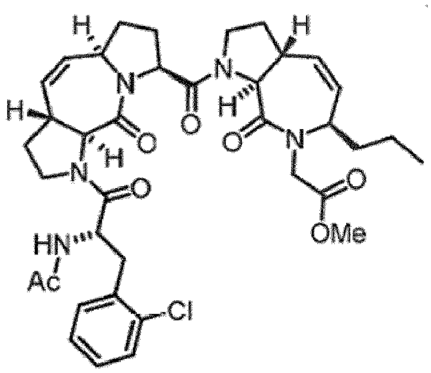

Formule VIII

**13.** Composition pharmaceutique comprenant un composé selon l'une des revendications précédentes, de préférence avec un support pharmaceutiquement acceptable.

**14.** Composé selon l'une des revendications précédentes pour une utilisation comme médicament pour le traitement des maladies tumorales, dans lequel la maladie tumorale est de préférence le cancer du sein.

**15.** Composé ou composition selon l'une des revendications précédentes pour une utilisation comme médicament pour le traitement des maladies tumorales, **caractérisé en ce que** le composé inhibe la métastase d'une tumeur.

**Fig. 1**

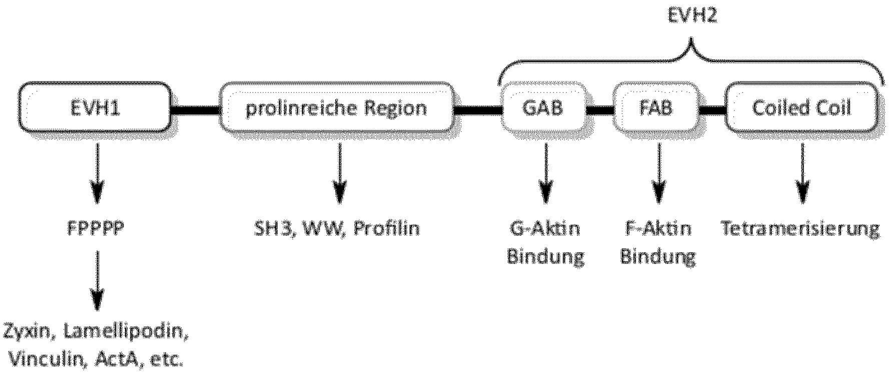


**Fig. 2**

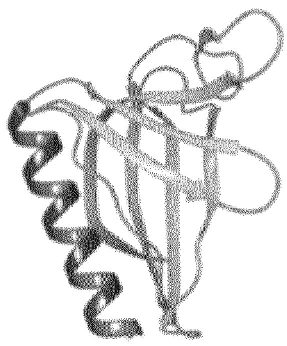

**Abbildung 2.8:**
Die Ena/VASP-EVH1-Domäne.[4]

**Fig. 3**

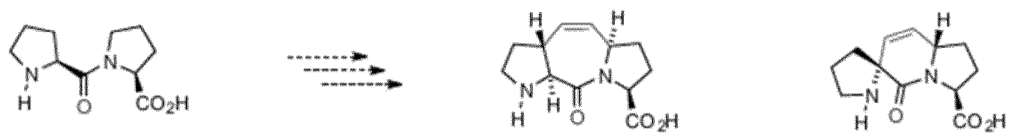

Pro-Pro (**2**) **ProM-1** **ProM-2**

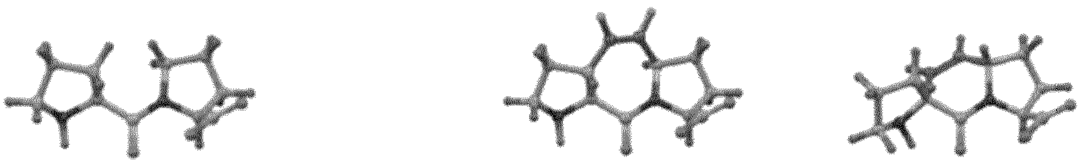

**Fig. 4**

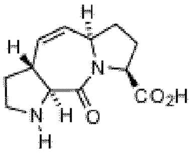

**ProM-1**[58,80]

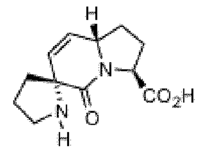

**ProM-2**[77]

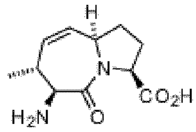

**ProM-3**[90]

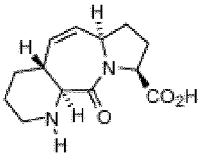

**ProM-4**[91]

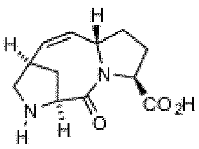

**ProM-5**[89]

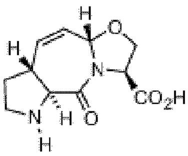

**ProM-6**[73]

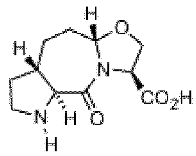

**Dihydro-ProM-6**[73]

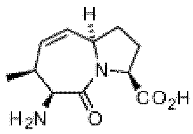

**ProM-7**[90]

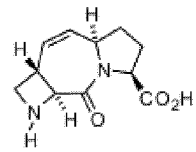

**ProM-8**[91]

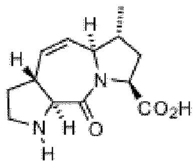

**ProM-9**[62]

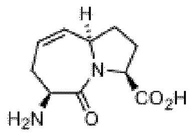

**ProM-10**

**Fig. 5**

**Ac-SFE-FPPPP-TEDEL-$NH_2$ (40)**

$K_d$ = 20 $\mu$M; LE = -0.2 kJ/mol; 1546-Da

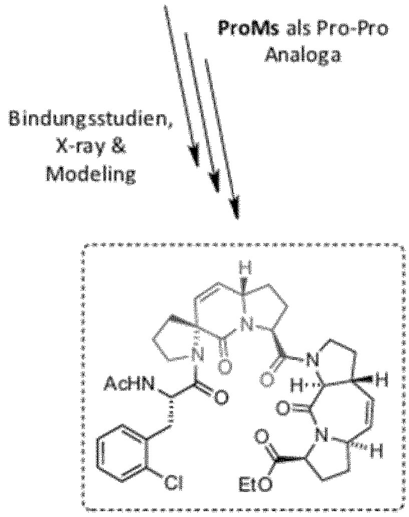


**Ac-[L-2Cl-F][ProM-2][ProM-1]-OEt (44)**

$K_d$ = 9.4 $\mu$M; LE = -0.6 kJ/mol; 706-Da

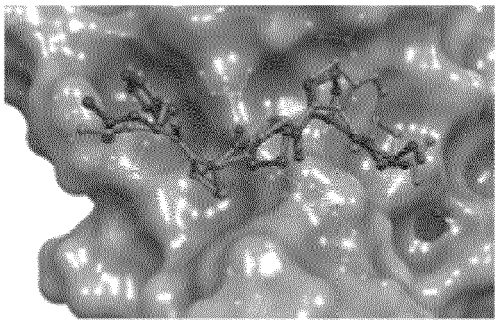

**Ac-SFE-[L-2Cl-F][ProM-2][ProM-1]-TEDEL-$NH_2$ (41)**

$K_d$ = 0.6 $\mu$M; LE = -0.3 kJ/mol

**Ac-FPPPP-OH (42)**

$K_d$ = 1300 $\mu$M; 596-Da

**Ac-[L-2Cl-F][ProM-2][ProM-1]-OH (43)**

$K_d$ = 4 $\mu$M; LE = -0.7 kJ/mol; 678-Da

**Einführung von nur fünf zusätzlichen Schweratomen!**

Fig. 6

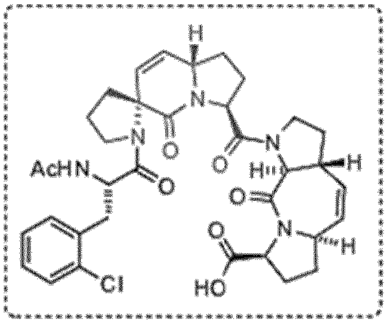

Ac-[L-2Cl-F]-[ProM-2]-[ProM-1]-OH (43)

Fig. 7

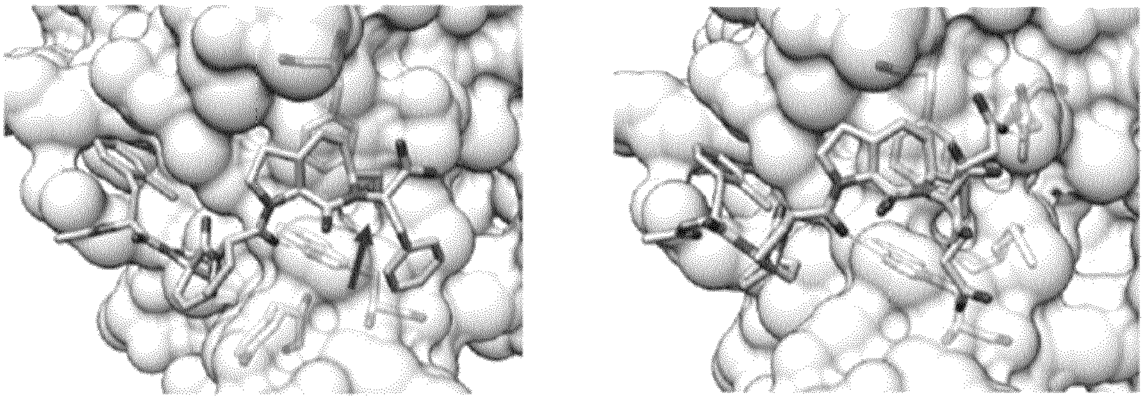

Fig. 8

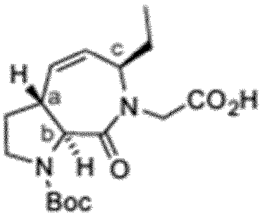

Boc-*R,S,R*-**Gly-[ProM-15]**-OH

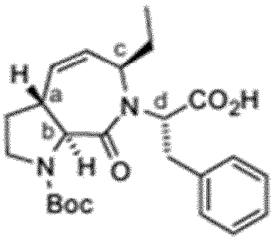

Boc-*R,S,R,S*-**Phe-[ProM-15]**-OH

**Fig. 9**

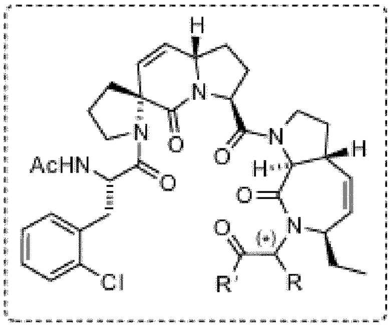

Ac-[L-2Cl-F]-**[ProM-2]**-**[ProM-15]**-R′ (**115**)

**Fig. 10**

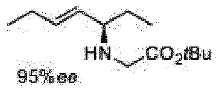
95%*ee*
**153**

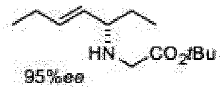
95%*ee*
*ent*-**153**

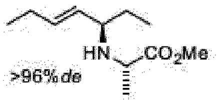
>96%*de*
**171**

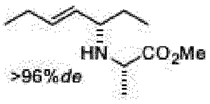
>96%*de*
*dia*-**171**

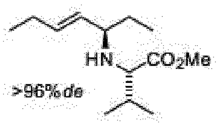
>96%*de*
**172**

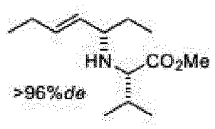
>96%*de*
*dia*-**172**

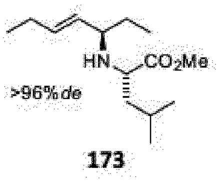
>96%*de*
**173**

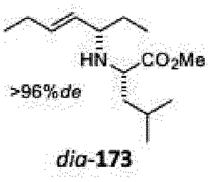
>96%*de*
*dia*-**173**

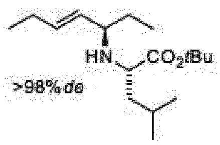
>98%*de*
**174**

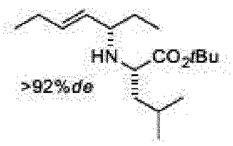
>92%*de*
*dia*-**174**

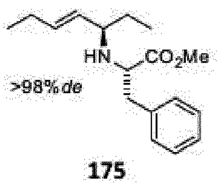
>98%*de*
**175**

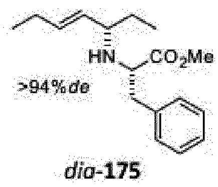
>94%*de*
*dia*-**175**

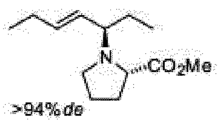
>94%*de*
**176**

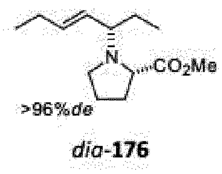
>96%*de*
*dia*-**176**

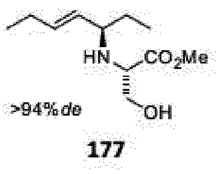
>94%*de*
**177**

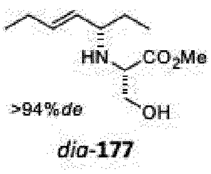
>94%*de*
*dia*-**177**

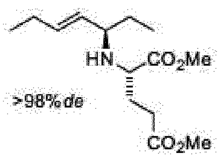
>98%*de*
**178**

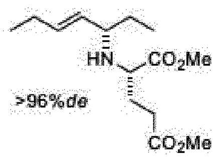
>96%*de*
*dia*-**178**

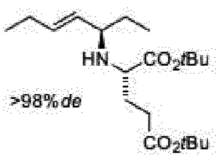
>98%*de*
**179**

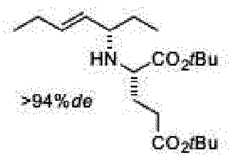
>94%*de*
*dia*-**179**

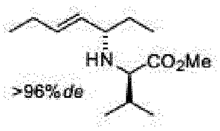
>96%*de*
*ent*-**172**

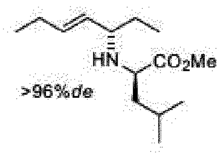
>96%*de*
*ent*-**174**

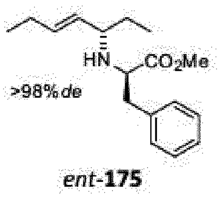
>98%*de*
*ent*-**175**

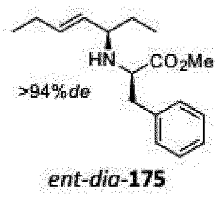
>94%*de*
*ent*-*dia*-**175**

Fig. 11

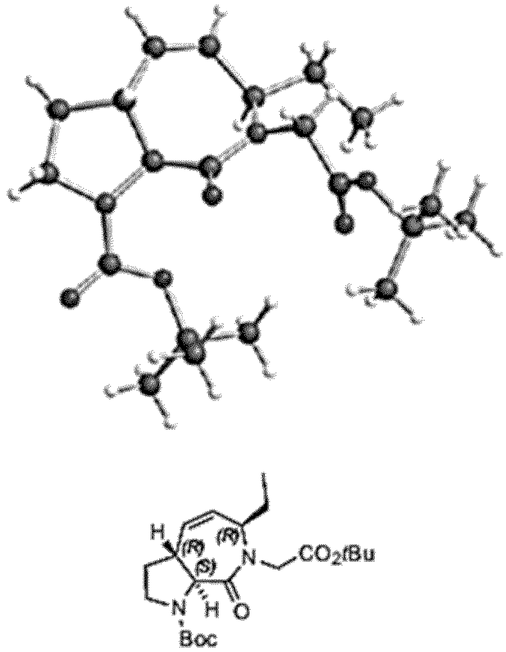

Boc-*R,S,R*-**Gly-[ProM-15]**-O*t*Bu

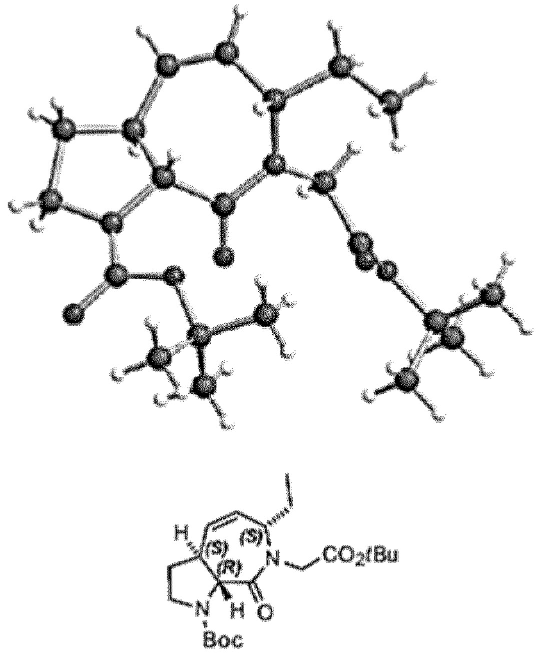

Boc-*S,R,S*-**Gly-[ProM-15]**-O*t*Bu

**Fig. 12**

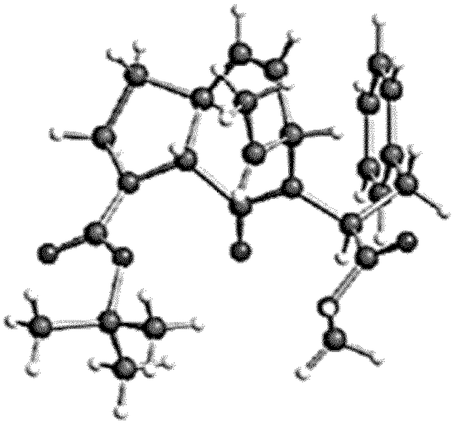

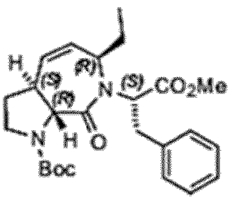

Boc-*S,R,R,S*-**Phe-[ProM-15]**-OMe

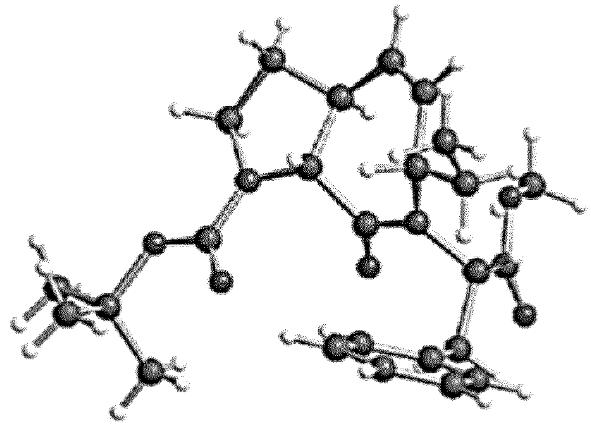

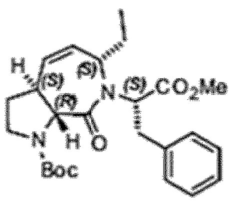

Boc-*S,R,S,S*-**Phe-[ProM-15]**-OMe

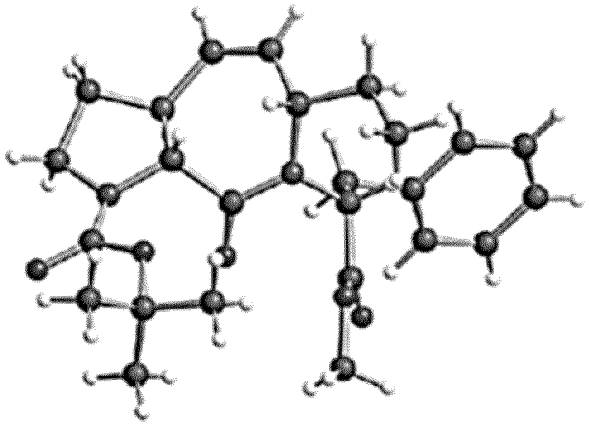

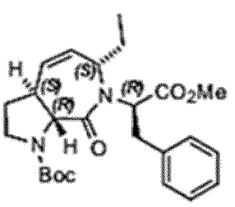

Boc-*S,R,S,R*-**Phe-[ProM-15]**-OMe

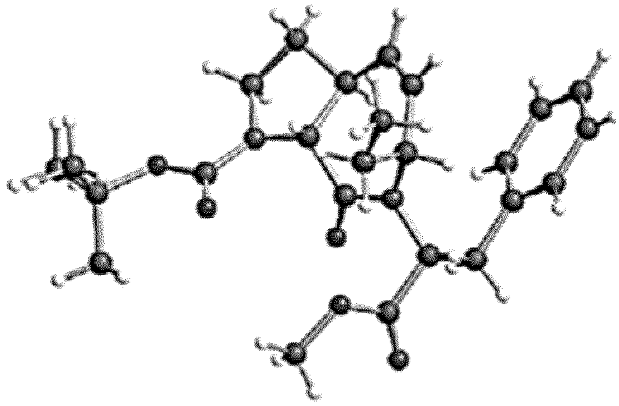

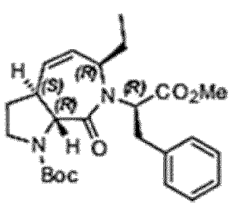

Boc-*S,R,R,R*-**Phe-[ProM-15]**-OMe

Fig. 13

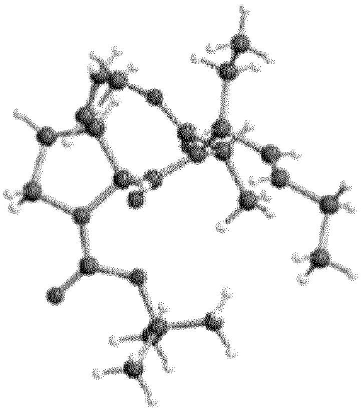

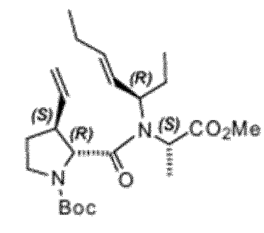

*dia*-**214-b**

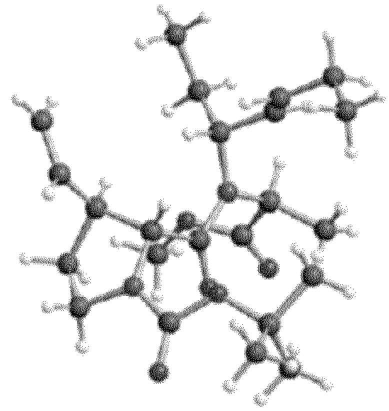

Fig. 14

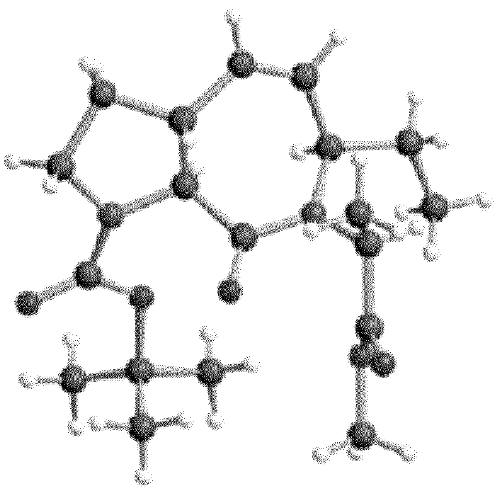

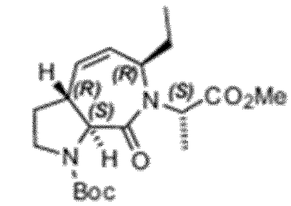

Boc-*R,S,R,S*-**Ala-[ProM-15]**-OMe

Fig. 15

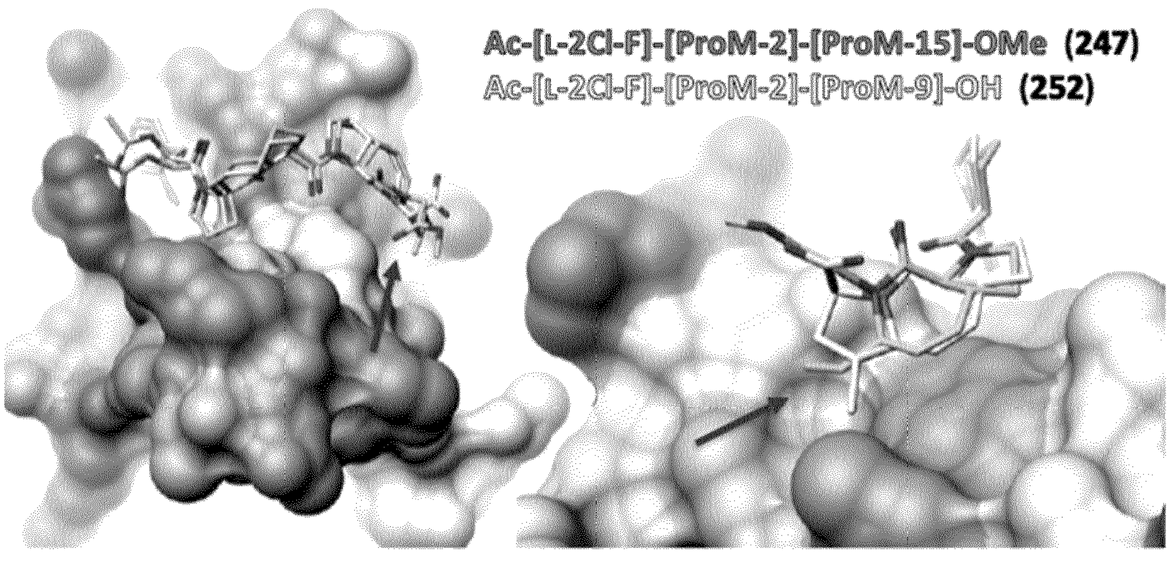

Fig. 16:

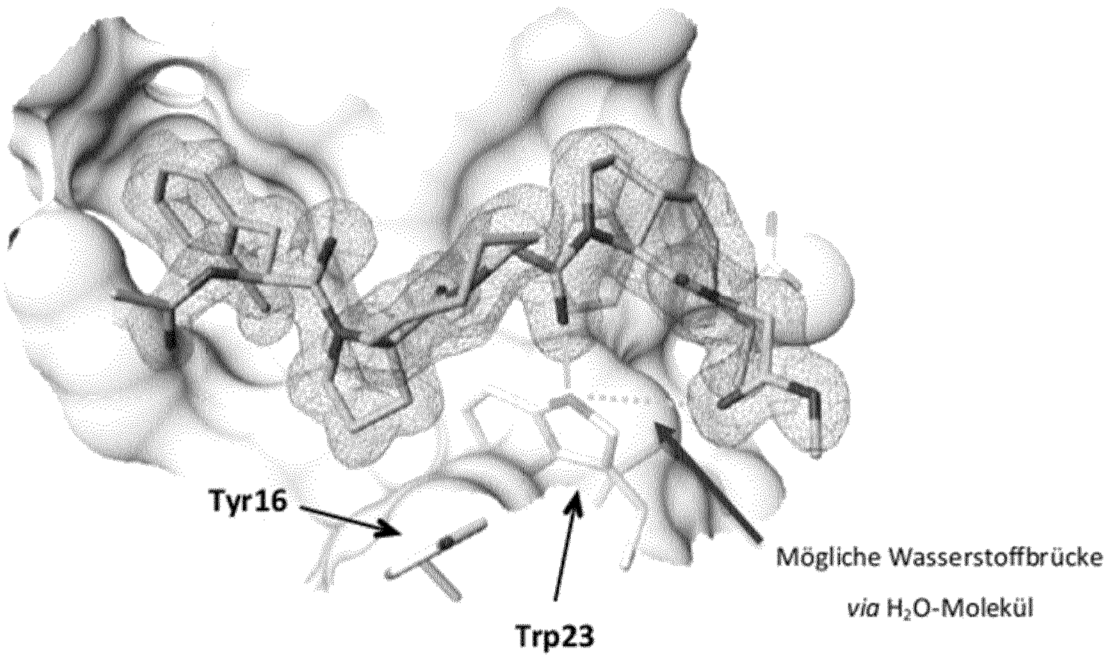

Fig. 17

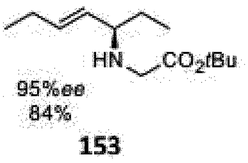
95%*ee*
84%
**153**

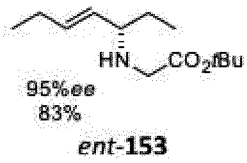
95%*ee*
83%
*ent*-**153**

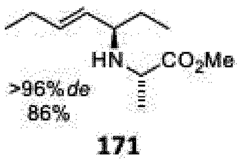
>96%*de*
86%
**171**

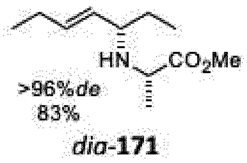
>96%*de*
83%
*dia*-**171**

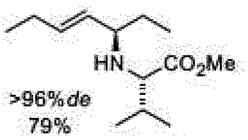
>96%*de*
79%
**172**

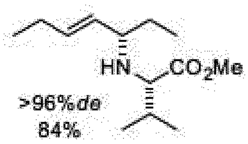
>96%*de*
84%
*dia*-**172**

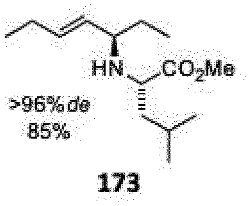
>96%*de*
85%
**173**

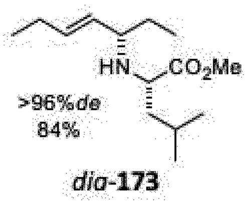
>96%*de*
84%
*dia*-**173**

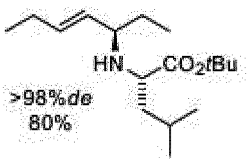
>98%*de*
80%
**174**

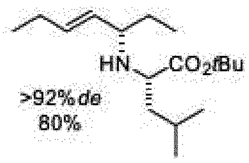
>92%*de*
80%
*dia*-**174**

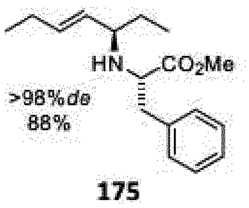
>98%*de*
88%
**175**

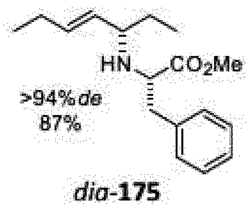
>94%*de*
87%
*dia*-**175**

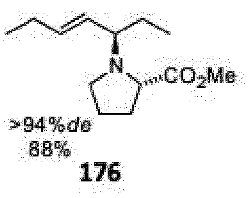
>94%*de*
88%
**176**

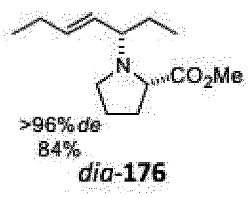
>96%*de*
84%
*dia*-**176**

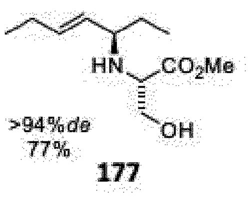
>94%*de*
77%
**177**

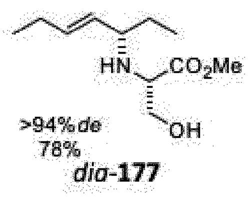
>94%*de*
78%
*dia*-**177**

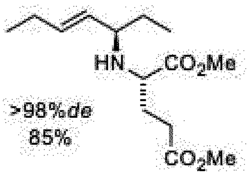
>98%*de*
85%
**178**

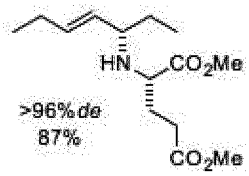
>96%*de*
87%
*dia*-**178**

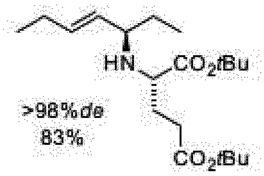
>98%*de*
83%
**179**

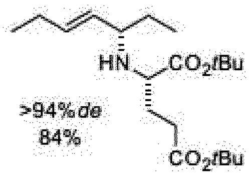
>94%*de*
84%
*dia*-**179**

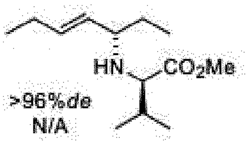
>96%*de*
N/A
*ent*-**172**

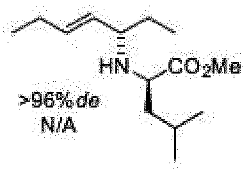
>96%*de*
N/A
*ent*-**174**

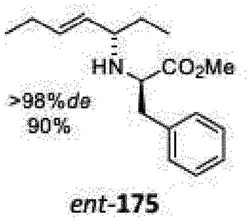
>98%*de*
90%
*ent*-**175**

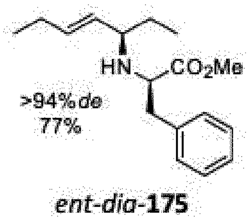
>94%*de*
77%
*ent*-*dia*-**175**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008040332 A
- WO 2013030111 A
- WO 2016092069 A


### In der Beschreibung aufgeführte Nicht-Patentliteratur


- **REUTER et al.** *Eur. J. Org. Chem.,* 2014, vol. 13, 2664-2667
- **J. ZAMINER ; C. BROCKMANN ; P. HUY ; R. OPITZ ; C. REUTER ; M. BEYERMANN ; C. FREUND ; M. MÜLLER ; H. OSCHKINAT ; R. KÜHNE.** *Angew. Chem.,* 2010, vol. 122, 7265-7269
- **C. REUTER ; P. HUY ; J.-M. NEUDÖRFL ; R. KÜHNE ; H.-G. SCHMALZ.** *Chem. Eur. J.,* 2011, vol. 17, 12037-12044
- **C. REUTER ; R. OPITZ ; A. SOICKE ; S. DOHMEN ; M. BARONE ; S. CHIHA ; M. T. KLEIN ; J.-M. NEUDÖRFL ; R. KÜHNE ; H.-G. SCHMALZ.** *Chem. Eur. J.,* 2015, vol. 21, 8464-8470
- **C. REUTER ; M. KLECZKA ; S. D. MAZANCOURT ; J.-M. NEUDÖRFL ; R. KÜHNE ; H.-G. SCHMALZ.** *Eur. J. Org. Chem.,* 2014, 2664-2667
- **A. SOICKE ; C. REUTER ; M. WINTER ; J.-M. NEUDÖRFL ; N. SCHLÖRER ; R. KÜHNE ; H.-G. SCHMALZ.** *Eur. J. Org. Chem.,* 2014, 6467-6480
- **V. HACK ; C. REUTER ; R. OPITZ ; P. SCHMIEDE ; M. BEYERMANN ; J.-M. NEUDÖRFL ; R. KÜHNE ; H.-G. SCHMALZ.** *Angew. Chem. Int. Ed.,* 2013, vol. 52, 9539-9543
- **P. HUY.** Dissertation. Universität zu Köln, 2011
- **A. SOICKE.** Dissertation. Universität zu Köln, 2014
- **R. OPITZ ; M. MÜLLER ; C. REUTER ; M. BARONE ; A. SOICKE ; Y. ROSKE ; K. PIOTUKH ; P. HUY ; M. BEERBAUM ; B. WIESNER.** *PNAS,* 2015, vol. 112, 5011-5016